(19) Europäisches Patentamt
European Patent Office
Office européen des brevets

(11) Publication number: **0 104 885**
**B1**

(12) **EUROPEAN PATENT SPECIFICATION**

(45) Date of publication of patent specification: **04.06.86**

(21) Application number: **83305588.2**

(22) Date of filing: **21.09.83**

(51) Int. Cl.⁴: **C 07 C 149/40,** C 07 C 147/14, C 07 C 147/107, C 07 D 317/14, C 07 D 233/78, C 07 D 207/404, C 07 D 303/02, C 07 C 59/90, A 61 K 31/19, C 07 C 59/64, C 07 C 51/347

(54) **Leukotriene antagonists, their production, and compositions containing them.**

(30) Priority: **23.09.82 US 422338**

(43) Date of publication of application:
**04.04.84 Bulletin 84/14**

(45) Publication of the grant of the patent:
**04.06.86 Bulletin 86/23**

(84) Designated Contracting States:
**AT BE CH DE FR GB IT LI LU NL SE**

(56) References cited:
**EP-A-0 043 736**
**EP-A-0 056 172**
**DE-A-1 768 334**
**DE-A-2 102 984**
**FR-A-2 509 725**
**GB-A-2 058 785**
**US-A-3 943 169**

(73) Proprietor: **MERCK FROSST CANADA INC.**
**16711 Trans-Canada Highway**
**Kirkland Quebec (CA)**

(72) Inventor: **Belanger, Patrice C.**
**419 Promenade d'Avignon**
**Dollard des Ormeaux Quebec, H9B 1Y4 (CA)**
Inventor: **Fortin, Rejean**
**4820 Boul. Leger**
**Montreal Quebec, H1H 1N3 (CA)**
Inventor: **Guindon, Yvan**
**409 Place Closse**
**Ile Bizard Quebec, H9C 1Y7 (CA)**
Inventor: **Rokach, Joshua**
**416 Canterbury Square Chomedey**
**Laval Quebec, H7W 4G9 (CA)**
Inventor: **Yoakim, Christiane**
**5770 Cote St. Luc Road Apt. 12**
**Montreal Quebec, H3X 2E5 (CA)**

(74) Representative: **Crampton, Keith John Allen et al**
**D. YOUNG & CO 10 Staple Inn**
**London WC1V 7RD (GB)**

**EP 0 104 885 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Although the chemical identity of leukotrienes was not discovered until 1979, their history actually began in Australia in 1938 when researchers discovered slow reacting substances (SRS) which caused slow contractions of smooth muscle. When their chemical identity was learned, SRS was found to be a mixture of three previously unknown substances which are related chemically to the prostaglandins and thromboxanes. They were named leukotrienes because they are made by leukocytes and have three conjugated double bonds. Leukotrienes have major effects on the smaller peripheral airways of the lungs and on the larger central passages which include the trachea and the bronchi. In the presence of an allergy trigger, like pollen or dust, leukotrienes are manufactured from fatty substances trapped in the membrane of a triggered cell. A series of reactions within the cell generates a set of different leukotrienes which are transported through the cell membrane into the blood. Then they bring about a constriction of the air passages leading to breathlessness.

The present invention provides compounds of Formula XI and XII below, and compounds that are their pharmaceutically acceptable salts. Compounds of these formulae can act as antagonists to prevent leukotriene action or as inhibitors to prevent synthesis, and can prevent or reverse leukotriene action or prevent leukotriene synthesis when administered orally. The compounds can be administered by insufflation, intravenously, rectally, topically, parenterally, including subcutaneously and intramuscularly, or nasally, and the present invention also provides pharmaceutical compositions containing the said compounds and a pharmaceutical carrier. Such compositions may be suitable for administration in the ways stated.

Because of their activity as leukotriene antagonists, compounds of the present invention are useful as anti-asthmatic, anti-allergic, and anti-inflammatory agents and are useful in treating allergic rhinitis and chronic bronchitis and for amelioration of skin diseases like psoriasis and atopic eczema. These compounds are also useful to antagonize or inhibit the properties of leukotrienes relating to cardiovascular and vascular systems.

The compounds of the present invention have the Formulae:

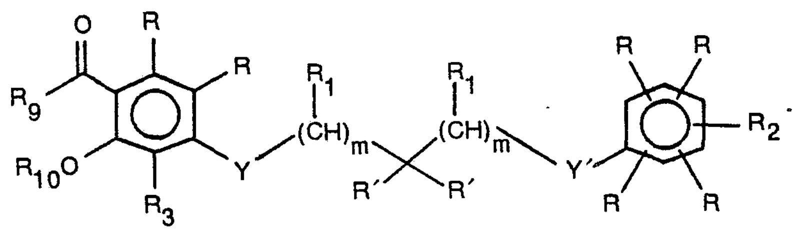

XI

and

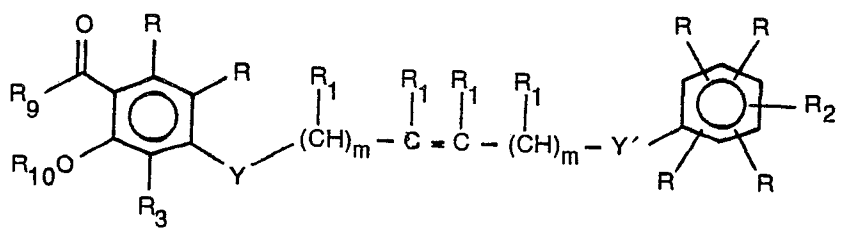

XII

wherein

each R is independently H, OH, alkyl of 1 to 6 carbon atoms which may be straight chain or branched; alkenyl of 2 to 6 carbon atoms which may be straight chain or branched; trifluoromethyl; alkoxy of 1 to 6 carbon atoms which may be straight chain or branched; SH; thioalkyl of 1 to 6 carbon atoms which may be straight chain or branched; phenyl; phenyl substituted by alkyl of 1 to 3 carbon atoms or by halogen; benzyl; phenalkyl with from 2 to 4 alkyl carbon atoms; halogen, amino; $N(R_4)_2$ wherein $R_4$ is H or alkyl of 1 to 6 carbon atoms which may be straight chain or branched; $COOR_4$; $CH_2OR_4$; formyl; CN; trifluoromethylthio; or nitro;

each R′ is independently $R_4$; $OR_4$; $COOR_4$; $N(R_4)_2$; $SR_4$; $CH_2OR_4$; CHO; or together R′ and R′ are O, $CH_2$ or

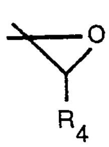 ;

Y is oxygen, sulfur, sulfoxide, sulfone;

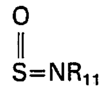

wherein $R_{11}$ is alkyl of 1—4 carbon atoms which may be straight chain or branched; $NR_{12}$ wherein $R_{12}$ is H, alkyl of 1—4 carbon atoms, which may be straight chain or branched, or

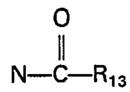

wherein $R_{13}$ is alkyl or 1—4 carbon atoms, which may be straight chain or branched, alkoxy of 1—4 carbon atoms, which may be straight chain or branched; or N—CN;

Y′ is Y, $—CH_2—$ or

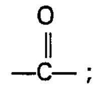

each $R_1$ is independently hydrogen or alkyl of 1—3 carbon atoms;

each m is independently an integer from 0—6; and $R_2$ is

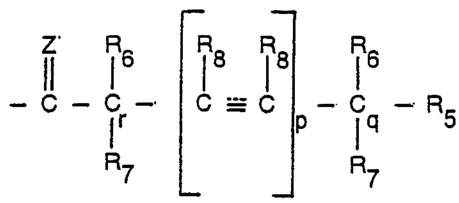

wherein

Z is O, S, $CH_2$, H and OH, alkenyl of 1—4 carbons; or $N—R_{14}$ wherein $R_{14}$ is OH, alkyl or alkoxy of 1 to 6 carbon atoms, perhaloalkyl of 1 to 6 carbon atoms, phenyl or phenyl substituted by alkyl or alkoxy groups of 1 to 3 carbon atoms, halogen, hydroxy, haloalkyl, COOH, CN, formyl or acyl of 1 to 6 carbon atoms;

each $R_6$ is independently H or alkyl of 1—4 carbons;

each $R_7$ is independently H, OH, or alkyl of 1—4 carbons;

each $R_8$ is independently H, or alkyl of 1—4 carbons, and is absent when a triple bond is present;

$R_5$ is $COOR_4$; $CH_2OH$; CHO; tetrazolyl; $NHSO_2R_{14}$; $CONHSO_2R_{14}$; hydroxymethylketone; CN; $CON(R_7)_2$; a monocyclic or bicyclic heterocyclic ring containing an acidic hydroxyl group; or $COOR_{15}$ where $R_{15}$ is:

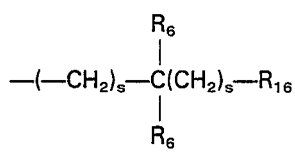

wherein each s is independently 0—3; $R_{16}$ is

A) a monocyclic or bicyclic heterocyclic radical containing from 3 to 12 nuclear carbon atoms and 1 to 2 nuclear heteroatoms selected from N and S with at least one being N, and with each ring in the heterocyclic radical being formed of 5 or 6 atoms, or

B) the radical $W—R_{17}$ wherein W is O, S or NH and $R_{17}$ contains up to 21 carbon atoms and is (1) a hydrocarbon radical or (2) an acyl radical of an organic acyclic or monocyclic carboxylic acid containing not more than 1 heteroatom in the ring;

r and q are each independently 0—20 provided that the total of r and q does not exceed 20; and

p is 0 or 1;

$R_3$ is alkyl of 1 to 6 carbon atoms which may be straight chain or branched; or alkenyl of 3 to 6 carbon atoms which may be straight chain or branched, e.g. as illustrated in Formulae IV and V;

$R_9$ is alkyl of 1 to 6 carbon atoms which may be straight chain or branched; alkoxy of 1 to 6 carbon atoms which may be straight chain or branched; or $(CH_2)_rR_5$;

$R_{10}$ is H; alkyl of 1 to 6 carbon atoms which may be straight chain or branched;

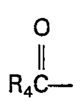 or $R_4OCH_2—$ ;

or are lactones thereof when, in $R_2$, Z is H and OH or $R_7$ is OH and $R_5$ is carboxy, or are pharmaceutically acceptable salts or acid-addition salts thereof.

As used herein, the terms "each independently" or the equivalents thereof are used to describe a number of possible position isomers and/or structural variations. For example, as described above, $R_2$ is:

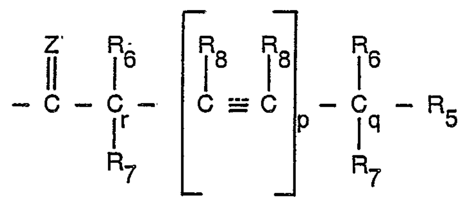

The letters r and q represent possible alkane chains of from 0—20 carbon atoms, each having the $R_6$ and $R_7$ substituent groups. On each carbon atom of the alkane chain, the $R_6$ and/or $R_7$ substituent may be different. The above description therefore contemplates structures such as the following for the segments $—(CH_6R_7)_r—$ and $—(CR_6R_7)_q—$;

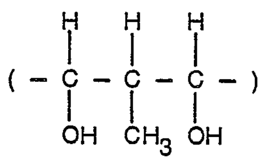 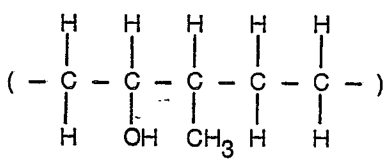 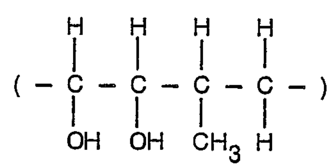

and the like.

The compounds of the present invention may be prepared by several different routes. According to one method a compound of Formula I is reacted with an optionally alkyl substituted alkenyl halide of Formula II wherein X is halogen and each $R_6$ is independently H or alkyl or 1—4 carbon atoms to yield the corresponding 2-hydroxy-4-alkenyloxy-acetophenone of Formula III. The compound of Formula III is then subjected to a Claisen rearrangement to yield a 2,4-dihydroxy-3-alkenyl-acetophenone compound of Formula IV. This rearrangement occurs on heating the compound of Formula III either neat or in a high boiling solvent, such as a halogenated hydrocarbon, e.g., dichlorobenzene, at from about 160° to about 210°C. The double bond in the compound of Formula IV may then be reduced, e.g., by catalytic hydrogenation with a catalyst such as Pd/C, to yield the corresponding saturated compound of Formula V.

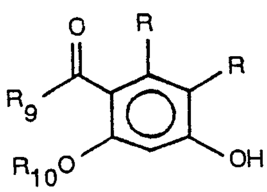

I

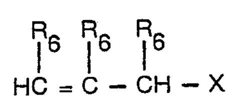

II

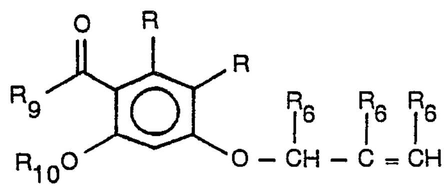

III

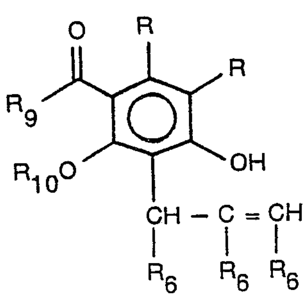

IV

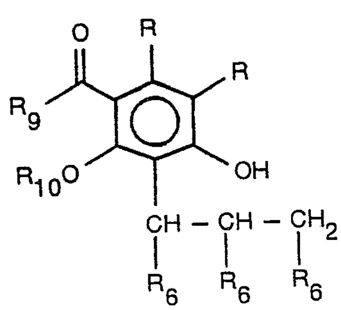

V

The compound of Formula V is then reacted with a dihaloalkane of Formula VIa or a dihaloalkene of Formula VIb wherein X, R and m have the meaning given previously, to yield a 4-(haloalkyloxy)-3-alkyl-2-hydroxyacetophenone compound of Formula VII. The reaction takes place by refluxing a mixture of the compounds of Formulae V and VIa or VIb in an inert solvent such as, for example, methylethylketone (MEK), acetone, tetrahydrofuran (THF), triglyme or dichloromethane in the presence of a base. The reflux temperature is preferably in the range of from 60 to 130°C. The base may be an alkali metal carbonate, for example, $Li_2CO_3$, $Na_2CO_3$ or $K_2CO_3$.

Specific examples of dihaloalkane compounds of Formula VIa are 1,3-dibromopropane, 2-methyl-1,3-dibromopropane, 2,2-dimethyl-1,3-dibromopropane, 3-chloro-2-2-chloromethyl-1-propene, 1,3-dibromobutane, 1,4-dibromobutane, 1,5-dibromopentane, 1,6-dibromohexane, 1,7-dibromoheptane, 1,8-dibromooctane, 1,9-dibromononane, 1,10-dibromodecane, and 1,12-dibromododecane. A specific example of a dihaloalkene compound of Formula VIb is 1,4-dibromo-2-butene.

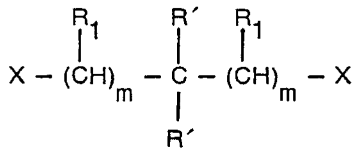

VIa

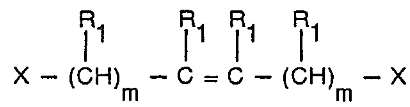

VIb

VII 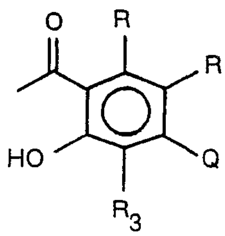 wherein Q is

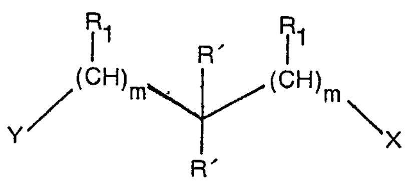

or

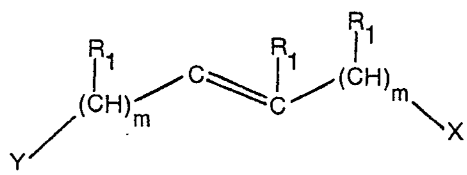

In lieu of the dihaloalkane or dihaloalkene, the compound of Formula IV or V may be reacted with an epihalohydrin, e.g., epichlorohydrin, under the same conditions to yield the 4-(2,3-epoxypropyloxy)-3-alkyl or 3-alkenyl-2-hydroxy-acetophenone compound of Formula VIII.

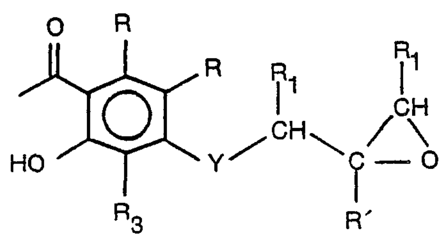

VIII

An alternative procedure is to react a compound of Formula II with a compound of Formula V to yield a 4-alkenyloxy-3-alkyl-2-hydroxy-acetophenone compound of Formula IX wich is then epoxidized with an organic peracid such as, for example, m-chloroperbenzoic acid to give the compound of Formula VIII.

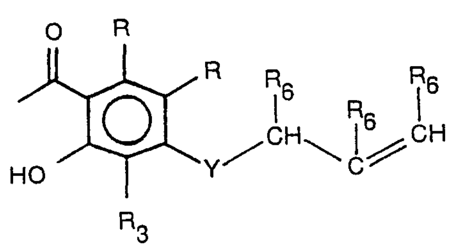

IX

The reaction of a compound of Formula VII with a compound of Formula X, under the same conditions used to react a compound of Formula V with a compound of Formula VIa or VIb, gives compounds of Formulae XI or XII.

The reaction of a compound of Formula VIII with a compound of Formula X under the same conditions used to react a compound of Formula V with a compound of Formula VIa or VIb gives a compound of Formula XI wherein each m is 1.

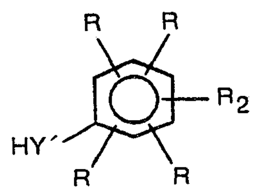

X

When Y' is —$CH_2$—, HY' is methyl. When Y' is

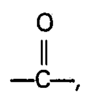

HY' is a protected aldehyde, such as, for example, a dithioacetal.

When Y' is methylene or carbonyl a stronger base such as, for example, lithium diisopropylamide or butyl lithium is employed in an inert solvent such as tetrahydrofuran with a suitable compound of Formula X.

A compound of Formula X is prepared by subjecting a compound of the Formula XIII:

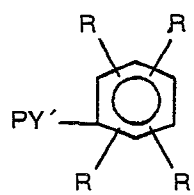

XIII

wherein P is H or a protecting group such as, for example, methyl, benzyl, *p*-nitrobenzyl or 3-(*m*-nitrophenyl)-1-phenyl-1-oxo-3-propyl to a Friedel-Crafts reaction with an acyl halide or an acid anhydride. Other compounds of Formulae XI and XII are obtained by reacting a compound of Formula VII with a compound of Formula X under the same conditions used to react a compound of Formula V with a compound of Formula VIa or VIb.

Alternatively, the compounds of Formulae XI and XII may be prepared by reacting a compound of Formula X with a compound of Formula VIa or VIb or an epihalohydrin under the same conditions as described for the preparation of the compound of Formula VII to give a compound of the Formula XIVa, XIVb or XIVc respectively. The latter are then reacted under the same conditions with a compound of Formula V to give compounds of Formulae XI or XII.

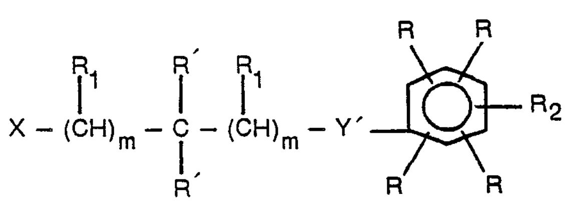

XIVa

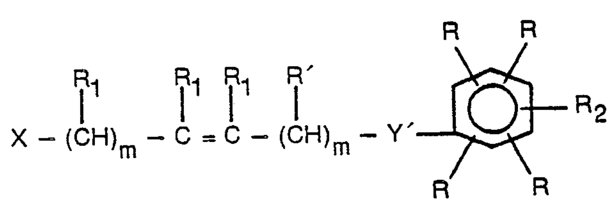

XIVb

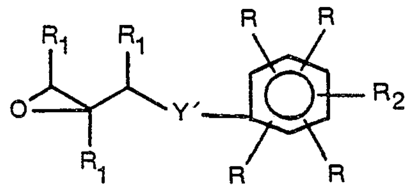

XIVc

Alternatively, but less preferably, the compounds of Formulae IV or V, X and either VIa, VIb or epihalohydrin may be reacted simultaneously under the conditions described above for reacting a compound of Formula VIa or VIb or epihalohydrin with a compound of formula X.

Prodrug ester derivatives of the compounds of Formulae XI and XII may be prepared using conventional synthetic techniques available to the skilled artisan. For example, compounds of the Formula XV:

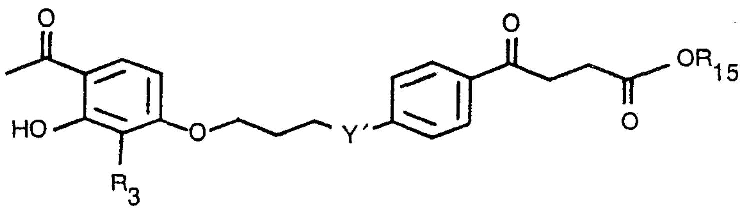

XV

may be prepared as follows:

METHOD A

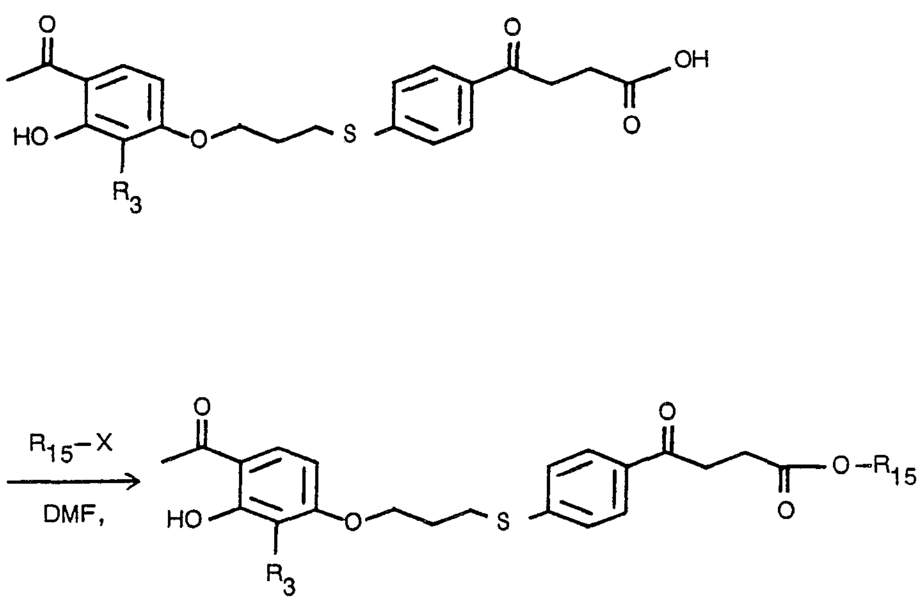


XVI

In Method A, the carboxylic acid of Formula XVI is reacted, in the presence of a base with an alkylhalide compound to provide the prodrug ester.

METHOD B

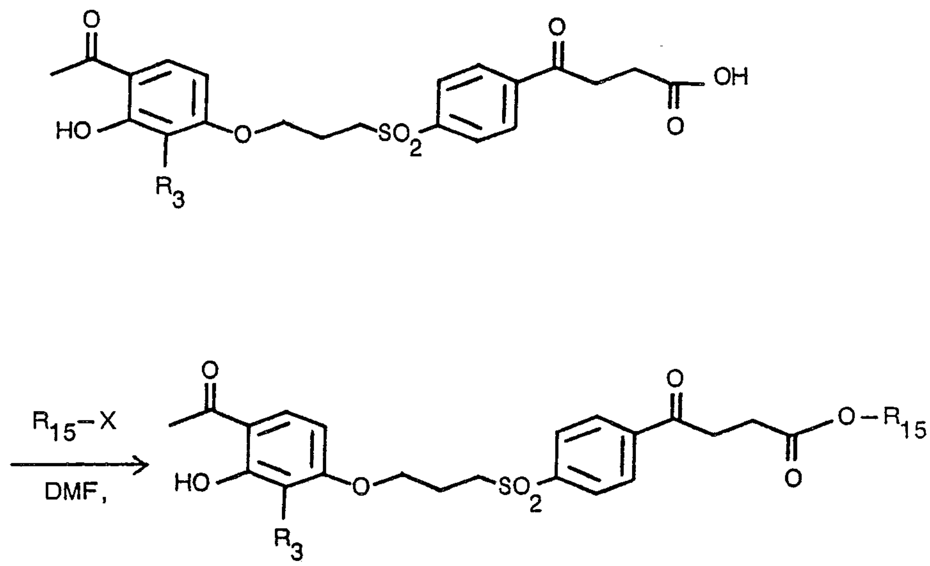


XVII

In Method B, the sulfone carboxylic acid of Formula XVII is similarly reacted with an appropriate alkylhalide, in the presence of base, to provide the corresponding prodrug ester.

METHOD C

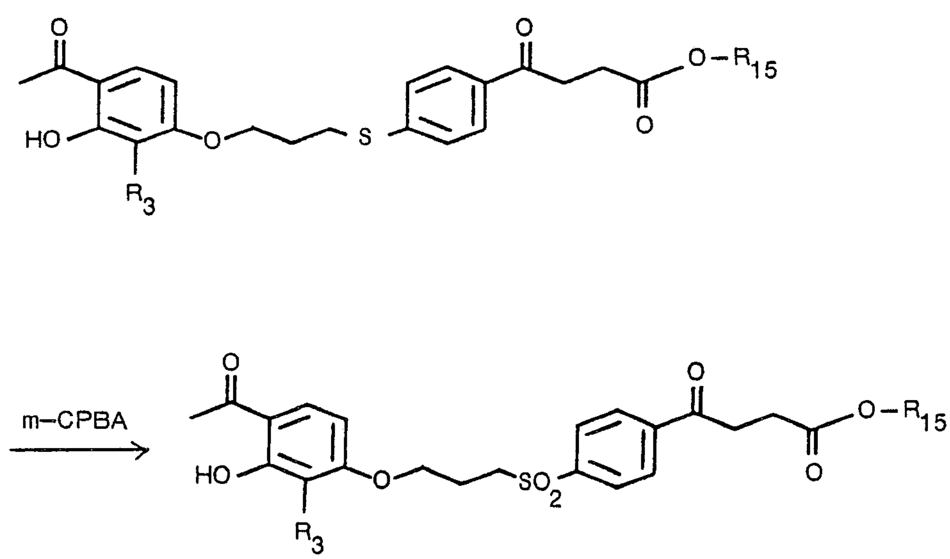


In Method C, the product of the Method A reaction, a prodrug ester, may be selectively oxidized to yield the sulfone or the sulfoxide compound.

In structures XI or XII, if Z=H+OH or $R_7$=OH and $R_5$=COOH, a lactone ring may be formed which would act as a prodrug form of the hydroxyacid. For example, a dehydration reaction of a compound having the Formula XVIII:

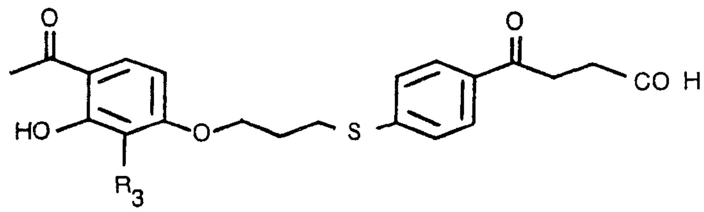

XVIII

such as, by reaction with trifluoroacetic acid, would yield a lactone compound having the Formula XIX:

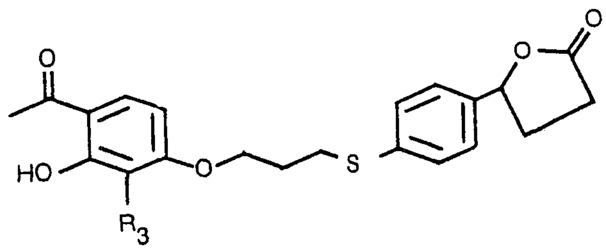

XIX

which when administered orally to a mammal would release the hydroxy acid form.

The magnitude of a prophylactic or therapeutic dose of a compound of Formula XI or XII will, of course, vary with the nature of the severity of the condition to be treated and with the particular compound of Formula XI or XII and its route of administration. In general, the daily dose range lies within the range of from about 0.2 mg to about 100 mg per kg body weight of a mammal.

The pharmaceutical compositions of the present invention comprise a compound of Formula XI or XII as an active ingredient, and may also contain a pharmaceutically acceptable carrier and optionally other therapeutic ingredients. The compositions include compositions suitable for oral, rectal, opthalmic, pulmonary, nasal, dermal, topical or parenteral (including subcutaneous, intramuscular and intravenous) administration, although the most suitable route in any given case will depend on the nature and severity of the conditions being treated and on the nature of the active ingredient. They may be conveniently presented in unit dosage form and prepared by any of the methods well-known in the art of pharmacy.

For use where a composition for intravenous administration is employed, a suitable dosage range is from about 0.1 to about 20 mg (preferably from about 0.1 to about 10 mg) of a compound of formula XI or XII per kg of body weight per day and in the case where an oral composition is employed a suitable dosage range is, e.g. from about 1 to about 100 mg of a compound of Formula XI or XII per kg of body weight per day, preferably from about 5 to about 40 mg/kg.

For administration by inhalation, the compounds of the present invention are conveniently delivered in the form of an aerosol spray presentation from pressurized packs or a nebuliser. The preferred composition for inhalation is a powder which may be formulated as a cartridge from which the powder composition may be inhaled with the aid of a suitable device. In the case of a pressurized aerosol, the dosage unit may be determined by providing a valve to deliver a metered amount.

Pharmaceutical compositions of the present invention suitable for oral administration and by inhalation in the case of asthma therapy may be presented as discrete units such as capsules, cachets or tablets each containing a predetermined amount of the active ingredient, as a powder or granules, or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or moulding, optionally with one or more accessory ingredients. Compressed tablets may be prepared by compressing in a suitable machine, the active ingredient in a free-flowing form such as powder or granules, optionally mixed with a binder, lubricant, inert diluent, lubricating, surface active or dispersing agent. Moulded tablets may be made by moulding in a suitable machine, a mixture of the powdered compound moistened with an inert liquid diluent. Desirably, each tablet contains from 25 mg to 500 mg of the active ingredient and each cachet or capsule contains from 25 to 500 mg of the active ingredient.

In addition to the Compounds of Formulae XI and XII the pharmaceutical compositions can also contain other active ingredients, such as non-steroidal anti-inflammatory agents such as non-steroidal anti-inflammatory agents e.g. indomethacin, ibuprofen, sulindac and fenbufen, peripheral analgesic agents such as zomepirac and diflunisal, and cyclooxygenase inhibitors. They may also contain inhibitors of the biosynthesis of the leukotrienes such as are disclosed copending Case 16876, U.S.S.N. 459,924, filed January 21, 1983 which is herein incorporated by reference and others known in the art. These pharmaceutical compositions may also contain antihistaminic agents such as benadryl, dramamine, histadyl, phenergan and the like. These pharmaceutical compositions containing Formulae XI or XII compounds and a second active ingredient are another embodiment of the invention. The weight ratio of the Formula XI or XII compound: second active ingredient may be varied and may range from 10:1 to 1:50.

ASSAY

Inhibition of $LTD_4$-Induced Bronchoconstriction in Artifically Ventilated Anesthetized Guinea Pigs

SPECIES: Guinea Pigs, male, 500 g (approx.), from Armand Frappier Inst.

METHOD: Guinea pigs were anesthetized with 1.5 g/kg of urethane given i.p. Fifteen minutes later, the jugular vein, carotid artery and trachea were surgically cannulated. After surgery, the animals received a subcutaneous dose of 0.1 ml of succinylcholine chloride (2 mg/animal). When breathing became shallow, the guinea pig was connected to the respirator. Respriratory volume was adjusted to produce a tracheal pressure of 10 cm of $H_2O$ (10 mm deflection on the chart). Electrodes were place for recording heart rate and the cannulated artery was connected to an appropriate transducer to monitor blood pressure.

The animal was left to stabilize for 15 minutes before administration of mediators.

Schedule of Treatments for:

1) *$LTD_4$ Dose Response Curve*

After leaving the animal to stabilize for 15 minutes, the lowest dose of $LTD_4$ was injected. If there was no response, the next higher dose of $LTD_4$ was injected after 15 minutes. When there was an increase in tracheal pressure following administration of $LTD_4$, the animal was hyperinflated by closing the outlet port of the respirator for 3 cycles. The hyperinflation maneuver reduced the tracheal pressure and facilitated the restoration of baseline values. The animal was hyperinflated at intervals of 7.5 minutes and successively increasing doses of $LTD_4$ were administered at 30 minute intervals. The dose response effects of $LTD_4$ on tracheal pressure were evaluated usng doubling doses of $LTD_4$ from 0.02—4.0 μg/kg. Each dose of $LTD_4$ was injected i.v. in 0.2 ml of saline followed by 0.2 ml of saline to flush the mediator through the tubing and cannula.

An appropriate amount of $LTD_4$ to give significant and reproducible increases in tracheal pressure was selected from the dose response curve. This dose was used to evaluate inhibitors, as described below. For the conditions and guinea pigs described above, 0.5 μg/kg of $LTD_4$ provided an appropriate dose.

A similar procedure was used for determining the dose response effects of other mediators on tracheal pressure. For example, histamine was administered in a range from 0.1—10 μg/kg.

2) *Evaluation of Compounds — $LTD_4$ Antagonists*

Each compound was evaluated against a dose of 0.5 μg/kg of $LTD_4$. Before testing the compound, the guinea pig was challenged until a constant increase in tracheal pressure was obtained from the administration of three successive doses of $LTD_4$. The guinea pig was hyperinflated as described between each dose of $LTD_4$. The compound was administered in 0.2 ml saline (or other solvent) and flushed in with 0.2 ml saline 2 minutes prior to the administration of $LTD_4$. The inhibition due to the compound was determined by the decrease elevation of tracheal pressure in comparison with controls.

By measuring the inhibition of $LTD_4$-induced elevation of tracheal pressure with increasing doses of the test compound, the dose which caused 50% inhibition could be obtained by linear regression.

3) *Evaluation of Compounds — Enzyme Inhibitors*

Compounds of this type were evaluated in a protocol similar to 2 above except they were administered 15 minutes prior to $LTD_4$.

Representative compounds of the Formulae XI and XII were tested for their ability to aleviate leukotriene $D_4$ induced bronchoconstriction in guinea pigs (i.v.). The median effective dose ($ED_{50}$) for these compounds are:

| | |
|---|---|
| Compound of Example 74B | 1 mg/kg |
| Compound of Example 74C | 0.21 mg/kg |
| Compound of Example 78 | 0.20 mg/kg |
| Compound of Example 82 | 1.45 mg/kg |

The following examples illustrate the present invention without, however, limiting it. All temperatures are expressed in degrees Celsius.

Example 1

4-((3-(4-Acetyl-3-hydroxy-2-propylphenoxy)-2-hydroxypropyl)thio)-gamma-oxobenzenebutanoic acid

A. Preparation of 4-methoxy-gamma-oxobenzenebutanic Acid

Anisole (70.0 g) and succinic anhydride (65.0 g) were dissolved in 1,2-dichloroethane (1 liter) and the mixture was cooled to 0°C. To the resulting suspension there was added, in portions, $AlCl_3$ (172 g) and the resulting mixture was stirred with a mechanical stirrer for 1 hour. The mixture was then poured into a mixture of ice and water (about 1 liter) containing 50 ml of concentrated HCl. The resulting white solid was collected by filtration, washed with water and air-dried to yield 4-methoxy-gamma-oxobenzenebutanoic acid, mp 145—147°C.

B. Preparation of 4-hydroxy-gamma-oxobenzenebutanoic acid, methyl ester

A mixture of the compound from Step A (77.3 g), 48% HBr (310 ml), and acetic acid (620 ml) was heated under reflux for 18 hours. The resulting mixture was cooled to room temperature and poured into 3 liters of water. The resulting solution was extracted with ethyl acetate (3 × 500 ml). The combined organic layers were washed with water (4 × 200 ml), dried over $Na_2SO_4$, the solvents were removed by evaporation and the residue was dissolved in 10% HCl/methanol (500 ml). After 1 hour at room temperature the volatile components were removed by evaporation *in vacuo*. The resulting residue was triturated with hexane to yield the title compound, mp 115—116°.

C. Preparation of 4-dimethylthiocarbamoyloxy-gamma-oxobenzenebutanoic acid, methyl ester

A solution of the product from Step B, 25 g, in anhydrous dimethylformamide (DMF) (300 ml) was cooled to 0° and 99% NaH, 3.46 g, was added in two portions. The mixture was stirred for 1 hour at 0° then dimethylthiocarbamoyl chloride, 19.3 g, was added and the mixture heated at 90° under a $N_2$ atmosphere for 1.5 hours. The mixture was cooled to room temperature and diluted with water to 1,200 ml. The resulting solution was then extracted with ethyl acetate (3 × 500 ml). The combined organic layers were washed with brine and then dried over $Na_2SO_4$ and evaporated to dryness *in vacuo* to yield a residue which was purified by chromatography on silica gel to yield the title compound, mp 62—64°.

D. Preparation of 4-dimethylcarbamoylthio-gamma-oxobenzenebutanoic acid, methyl ester

The compound from Step C, 29.6 g, was heated at 200° for 10 hours under an $N_2$ atmosphere. The mixture was cooled to room temperature, dissolved in methylene chloride and purified by chromatography on silica gel to provide the title compound, mp 98—100°.

E. Preparation of 4-mercapto-gamma-oxobenzenebutanoic acid, methyl ester

Sodium 280 mg, was dissolved in anhydrous methanol, 50 ml, under an $N_2$ atmosphere. To the resulting solution there was added 5.0 g of the compound from Step D. The mixture was stirred at room temperature overnight, then poured into a mixture containing 30 ml of water and 7 ml of concentrated HCl. The resulting yellow solid was collected by filtration, washed with water and dried in air to give the title compound, mp 83—84°.

F. Preparation of 4-((3-(4-acetyl-3-hydroxy-2-propylphenoxy)-2-hydroxypropyl)thio)-gamma-oxobenzenebutanoic acid

2-Hydroxy-3-propyl-4-(2,3-epoxypropoxy)-acetophenone (0.6 g, 2.4 mmole) was refluxed in 15 ml of methylethyl ketone with the product from step E (0.54 g, 2.4 mmole) for two days in the presence of 1.0 equivalent of $K_2CO_3$. The product was purified by chromatography on silica gel. The product, 0.9 g was saponified with KOH (1.5 equivalents) in a 25 ml mixture of methanol and water (10:1). The volatile components were removed under vacuum. The residue was taken up in water and acidified with citric acid. The aqueous phase was extracted twice with ethyl acetate, the organic layer was washed with brine, dried ($Na_2SO_4$) and evaporated to dryness to leave the acid residue which was triturated in hexane and filtered.

Analysis, *Calculated*: C, 62.18; H, 6.74.
*Observed*: C, 62.14; H, 6.32.

Example 2

4-((3-(4-Acetyl-3-hydroxy-2-propylphenoxy)-2-hydroxypropyl)thio)-2-fluoro-gamma-oxobenzenebutanoic acid

Following the procedure of Example 1 but substituting an equivalent amount of 3-fluoroanisole for anisole in Step A, the title compound was obtained, mp 136—137°.

Example 3

4-((3-(4-Acetyl-3-hydroxy-2-propylphenoxy)-2-hydroxypropyl)thio)-3-fluoro-gamma-oxobenzenebutanoic acid

Following the procedure of Example 1 but substituting an equivalent amount of 2-fluoroanisole for anisole in Step A, the title compound was obtained, m.p. 106—109°C.

Example 4

Methyl 4-((3-(4-acetyl-3-hydroxy-2-propylphenoxy)-2-hydroxypropyl)thio)-2-hydroxy-gamma-oxobenzenebutanoate

A. Preparation of 3-(3-nitrophenyl)-3-(3-methoxyphenylthio)-1-phenyl-1-propanone

Piperidine, 1.2 ml, was added to a boiling solution of 3-nitrobenzalacetophenone (10 g, 0.04 mole) and 3-methoxybenzenethiol (6.64 g, 0.048 mole) in 100 ml benzene. After standing for 30 minutes without further heating, acetic acid, 10 ml, was added. The solution then was poured into water (about 100 ml), extracted with methylene chloride (5 × 100 ml), washed with water, dried ($Na_2SO_4$) and evaporated to dryness to yield the title compound as a solid, mp 105°.

B. Preparation of 4-(3-phenyl-3-oxo-1-(3-nitrophenyl)-1-propylthio)-2-hydroxy-gamma-oxobenzenebutanoic acid, methyl ester

Succinic anhydride (11.45 g, 0.11 mole) was dissolved in 1,2-dichloroethane (300 ml) and $AlCl_3$ (50.76 g, 0.38 mole) was added under $N_2$. The resulting mixture was stirred for 3 hours at room temperature, cooled to 0°C and the compound from Step A (13 g, 0.03 mole), dissolved in 1,2-dichloroethane, was added slowly. The reaction mixture was kept at 5°C for 3 days, then poured on ice, stirred for one hour and the two phases separated by decanting. The aqueous phase was extracted several times with $CH_2Cl_2$ (600 ml total). The organic phases were combined, washed with water, dried ($MgSO_4$) and evaporated to dryness to yield the title compound as the free acid which then was converted to the methyl ester in conventional manner by treating the acid with methanol and anhydrous HCl. The ester had the following analysis: calculated: C, 63.27; H, 4.70; S, 6.50; observed: C, 63.19; H, 4.81; S, 6.63.

C. Preparation of 4-mercapto-2-hydroxy-gamma-oxobenzenebutanoic acid, methyl ester

To a well stirred solution of the ester from Step B (1.0 g, 2.02 mmoles) in a mixture of $CHCl_3$ (20 ml) and $C_2H_5OH$ (20 ml) was added lead acetate trihydrate (6 equivalents). After stirring the solution for 15 minutes, 10% NaOH was added to maintain the pH at 9—10. A yellow precipitate formed. After stirring for three hours, the suspension was filtered and washed with $CHCl_3$. The solid was taken up in methanolic HCl (10 ml), stirred for one hour and evaporated to dryness. The residue was taken up in ethyl acetate, the white solid was filtered off and the filtrate evaporated to dryness to yield the title compound calculated: C, 54.98, H, 5.04, S, 13.35; observed: C, 55.10, H, 4.94, S, 13.21.

D. Preparation of methyl 4-((3-(4-acetyl-3-hydroxy-2-propylphenoxy)-2-hydroxypropyl)thio)-2-hydroxy-gamma-oxobenzenebutanoate

Following the procedure of Step F of the Example 1 but substituting an equivalent amount of the ester from Step C above for 4-mercapto-gamma-oxobenzenebutanoic acid, methyl ester, the title compound was obtained.

| | | |
|---|---|---|
| Analysis, *calculated*: | C, 61.21; | H, 6.16. |
| *Observed*: | C, 61.13; | H, 6.30. |

Example 5

4-((3-(4-Acetyl-3-hydroxy-2-propylphenoxy)-2-hydroxypropyl)thio)-2-hydroxy-gamma-oxobenzenebutanoic acid

The compound prepared according to Example 4 (544 mg, 1.15 mmol) was refluxed with $NaHCO_3$ (482 mg, 5.75 mmole) in a 20 ml mixture of methanol and water (3:1) for 4 hours. Then 1 N hydrochloric acid was added and the mixture was extracted with $CH_2Cl_2$, washed with water, dried ($Na_2SO_4$), and evaporated to dryness to yield a residue which was recrystallized from methanol/hexane, mp 149.5°.

| | | | |
|---|---|---|---|
| Analysis, *calculated*: | C, 62.45; | H, 6.33; | S, 6.95. |
| *Observed*: | C, 62.46; | H, 6.37; | S, 7.24. |

Example 6

4-((3-(4-Acetyl-3-hydroxy-2-propylphenoxy)propyl)thio)-gamma-oxobenzenebutanoic acid

Following the procedure of Step F of Example 1 but substituting an equivalent amount of 2-hydroxy-3-propyl-4-(3-bromopropyloxy)acetophenone for 2-hydroxy-3-propyl-4-(2,3-epoxypropoxy)-acetophenone, the title compound was obtained.

| | | | |
|---|---|---|---|
| Analysis, *calculated*: | C, 64.98; | H, 6.14; | S, 7.23. |
| *Observed*: | C, 65.09; | H, 6.09; | S, 7.28. |

Example 7

4-((3-(4-Acetyl-3-hydroxyphenoxy)propylthio)-gamma-oxobenzenebutanoic acid

Following the procedure of Step F or Example 1, but substituting 2-hydroxy-4-(3-bromopropyloxy)-acetophenone for 2-hydroxy-3-propyl-4-(2,3-epoxypropoxy)acetophenone, the title compound was obtained.

| | | | |
|---|---|---|---|
| Analysis, *calculated*: | C, 62.66; | H, 5.51; | S, 7.96. |
| *Observed*: | C, 62.72; | H, 5.64; | S, 7.90. |

Example 8

4-((3-(4-Acetyl-3-hydroxy-2-propylphenoxy)propyl)thio)-2-hydroxy-gamma-oxobenzenebutanoic acid

A. Preparation of methyl ester of 4-((3-(4-acetyl-3-hydroxy-2-propylphenoxy)propyl)thio)-2-hydroxy-gamma-oxobenzenebutanoic acid

Following the procedure of Step F of Example 1 but substituting and equivalent amount of 2-hydroxy-3-propyl-4-(3-bromopropyloxy)acetophenone for 2-hydroxy-3-propyl-4-(2,3-epoxypropoxy)acetophenone and substituting an equivalent amount of the product from Step C of Example 4 for 4-mercapto-gamma-oxobenzenebutanoic acid, methyl ester, the title compound was obtained.

Analysis, *calculated*: C, 63.27; H, 6.37; S, 6.76.
*Observed*: C, 63.30; H, 6.54; S, 6.70.

B. Preparation of 4-((3-(4-acetyl-3-hydroxy-2-propylphenoxy)propyl)thio)-2-hydroxy-gamma-oxobenzenebutanoic acid

By treating the product from Step A of this example according to the procedure of Example 5, the title compound was obtained, mp 140°C.

Analysis, *calculated*: C, 62.59; H, 6.13; S, 6.96.
*Observed*: C, 62.34; H, 6.47; S, 7.19.

Example 9

4-((3-(4-Acetyl-3-hydroxy-2-propylphenoxy)-2-hydroxypropyl)thio)-2-methoxy-gamma-oxobenzenebutanoic acid

A. Preparation of 4-mercapto-2-methoxy-gamma-oxobenzenebutanoic acid, methyl ester

The free acid from Step B of Example 4 was dissolved in $CH_3OH$ and treated with excess diazomethane at room temperature. Volatiles were removed under vacuum. After purification on silica gel, there was obtained the methyl ester of 4-(3-phenyl-3-oxo-1-(m-nitrophenyl)-1-propylthio)-2-methoxy-gamma-oxo-benzenebutanoic acid. This ester was reacted with lead acetate trihydrate to yield 4-mercapto-2-methoxy-gamma-oxobenzenebutanoic acid. The methyl ester of the acid was obtained in conventional manner by treatment with methanolic HCl.

B. Preparation of 4-((3-(4-acetyl-3-hydroxy-2-propylphenoxy)-2-hydroxypropyl)thio)-2-methoxy-gamma-oxobenzenebutanoic acid

Following the procedure of Step F of Example 1 but substituting an equivalent amount of the ester from Step A of this example for 4-mercapto-gamma-oxobenzenebutanoic acid, methyl ester, the title compound was obtained.

Analysis, *calculated*: C, 61.20; H, 6.16; S, 6.53.
*Observed*: C, 61.14; H, 6.26; S, 6.51.

Example 10

4-((3-(4-Acetyl-3-hydroxy-2-propylphenoxy)propyl)thio)-3-fluoro-gamma-oxobenzenebutanoic acid

Following the procedure of Example 1 but substituting an equivalent amount of 2-fluoroanisole for anisole in Step A and substituting an equivalent amount of 2-hydroxy-3-propyl-4-(3-bromopropoxy)acetophenone for 2-hydroxy-3-propyl-4-(2,3-epoxypropoxy)acetophenone in Step F, the title compound was obtained, m.p. 115—116°C.

Example 11

4-((3-(4-Acetyl-3-hydroxy-2-propylphenoxy)propyl)thio)-2-fluoro-gamma-oxobenzenebutanoic acid

Following the procedure of Example 10 but substituting 3-fluoroanisole for 2-fluoroanisole, the title compound was obtained, mp 149—150°C.

Example 12

4-(3-(4-Acetyl-3-hydroxy-2-propylphenoxy)propoxy)-3-fluoro-gamma-oxobenzenebutanoic acid

A. Preparation of 4-Hydroxy-3-fluoro-gamma-oxobenzenebutanoic acid, methyl ester

To a solution of 2-fluorophenol (22.4 g, 200 mmole) in 1,2-dichloroethane (250 ml) at 0° there was added $AlCl_3$ (54 g, 400 mmole) and then succinic anhydride (20 g, 200 mmole). The reaction mixture was heated at 85°C for 18 hours, then cooled, poured on ice and stirred for several hours at room temperature. The layers were separated and the aqueous layers extracted with $CH_2Cl_2$. The combined extracts were washed with water, dried ($MgSO_4$) and evaporated to a semi-solid which was taken up in a solution of methanolic HCl (500 ml, approximately 3N). After standing for 24 hours the solution was poured into water, extracted with $CH_2Cl_2$, washed with water, dried and evaporated to a small volume. The crude product was purified by chromatography on silica gel to yield the title compound.

Analysis, *calculated*: C, 58.41; H, 4.91; F, 8.40.
*Observed*: C, 58.63; H, 4.96; F, 8.14.

Its isomer, 2-hydroxy-3-fluoro-gamma-oxobenzenebutanoic acid, methyl ester, was also isolated in the chromatographic step.

Analysis, *calculated*: C, 58.41; H, 4.91; F, 8.40.
*Observed*: C, 58.53; H, 4.82; F, 8.10.

B. Preparation of 4-(3-(4-acetyl-3-hydroxy-2-propylphenoxy)propoxy)-3-fluoro-gamma-oxobenzenebutanoic acid

Following the procedure of Example 6 but substituting the title ester from Step A of this example for 4-mercapto-gamma-oxobenzenebutanoic acid, methyl ester, the title compound was obtained, m.p. 142—144°C.

| | | | |
|---|---|---|---|
| Analysis, *calculated*: | C, 64.56; | H, 6.10; | F, 4.26. |
| *Observed*: | C, 64.74; | H, 6.15; | F, 4.55. |

C. Preparation of 2-(3-(4-acetyl-3-hydroxy-2-propylphenoxy)propoxy)-3-fluoro-gamma-oxobenzenebutanoic acid

Following the procedure of Step B of this example but substituting 2-hydroxy-3-fluoro-gamma-oxo-benzenebutanoic acid, methyl ester for 4-hydroxy-3-fluoro-gamma-oxobenzenebutanoic acid, methyl ester, the title compound was obtained.

| | | | |
|---|---|---|---|
| Analysis, *calculated*: | C, 64.56; | H, 6.10; | F, 4.26. |
| *Observed*: | C, 64.41; | H, 6.22; | F, 4.24. |

Example 13

4-(3-(4-Acetyl-3-hydroxy-2-propylphenoxy)propoxy)-3-fluoro-epsilon-oxobenzenehexanoic acid

A. Preparation of 4-hydroxy-3-fluoro-epsilon-oxobenzenehexanoic acid, methyl ether

Following the procedure of Example 12 but adding $TiCl_4$ (2.5 ml, 21.8 mmole) at −15°C in lieu of $AlCl_3$ in Step A and substituting methyl 5-chloroformylpentanoate (3.57 g, 20 mmole) for succinic anhydride, and then heating the reaction mixture to 90°C for 3 hours, the title compound was obtained.

| | | | |
|---|---|---|---|
| Analysis, *calculated*: | C, 61.41; | H, 5.95; | F, 7.47. |
| *Observed*: | C, 61.51; | H, 6.14; | F, 7.08. |

Its isomer, 2-hydroxy-3-fluoro- -epsilon-oxobenzenehexanoic acid, methyl ester, was also obtained, m.p. 59—60°C.

| | | | |
|---|---|---|---|
| Analysis, *calculated*: | C, 61.41; | H, 5.95; | F, 7.47. |
| *Observed*: | C, 61.58; | H, 6.05; | F, 7.17. |

B. Preparation of 4-(3-(4-acetyl-3-hydroxy-2-propylphenoxy)propoxy)-3-fluoro-epsilon-oxobenzenehexanoic acid

Following the procedure of Step B of Example 12, but substituting the title ester from Step A of this example for 4-hydroxy-3-fluoro-gamma-oxobenzenebutanoic acid, methyl ester, the title compound was obtained, m.p. 106—107°C.

| | | | |
|---|---|---|---|
| Analysis, *calculated*: | C, 65.81; | H, 6.59; | F, 4.00. |
| *Observed*: | C, 65.90; | H, 6.18; | F, 3.71. |

C. Preparation of 2-(3-(4-Acetyl-3-hydroxy-2-propylphenoxy)propoxy)-3-fluoro-epsilon-oxobenzenehexanoic acid

Following the procedure of step B of this example but substituting 2-hydroxy-3-fluoro-epsilon-oxobenzenehexanoic acid, methyl ester for the title compound of Step A of this example, the title compound was obtained.

| | | | |
|---|---|---|---|
| Analysis, *calculated*: | C, 65.81; | H, 6.59; | F, 4.00. |
| *Observed*: | C, 65.90; | H, 6.13; | F, 3.71. |

Example 14

4-(3-(4-Acetyl-3-hydroxy-2-propylphenoxy)propoxy)-2-fluoro-gamma-oxobenzenebutanoic acid

Following the procedure of Steps A and B Example 12 but substituting an equivalent amount of 3-fluorophenol for 2-fluorophenol in Step A, the title compound was obtained, m.p. 113—118°C.

Example 15

4-(3-(4-Acetyl-3-hydroxy-2-propylphenoxy)propoxy)-gamma-oxobenzenebutanoic acid

By reacting the compound of Step B of Example 1 with 2-hydroxy-3-propyl-4-(3-bromopropyloxy)-acetophenone according to the procedure of Step F of Example 1, the title compound was obtained.

| | | |
|---|---|---|
| Analysis, *calculated*: | C, 67.27; | H, 6.58. |
| *Observed*: | C, 67.30; | H, 6.56. |

Example 16

4-(3-(4-Acetyl-3-hydroxy-2-propylphenoxy)propoxy)-2,3-dichloro-gamma-oxobenzenebutanoic acid

Following the procedure of Steps A and B of Example 1, but substituting 2,3-dichloroanisole for anisole in Step A, there was obtained 2,3-dichloro-4-hydroxy-gamma-oxobenzenebutanoic acid, methyl ester. The ester was reacted with 2-hydroxy-3-propyl-4-(3-bromopropyloxy)acetophenone according to the procedure of Step F of Example 1 to give the title compound, mp 119—123°C.

Example 17

4-(3-(4-Acetyl-3-hydroxy-2-propylphenoxy)propoxy)-3-methyl-gamma-oxobenzenebutanoic acid

Following the procedure of Example 16, but substituting 2-methyl anisole for 2,3-dichloroanisole there was obtained 3-methyl-4-hydroxy-gamma-oxobenzenebutanoic acid, methyl. ester. The ester then was reacted with 2-hydroxy-3-propyl-4-(3-bromopropyloxy)acetophenone according to the procedure of Step F of Example 1 to give the title compound, mp 179—180°C.

| | | | |
|---|---|---|---|
| Analysis, *calculated*: | C, 67.86; | H, 6.83. | |
| *Observed*: | C, 67.83; | H, 6.80. | |

Example 18

4-(3-(4-Acetyl-3-hydroxy-2-propylphenoxy)propoxy)-2-chloro-gamma-oxobenzenebutanoic acid

Following the procedure of Example 16 but substituting 3-chloroanisole for 2,3-dichloroanisole, there was obtained 2-chloro-4-hydroxy-gamma-oxobenzenebutanoic acid, methyl ester. The ester then was reacted with 2-hydroxy-3-propyl-4-(3-bromopropyloxy)acetophenone according to the procedure of Step F of Example 1 to give the title compound, mp 143—144.5°C.

| | | | |
|---|---|---|---|
| Analysis, *calculated*: | C, 62.27; | H, 5.88; | Cl, 7.66. |
| *Observed*: | C, 62.14; | H, 5.92; | Cl, 7.89. |

Example 19

4-(4-(4-Acetyl-3-hydroxy-2-propylphenoxy)-2-butenoxy)-3-fluoro-gamma-oxobenzenebutanoic acid

Following the procedure of Step F of Example 1 but substituting 2-hydroxy-3-propyl-4-(4-chloro-2-butenoxy)acetophenone for 2-hydroxy-3-propyl-4-(2,3-epoxypropoxy)acetophenone, and substituting 4-hydroxy-3-fluoro-gamma-oxobenzenebutanoic acid, methyl ester for the methyl ester product of Step E of Example 1, the title compound was obtained, mp 170—171.5°C.

| | | | |
|---|---|---|---|
| Analysis, *calculated*: | C, 65.49; | H, 5.94; | F, 4.14. |
| *Observed*: | C, 65.58; | H, 6.02; | F, 4.22. |

Example 20

4-(3-(4-Acetyl-3-hydroxy-2-propylphenoxy)propoxy)-3-propyl-gamma-oxobenzenebutanoic acid

Following the procedure of Example 17 but substituting 2-n-propyl anisole for 2-methylanisole, the title compound was obtained.

| | | | |
|---|---|---|---|
| Analysis, *calculated*: | C, 67.27; | H, 6.58. | |
| *Observed*: | C, 67.30; | H, 6.54. | |

Example 21

4-(3-(4-Acetyl-3-hydroxy-2-propylphenoxy)-2-hydroxypropylthio)-3-fluoro-gamma-oxobenzenebutanoic acid-S-oxide

The ester prepared according to the procedure of Example 3 was dissolved in 50 mL $CH_2Cl_2$, the solution was cooled to 0° and 1 equivalent of m-chloroperbenzoic acid was added. The reaction mixture was stirred at 0°C for 5 minutes and was purified by chromatography on silica gel to yield the methyl ester of the title compound, that was saponified by stirring with sodium hydroxide in methanol to yield the title compound, m.p. 179—181°C.

Example 22

4-(3-(4-Acetyl-3-hydroxy-2-propylphenoxy)propylthio)-3-fluoro-gamma-oxobenzenebutanoic acid-S-oxide

The ester prepared according to the procedure of Example 10 was treated as in Example 21 to give, after saponification, the title compound, m.p. 135—136°C.

Example 23

4-(3-(4-Acetyl-3-hydroxy-2-propylphenoxy)propylthio)-2-fluoro-gamma-oxobenzenebutanoic acid-S-oxide

The ester prepared according to the procedure of Example 11 was treated as in Example 21 to give, after saponification, the title compound, m.p. 168—169°C.

Example 24

4-(3-(4-Acetyl-3-hydroxy-2-propylphenoxy)-2-hydroxypropylthio)-2-fluoro-gamma-oxobenzenebutanoic acid-S-oxide

The ester prepared according to the procedure of Example 2 was treated as in Example 21 to give, after saponification, the title compound, m.p. 69—72°C.

Example 25

4-(3-(4-Acetyl-3-hydroxy-2-propylphenoxy)propylsulfonyl)-2-fluoro-gamma-oxobenzenebutanoic acid

The ester prepared according to the procedure of Example 11 dissolved in 50 mL $CH_2Cl_2$ was treated at room temperature with 2.5 equivalents of m-chloroperbenzoic acid for 2 hours. The reaction mixture was purified by chromatography on silica gel to yield the methyl ester of the title compound, that then was saponified by stirring with NaOH in methanol to yield the title compound, m.p. 116—118°.

Example 26

4-(3-(4-Acetyl-3-hydroxy-2-propylphenoxy)-2-hydroxypropylsulfonyl)-2-fluoro-gamma-oxobenzenebutanoic acid

The ester prepared according to the procedure of Example 2 was treated as in Example 25 to give, after saponification, the title compound, m.p. 80—85° (decomp).

Example 27

4-(3-(4-Acetyl-3-hydroxy-2-propylphenoxy)-propylsulfonyl)-3-fluoro-gamma-oxobenzenebutanoic acid

The ester prepared according to the procedure of Example 10 was treated as in Example 25 to give, after saponification, the title compound, m.p. 154—156 °C.

Example 28

4-(3-(4-Acetyl-3-hydroxy-2-propylphenoxy)-2-hydroxypropylsulfonyl)-3-fluoro-gamma-oxobenzenebutanoic acid

The ester prepared according to the procedure of Example 3 was treated as in Example 25 to give, after saponification, the title compound, m.p. 96—99° resolidifies, 105—108°.

Example 29

4-(3-(4-Acetyl-3-hydroxy-2-propylphenoxy)propylsulfonyl)-gamma-oxobenzenebutanoic acid

The ester prepared according to the procedure of Example 6 was treated as in Example 25 to give, after saponification, the title compound.

| | | | |
|---|---|---|---|
| Analysis, *calculated*: | C, 60.48; | H, 5.92; | S, 6.72. |
| *Observed*: | C, 60.51; | H, 5.90; | S, 6.48. |

Example 30

4-(3-(4-Acetyl-3-hydroxy-2-propylphenoxy)-2-hydroxypropylsulfonyl)-gamma-oxobenzenebutanoic acid

The ester prepared according to the procedure of Example 1 was treated as in Example 25 to give, after saponification, the title compound.

| | | | |
|---|---|---|---|
| Analysis, *calculated*: | C, 59.27; | H, 5.97; | S, 6.33. |
| *Observed*: | C, 59.22; | H, 6.13; | S, 6.02. |

Example 31

4-(3-(4-Acetyl-3-hydroxy-2-propylphenoxy)propoxy)-3-chloro-gamma-oxobenzenebutanoic acid

By following the procedure of Example 12 but substituting in Step A, 2-chlorophenol for 2-fluorophenol, the title compound is obtained, m.p. 167.5—169.5°.

Example 32

4-(3-(4-Acetyl-3-hydroxy-2-propylphenoxy)propoxy)-2-hydroxypropoxy)-3-fluoro-gamma-oxobenzenebutanoic acid, sodium salt, monohydrate

Following the procedure of Step F of Example 1 but substituting an equivalent amount of 2-hydroxy-3-propyl-4-(3-bromopropyloxy)acetophenone for 2-hydroxy-3-propyl-4-(2,3-epoxypropoxy)acetophenone and substituting the product from Step A of Example 12 for 4-mercapto-gamma-oxobenzenebutanoic acid, methyl ester, the corresponding free acid of the title compound was isolated. It was converted to the sodium salt by treating with 1 equivalent of NaOH.

| | | | |
|---|---|---|---|
| Analysis, *calculated*: | C, 57.42; | H, 5.81; | F, 3.78. |
| *Observed*: | C, 57.49; | H, 5.78; | F, 4.19. |

Example 33

4-(3-(4-Acetyl-3-hydroxy-2-propylphenoxy)propoxy)-alpha,alpha-dimethyl-gamma-oxobenzenebutanoic acid

A: Preparation of 3,4-dehydro-2,2-dimethyl-4-(4-methoxyphenyl)-gamma-butyrolactone

To a stirring suspension of KH (16 mmole) in THF (50 ml) was added a solution of 4-methoxy-gamma-oxobenzenebutanoic acid (8 mmole) prepared as in Step A of Example 1 in THF (10 ml) at room temperature. The mixture was stirred at room temperature for 2 hours. Methyl iodide (32 mmole) was added and the resulting reaction mixture stirred at room temperature for 16 hours. Cold, dilute HCl solution was added and the resulting mixture extracted with ethyl acetate. The organic layer was washed successively with $NaHCO_3$ solution, brine, dried and evaporated.

The resulting oil was chromatographed on silica gel to give 3,4-dehydro-2,2-dimethyl-4-(4-methoxyphenyl)-gamma-butyrolactone.

B: Preparation of methyl 4-methoxy-alpha,alpha-dimethyl-gamma-oxobenzenebutanoate

To 3,4-dehydro-2,2-dimethyl-4(4-methoxyphenyl)-gamma-butyrolactone (1.35 mmole), prepared in Step A of this Example, in methanol (8 ml) was added 2.7 mmole of sodium methoxide. The reaction was stirred at room temperature for 1.5 hour. Cold dilute hydrochloric acid was then added, the methanol evaporated to dryness and the resulting aqueous layer extracted with ethyl acetate. The organic layer was

then washed with brine and dried over $MgSO_4$. Evaporation of the volatiles gave a quantitive yield of the title compound.

C: Preparation of 4-(3-(4-Acetyl-3-hydroxy-2-propylphenoxy)propoxy)-alpha,alpha-dimethyl-gamma-oxobenzenebutanoic acid

Following the procedure of Step B of Example 1 but substituting the product from Step B of this example for 4-methoxy-gamma-oxobenzenebutanoic acid there was obtained 4-hydroxy-alpha,alpha-dimethyl-gamma-oxobenzenebutanoic acid, methyl ester.

This ester was reacted according to the procedure of Example 6 to yield the title compound.

Analysis, *calculated*: C, 68.40; H, 7.07.
*Observed*: C, 68.54; H, 7.06.

Example 34

4-(3-(4-Acetyl-3-hydroxy-2-propylphenoxy)propoxy)-beta-methyl-gamma-oxobenzenebutanoic acid

The title compound was prepared by following the procedure of Step C of Example 33 but substituting the methyl 4-methoxy-beta-methyl-gamma-oxobenzenebutanoate (prepared as described in Step A of Example 33) for the product of Step B of Example 33.

Analysis, *calculated*: C, 67.86; H, 6.83.
*Observed*: C, 67.94; H, 6.87.

Example 35

4-(3-(4-Acetyl-3-hydroxy-2-propylphenoxy)-2-methylidenylpropoxy-gamma-oxobenzenebutanoic acid

A: Preparation of 2-Hydroxy-3-propyl-4-(3-chloro-2-methylidenylpropoxy)acetophenone

To a solution of 2,4-dihydroxy-3-propylacetophenone, 30 g, in acetone, 600 ml, were added $K_2CO_3$, 64 g, and 3-chloro-2-chloromethyl-1-propene, 54 ml. The heterogeneous purple mixture was stirred at reflux overnight. The reaction mixture was filtered, the solids were washed with acetone and the filtrate was evaporated to a red oil that was chromatographed on silica gel to yield the title compound, mp 50—52°C as a white solid.

B: Preparation of 4-(3-(4-Acetyl-3-hydroxy-2-propylphenoxy)-2-methylidenylpropoxy)-gamma-oxobenzenebutanoic acid

Following the procedure of Step F of Example 1, but substituting the title compound of Step A of this example for 2-hydroxy-3-propyl-4-(2,3-epoxypropoxy)acetophenone and substituting 4-hydroxy-gamma-oxobenzenebutanoic acid, methyl ester for 4-mercapto-gamma-oxobenzenebutanoic acid, methyl ester, the title compound was obtained, mp 136—137°C.

Analysis, *calculated*: C, 68.17; H, 6.41.
*Observed*: C, 68.10; H, 6.40.

Example 36

4-(3-(4-Acetyl-3-hydroxy-2-propylphenoxy)-2-methylpropoxy)-gamma-oxobenzenebutanoic acid

A: Preparation of 2-Hydroxy-3-propyl-4-(2-methyl-3-chloro-propoxy)acetophenone

Following the procedure of Step A of Example 35 but substituting 2-methyl-1,3-dichloropropane for 3-chloro-2-chloromethyl-1-propene, the title compound was obtained.

B: Preparation of 4-(3-(4-Acetyl-3-hydroxy-2-propylphenoxy)-2-methylpropoxy)-gamma-oxobenzenebutanoic acid

Following the procedure of Step B of Example 35, but substituting 2-hydroxy-3-propyl-4-(2-methyl-3-chloro-propoxy)acetophenone for 2-hydroxy-3-propyl-4-(3-chloro-2-methylidenylpropoxy)acetophenone, the title compound was obtained.

Analysis, *calculated*: C, 67.86; H, 6.83.
*Observed*: C, 67.62; H, 6.85.

Example 37

4-(3-(4-acetyl-3-hydroxy-2-propylphenoxy)-2-methylidenepropoxy)-gamma-oxo-benzenebutanoic acid

A: Preparation of 2-hydroxy-3-propyl-4(3-chloro-2-methylidenepropoxy)acetophenone

2,4-Dihydroxy-3-propyl-acetophenone (30 g, 15.5 mmole) was refluxed with 3-chloro-2-chloro-methyl-1-propene (54 ml, 464 mmole) in 600 ml acetone for 14 hours in the presence of 3.0 equivalents of $K_2CO_3$. The product was purified by chromatography on silica gel using hexane/EtOAc (10:1—10:5) as eluent. The title compound was obtained as a white solid, m.p. 50—52°.

B. Preparation of methyl 4-(3-(4-acetyl-3-hydroxy-2-propylphenoxy)-2-methylidenepropoxy)-gamma-oxo-benzenebutanoate

To a stirred mixture of the compound from Step A (6.22 g, 22.0 mmole) in methylethyl ketone (110 ml) was added the compound of Example 1, Step B (5.54 g, 26.6 mmole) and 3.0 equivalents of $K_2CO_3$. The mixture was heated at reflux for 30 hours. The reaction mixture was cooled to room temperature, filtered,

washed with acetone and evaporated to dryness. The residue was dissolved in methylene chloride, washed with 0.1N NaOH, and evaporated to an oil. The oil was purified by HPLC using hexane: EtOAc (10:4) to yield the title compound as a white solid, m.p. 89—90°C.

Analysis, *calculated*: C, 68.70; H, 6.65.
*Observed*: C, 68.68; H, 6.70.

C: 4-(3-(4-acetyl-3-hydroxy-2-propylphenoxy)-2-methylidenepropoxy)-gamma-oxo-benzenebutanoic acid

To a stirred solution of the compound from Step B (550 mg, 1.21 mmole) in THF (15 ml) was added 3 ml of 1N NaOH. The mixture was stirred at room temperature for 1.5 hours. The reaction mixture was diluted with $H_2O$ (50 ml) and acidified with concentrated HCl to pH=5. A white solid formed which was extracted with methylene chloride (2X). The combined organic extracts were washed with brine, dried ($MgSO_4$) and concentrated *in vacuo* to yield the title compound as a white solid, m.p. 136—137°.

Analysis, *calculated*: C, 68.17; H, 6.41.
*Observed*: C, 68.10; H, 6.40

Example 38

4-(3-(4-acetyl-3-hydroxy-2-propylphenoxy)-2-methylidenepropylthio)gamma-oxo-benzenebutanoic acid

Following the procedure of Example 37, Steps B and C but substituting an equivalent amount of the compound of Example 1, Step E (for the compound of Example 1, Step B), in Step B of Example 37, the title compound m.p. 123—125° was obtained following hydrolysis of the corresponding methyl ester.

Analysis, *calculated*: C, 65.77; H, 6.18; S, 7.02.
*Observed*: C, 65.74; H, 6.23; S, 7.23.

Example 39

4-(3-(4-acetyl-3-hydroxy-2-propylphenoxy)-2-methylidenepropoxy)-3-fluoro-gamma-oxobenzenebutanoic acid

Following the procedure of Example 37, Steps B and C, but substituting an equivalent amount of the compound of Example 12, Step A, (for the compound of Example 1, Step B) in Step B of Example 37, the title compound was obtained, m.p. 132—134°.

Analysis, *calculated*: C, 65.49; H, 5.94.
*Observed*: C, 65.48; H, 5.99.

Example 40

4-(3-(4-acetyl-4-hydroxy-2-propyl-phenoxy)propylthio)gamma-oxobenzenebutyronitrile

A: Preparation of 4-methylthio-gamma-oxobenzenebutyronitrile

A solution of 4-methylthiobenzaldehyde (15.2 g, 100 mmole) in dry DMF (50 ml) was added over 10 minutes to a solution of sodium cyanide (2.45 g, 50 mmole) in DMF (50 ml) at 35°C. After 5 minutes, acrylonitrile (4.0 g, 75 mmole) in DMF (100 ml) was added dropwise over 20 minutes. The reaction mixture stirred at 35° for 3 hours. The reaction mixture was cooled to room temperature, diluted with water (400 ml), extracted with chloroform, washed with 0.01N $H_2SO_4$, 5% $NaHCO_3$ and water, dried, and evaporated to a solid which was crystallized from ethyl acetate and hexane to yield the title compound, m.p. 122—124°.

B: Preparation of 4-methylthio-gamma-oxobenzenebutyronitrile-S-oxide

The compound of Step A above (205 mg, 1.0 mmole) was dissolved in chloroform (1 ml) and cooled to 0°. m-Chloroperbenzoic acid (200 mg, 1.1 mmole) in chloroform (1 ml) was added to the cooled solution, and the reaction mixture warmed to room temperature over 30 minutes. Calcium hydroxide (210 mg) was added and the reaction stirred 15 minutes. The solution was filtered and evaporated to dryness to yield the title compound which was used directly in the following step.

C: Preparation of 4-trifluoroacetyloxymethylthiogamma-oxo-benzenebutyronitrile

The compound of Step B above, (300 mg) was dissolved in 2.0 ml trifluoroacetic anhydride. The reaction mixture was heated at reflux for 30 minutes, cooled to room temperature, and evaporated to dryness. The title compound crystallized on standing. Mass spectrum m/e 317 ($M^+$).

D: Preparation of 4-mercapto-gamma-oxobenzenebutyronitrile

The compound of Step C above, (75 mg, 0.24 mmole) was dissolved in methanol (5 ml). To this solution was added 1N $Na_2CO_3$ (0.5 ml, 0.5 mmole) and the mixture was stirred at room temperature for 30 minutes. 6N HCl (0.2 ml) was added and the reaction mixture was extracted with chloroform, washed with brine, dried and evaporated to dryness to yield the title compound. Mass spectrum m/e 191 ($M^+$).

E: 4-(3-(4-acetyl-3-hydroxy-2-propyl-phenoxy)propylthio)gamma-oxobenzenebutyronitrile

The compound of Step D above, (2.4 g, mmole), 2-hydroxy-3-propyl-4-(3-bromopropyloxy)-acetophenone (2.92 g) and $K_2CO_3$ (4.0 g); were dissolved in methylethyl ketone (50 ml). The reaction mixture was heated at reflux for 90 minutes. The reaction mixture was poured into water, extracted with chloroform, washed with brine, dried and evaporated to yield the title compound. Recrystallization from

ether:hexane (1:1) afforded the title compound as needles, m.p., 94—95°.

Analysis, *calculated*: C, 67.74; H, 6.39; S, 3.29.
*Observed*: C, 67.78; H, 6.65; s, 3.25.

Example 41

5-(3(4-(3-(4-acetyl-3-hydroxy-2-propylphenoxy)propylthio)phenyl)-3-oxopropyl)-1H-tetrazole

The nitrile from Example 40, Step E, (1.0 g, 2.35 mmole) and tri-n-butyltin azide (1.0 g, 3.0 mmole) in dry THF (20 ml) were stirred at 70° for 48 hours, and then heated at reflux for 24 hours. The reaction mixture was cooled to room temperature, diluted with ether (20 ml) and concentrated HCl was added. After 1 hour, the solution was filtered, the solid was washed with ether and dried to yield the title compound.

Analysis, *calculated*: C, 61.52; H, 6.02; N, 11.96; S,6.84.
*Observed*: C, 61.60; H, 6.13; N, 11.91; S, 6.99.

Example 42

4-(3-(4-acetyl-3-hydroxy-2-propylphenoxy)propylsulfonyl)-gamma-oxobenzenebutyronitrile

The nitrile from Example 40, Step E, (680 mg, 1.6 mmole) and m-chloroperbenzoic acid (85%, 760 mg, 3.75 mmole) were stirred at 0° for two hours in 20 ml of $CH_2Cl_2$. Calcium hydroxide (400 mg) was added to the reaction mixture which was stirred for 1.5 hours. The reaction mixture was filtered and evaporated to dryness, affording the title compound, m.p. 143—144°.

Analysis, *calculated*: C, 63,00; H, 5.95; N, 3.06; S, 7.01.
*Observed*: C, 63.09; H, 5.95; N, 3.38; S, 7.02.

Example 43

5-(3-(4-(3-(4-acetyl-3-hydroxy-2-propylphenoxy)propylsulfonylphenyl)-3-oxopropyl)-1H-tetrazole

Following the procedure of Example 41, but substituting an equivalent amount of the nitrile from Example 42 for the nitrile from Example 40, Step E, there was obtained the title compound; m.p. 175—177°.

Analysis, *calculated*: C, 57.59; H, 5.64; N, 11.19; S, 6.41.
*Observed*: C, 57.64; H, 5.74; N, 11.18; S, 6.61.

Example 44

4-(3-(4-acetyl-3-hydroxy-2-propyl-phenoxy)propylsulfonyl)-epsilon-oxobenzenehexanoic acid

A: Preparation of methyl 4-(3-bromopropylthio)-epsilon-oxobenzenehexanoate

Adipoylchloride monomethylester (5.95 g, 33.3 mmole) in dichloroethane (15 ml) was added dropwise to a suspension of aluminum chloride (4.5 g, 33.7 mmole) in dichloroethane (50 ml). The reaction mixture was stirred at room temperature for 20 minutes. 3-Bromopropylthiobenzene (7.0 g, 30.0 mmole) in dichloroethane (15 ml) was added dropwise to the stirred suspension over 30 minutes. Additional aluminim chloride (4.0 g) was added to complete the reaction. After 30 minutes the reaction mixture was poured into ice water (500 ml) and extracted with chloroform. The organic layer was washed with brine, dried and concentrated. The residue was crystallized from ether hexanes to yield the title compound.

NMR ($CDCl_3$): 1.73 (4H, m), 2.26 (4H, m), 2.93 (2H, m), 3.15 (2H, m), 3.50 (2H, m), 3.63 (3H, s), 7.32 (2H, d), 7.90 (2H, d).

B: Preparation of methyl 4-(3-(4-acetyl-3-hydroxy-2-propylphenoxy)-propylthio)-epsilon-oxo-benzenehexanoate

The compound of Step A above, (2.0 g, 5.36 mmoles), 2,4-dihydroxy-3-propylacetophenone (2.08 g, 10.72 mmole) and potassium carbonate (3.0 g, 21.44 mmole) were stirred in methyl ethyl ketone (100 ml) and heated at reflux for three hours. The reaction mixture was filtered and concentrated *in vacuo*. The residue was dissolved in ether-methanol and extracted with 1N NaOH (3 × 50 ml). The organics were dried and concentrated *in vacuo*. The residue was triturated with methanol, filtered and dried, to yield the title compound.

NMR ($CDCl_3$): 0.94 (3H, m), 1.73 (6H, m), 2.28 (6H, m), 2.95 (2H, m), 3.22 (2H, m), 3.66 (3H, s) 4.17 (2H, m), 6.42 (1H, d), 7.40 (2H, d), 7.61 (1H, d), 7.90 (2H, d) 12.73 (1H, s).

C: Preparation of methyl 4-(3-(4-acetyl-3-hydroxy-2-propylphenoxy)-propylsulfonyl)-epsilon-oxo-benzenehexanoate

The compound of Step B above, (1.0 g, 2.05 mmoles) was dissolved in dry methylene chloride (25 ml) to which was added one equivalent of m-chloroperbenzoic acid in methylene chloride (10 ml). After 1 hour stirring at room temperature, additional m-CPBA (450 mg) was added. The reaction was complete in two hours. Calcium hydroxide (1.0 g) was added and the reaction mixture was stirred for 10 minutes. The mixture was filtered and the filtrate concentrated. The residue was purified by chromatography to yield the title compound.

NMR ($CDCl_3$): 0.90 (3H, m), 1.50 (2H, m), 1.74 (4H, m), 2.33 (6H, m), 2.55 (3H, s), 3.03 (2H, m), 3.37 (2H, m), 3.68 (3H, s), 4.12 (2H, m), 6.39 (2H, d), 7.60 (2H, d), 8.12 (4H, m), 12.73 (1H, s).

D: Preparation of 4-(3-(4-acetyl-3-hydroxy-2-propylphenoxy)propylsulfonyl)-epsilon-oxobenzenehexanoic acid

The compound of Step C above, (800 mg, 154 mmoles, 1N NaOH (3.2 ml), water (3.2 ml) and THF (10 ml) were stirred at room temperature for three hours. The THF layer was removed and the aqueous portion diluted with water and extracted with ether. The aqueous portion was acidified with concentrated HCl. A solid precipitate formed which was extracted into ether, dried and concentrated. Trituration of the residue with ether-methanol afforded the title compound; m.p. 138—140°.

Example 45

4(3-(4-acetyl-3-hydroxy-2-propylphenoxy)propylthio)-epsilon-oxobenzenehexanoic acid

The compound of Example 44, Step B (1.0 g, 2.055 mmole) was dissolved in THF (10 ml). To this solution was added 1N NaOH (4.1 ml). The reaction mixture was stirred at room temperature for two hours. Additional 1N NaOH was added and stirring continued for two hours. The THF solution was removed and the aqueous portion diluted with water and extracted with ether. The aqueous layer was acidified with concentrated HCl, extracted with chloroform, dried and evaporated. Trituration of the residue with ether/methanol afforded the title compound; m.p. 130—132°

Example 46

4-(3-(4-acetyl-3-hydroxy-2-propylphenoxy)-propylthio)-delta-oxo-benzenebutanol

A: Preparation of 4-(3-bromopropylthio)-gamma-oxobenzenebutanoic acid

A mixture of the product of Example 1, Step E (5 g), 1,3-dibromopropane (20 g) and $K_2CO_3$ (10 g) was refluxed in methylethylketone (100 ml) for 20 hours. The cooled reaction mixture was filtered, the solvent evaporated and the residue purified by chromatography over silica gel. The resulting ester of the title compound was hydrolyzed by dissolving in a mixture of 25 ml of methanol and 15 ml of 1N NaOH and stirring at 25°C for 4 hours. The reaction mixture was diluted with 100 ml of water, acidified with citric acid and extracted with 2 × 100 ml of EtOAc. The combined organic layers were washed with brine, dried over $Na_2SO_4$ and evaporated to yield the title compound which was sufficiently pure for use in the next step, m.p. 91—94°.

B: Preparation of 4(3-bromopropylthio)-delta-oxo-benzenebutanol (A) and 4-(3-bromopropylthio)delta-hydroxy-benzenebutanol (B)

4-(3-Bromopropylthio)-gamma-oxobenzenebutanoic acid (3.4 g, 10.3 mmoles) was dissolved in THF (25 ml) and cooled to −15°. Borane-THF complex (10 ml, 10.4 mmoles) was added dropwise over one hour. The solution warmed to room temperature with stirring over 12 hours. The reaction mixture was diluted with methanol, stirred for 30 minutes and evaporated *in vacuo.* The residue was treated with an excess of diazomethane and following workup and separation by HPLC the title compounds were obtained.

C: Preparation of 4-(3-(4-acetyl-3-hydroxy-2-propylphenoxy)-propylthio)-delta-oxo-benzenebutanol

The compound A of Step B above (96 mg, 0.29 mmoles), 2,4-dihydroxy-3-propylacetophenone (78 mg, 0.4 mmole) and potassium carbonate (270 mg, 2.0 mmoles) were refluxed in methyl ethyl ketone (3 ml) for twelve hours. The reaction mixture was filtered and purified by chromatography to yield the title compound; m.p. 73—75°.

| | | | |
|---|---|---|---|
| Analysis, *calculated*: | C, 66.95; | H, 7.02; | S, 7.45. |
| *Observed*: | C, 67.00; | H, 7.11; | S, 7.22. |

Example 47

4(3-(4-acetyl-3-hydroxy-2-propylphenoxy)propylsulfonyl)-delta-oxobenzenebutanol

The compound of Example 46, Step B (330 mg, 0.767 mmoles) was dissolved in chloroform and cooled to 0°. m-Chloroperbenzoic acid (328 mg, 1.57 mmoles) was added and the reaction stirred for one hour. Calcium hydroxide (195 mg) was added and the reaction stirred for 90 minutes at room temperature. The mixture was filtered and the filtrate evaporated to dryness. The residue crystallized from ethyl acetate-hexane to yield the title compound; m.p. 99—100°.

| | | | |
|---|---|---|---|
| Analysis, *calculated*: | C, 62.32; | H, 6,54; | S, 6.93. |
| *Observed*: | C, 62.23; | H, 6.50; | S, 6.68. |

Example 48

4-(3-(4-acetyl-3-hydroxy-2-propylphenoxy)propylthio-beta,epsilon-dioxobenzenepentanol

A: Preparation of 4-(3-(4-acetyl-3-hydroxy-3-propyl-phenoxy)propylthio-alpha-isobutylyloxycarbonyloxy-alpha-delta-dioxobenzenebutane

Isobutylchloroformate (260 µl, 20 mmoles) was added to 10 ml methylene chloride and cooled to −30°. The compound of Example 6, (890 mg, 2.0 mmoles) was dissolved in a mixture of methylene chloride (6 ml) and triethylamine (280 µl) and added dropwise to the −30° solution. The reaction mixture was maintained at −30° for 30 minutes, then held at 0° for 30 minutes. The reaction mixture was concentrated *in vacuo* to yield the title compound which was used without further purification in the next step.

B: Preparation of 4-(3-(4-acetyl-3-hydroxy-2-propylphenoxy)propylthio-alpha-diazomethyl-alpha,delta-dioxobenzenebutane

The crude compound from Step A above (1.1 g) was dissolved in toluene (20 ml) and cooled to 0°. An excess amount of diazomethane was added to the solution and the reaction mixture was stirred for twelve hours. The reaction mixture was purged with air, filtered and concentrated *in vacuo,* to yield the title compound which was used in the next step without further purification.

C: Preparation of 4-(3-(4-acetyl-3-hydroxy-2-propylphenoxy)propylthio-beta-epsilon-dioxobenzenepentanol

The crude compound from Step B above (800 mg) was dissolved in chloroform (20 ml) to which was added trifluoroacetic acid (excess). The reaction mixture stirred at room temperature for two hours, was filtered, and concentrated *in vacuo*. The residue was purified by column chromatography to yield the title compound, m.p. 87—89°.

Example 49

4-(3-(4-acetyl-3-acetoxy-2-propylphenoxy)propylthio)-gamma-oxo-benzenebutanoic acid

To a suspension of the compound of Example 6, (1.2 g, 2.7 mmoles) and dihydropyran (0.8 ml, 4.4 mmoles) in methylene chloride (20 ml) was added p-toluenesulfonic acid monohydrate (5 mg). The reaction mixture was stirred at room temperature for two hours. The solution was cooled to 0° and triethylamine (6 ml), 4-dimethylaminopyridine (DMAP) (15 mg) and acetic anhydride (5 ml) were added. The resulting solution stirred at room temperature for two hours. The solution was washed with 5% $NaHCO_3$ (2×), water, 1N HCl and concentrated *in vacuo*. The residue was dissolved in THF (6 ml) and stirred with 1N HCl (1 ml) at room temperature of one hour. Dichloromethane (50 ml) and water (50 ml) were added to the solution and the organic layer was collected, washed with brine (4×) dried ($Na_2SO_4$) and concentrated *in vacuo*. The crystalline residue was washed with hexane and recrystallized to afford the title compound, m.p. 121—123°.

| | | | |
|---|---|---|---|
| Analysis, *calculated*: | C, 64.18; | H, 6.21; | S, 6.59. |
| *Observed*: | C, 64.20; | H, 6.30; | S, 6.62. |

Example 50

4-(3-(4-acetyl-3-acetoxy-2-propylphenoxy)-propylsulfonyl)-gamma-oxobenzenebutanoic acid

Following the procedure of Example 49, but substituting an equivalent amount of the compound of Example 29, for the compound of Example 6, was obtained the title compound, m.p. 128—131°.

| | | | |
|---|---|---|---|
| Analysis, *calculated*: | C, 60.22; | H, 5.83; | S, 6.18. |
| *Observed*: | C, 60.30; | H, 5.95; | S, 6.29. |

Example 51

4-(3-(4-acetyl-3-hydroxy-3-propylphenoxy)-propylsulfonyl-gamma-oxobenzenebutyramide

A solution of the compound of Example 29, (1 g, 2.12 mmoles) and triethylamine (310 µl, 2.2 mmoles) in methylene chloride (5 ml) was cooled to −30°. Isobutylchloroformate (286 µl, 2.2 mmoles) was added to the −30° solution over ten minutes. The reaction mixture warmed to 0° and stirred for 30 minutes. Anhydrous ammonia was bubbled into the cold solution for 10 minutes. The reaction mixture was warmed to room temperature and stirred for 15 minutes. the solution was filtered and the solids washed with chloroform, to yield the title compound, m.p. 198—199° (dec.).

| | | | | |
|---|---|---|---|---|
| Analysis, *calculated*: | C, 60.62; | H, 6.15; | N, 2.94; | S, 6.74. |
| *Observed*: | C, 60.53; | H, 6.32; | N, 2.98; | S, 6.84. |

Example 52

N-methylsulfonyl 4-(3-(4-acetyl-3-hydroxy-2-propylphenoxy)propylthio-gamma-oxobenzenebutyramide

A solution of the compound of Example 6, (1.32 g, 3.0 mmoles) and triethylamine (0.42 ml, 3.0 mmoles) in methylene chloride (6 ml) was added to a solution of isobutylchloroformate (0.39 ml, 3.0 mmoles) in methylene chloride (6 ml) at −30°. The mixture was stirred at 0° for thirty minutes, then triethylamine (1.26 ml, 9.7 mmoles and methanesulfonamide (0.60 g, 9.5 mmoles) were added and the reaction stirred at room temperature for 24 hours. The mixture was poured into water, acidified with 1N HCl, and extracted with methylene chloride (3×). The combined organic extracts were washed with brine, dried ($Na_2SO_4$) and concentrated *in vacuo.* The residue was washed with ether and recrystallized to afford the title compound as needles, m.p. 148—150°.

| | | | | |
|---|---|---|---|---|
| Analysis, *calculated*: | C, 57.56; | H, 5.99; | N, 2.69; | S, 12.29. |
| *Observed*: | C, 57.89; | H, 6.31; | N, 2.90; | S, 12.29. |

Example 53

5(4-(3-(4-acetyl-3-hydroxy-2-propylphenoxy)propylsulfonyl)phenyl)-2(3H)-furanone

The compound of Example 29, (2.0 g, 4.197 mmoles) was suspended in chloroform (120 mls) to which was added 1,1-dichloromethylmethyl ether (10 ml). The reaction mixture was heated at 40° for 48 hours, and concentrated *in vacuo.* The residue was recrystallized to afford the title compound, m.p. 135—136°.

Analysis, *calculated*: C, 62.87; H, 5.72; S, 6.99.
*Observed*: C, 62.70; H, 5.75; S, 7.11.

Prodrug Examples

The following Table lists various prodrug derivatives (Formula XV) of the compounds of the present invention. The processes for preparing these compounds are discussed *supra.*

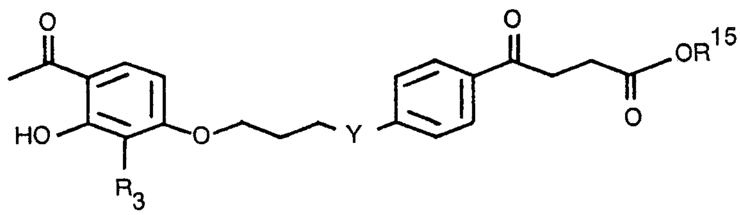

XV

$R_3$ in Formula XV is n-propyl.

TABLE I

| Example No. | L-Number | $Y^1$ | $OR^{15}$ |
|---|---|---|---|
| 54 | L–649,874–00M01 | S | 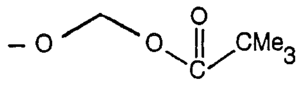 |
| 55 | L–649,888–00P01 | $SO_2$ | 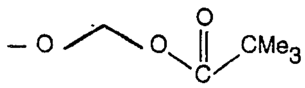 |
| 56 | – | S | 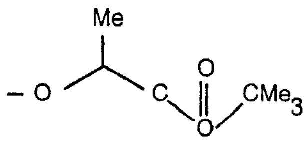 |
| 57 | L–649,948–00E01 | $SO_2$ | 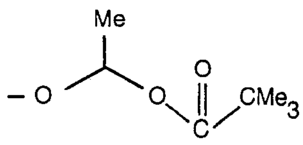 |
| 58 | L–649,875–00W01 | S | 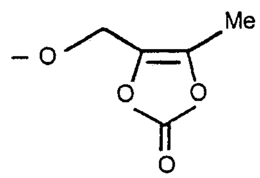 |
| 59 | L–649,893–00N01 | $SO_2$ | 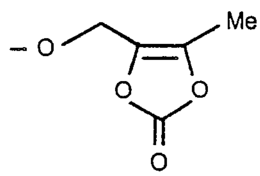 |
| 60 | L–649,876–00E01 | S | 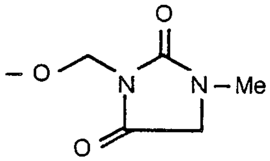 |

TABLE I (Continued)

| Example No. | L-Number | $Y^1$ | $OR^{15}$ |
|---|---|---|---|
| 61 | L–649,877–00N01 | $SO_2$ | 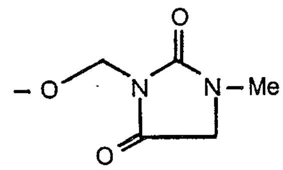 |
| 62 | – | S | 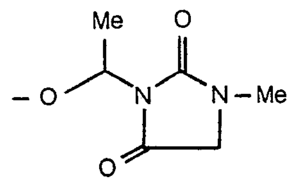 |
| 63 | L–649,951–00L01 | $SO_2$ | 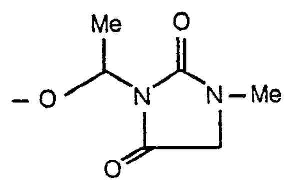 |
| 64 | L–649,930–00T01 | S | 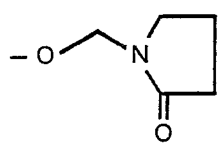 |
| 65 | L–649,931–00B01 | $SO_2$ | 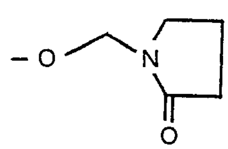 |
| 66 | L–649,932–00K01 | S | 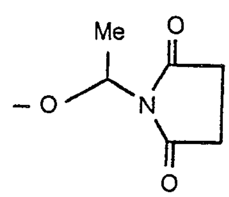 |

TABLE I (Continued)

| Example No. | L-Number | $Y^1$ | $OR^{15}$ |
|---|---|---|---|
| 67 | L—649,933—00001 | $SO_2$ | 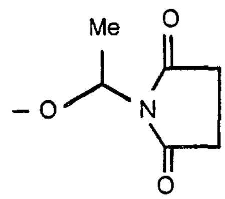 |
| 68 | – | S | 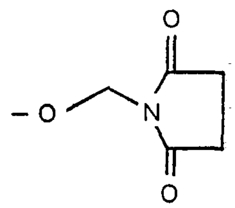 |
| 69 | L—649,952—00V01 | $SO_2$ | 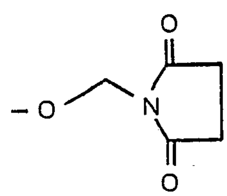 |

Example 70

4(3(4-Acetyl-3-hydroxy-2-propylphenoxy)propyloxy)-gamma-oxo-beta,beta-dimethylbenzenebutanoic acid

A: Preparation of Methyl 4-methoxy-gamma-oxo-beta-methylbenzenebutanoate

A solution of diisopropylamine (5.1 ml) in anhydrous THF (90 ml) at 0°C is treated under $N_2$ atmosphere with 22 ml of 1.6 M n-butyllithium for 20 minutes. The mixture was cooled to −78°C, and a solution of 1(4-methoxyphenyl)-1-oxopropane (5 g) in anhydrous THF (40 ml) is added. The mixture was stirred 30 minutes at −78°C, then methyl bromoacetate (3.05 ml) was added dropwise and then mixture was warmed to ambient temperature over 2 hours. After stirring 18 hours at room temperature the mixture was poured onto ice, water and conc. HCl (3 ml) was added and the solution was extracted with dichloromethane. The organic extracts were washed with brine, dried over $Na_2SO_4$ and concentrated to an oil which was purified by chromatography on silica gel to provide the title compound as an oil.

NMR ($CDCl_3$): 1.2 (3H, m), 2.2—3.1 (2H, m), 3.6 (3H, s), 3.85 (3H, s) 3.7—4.0 (1H, m), 6.9 (2H, d), 8.0 (2H, d).

B: Preparation of Methyl 4-methoxy-gamma-oxo-beta,beta-dimethylbenzenebutanoate

The product from Step A, (3.05 g) was stirred in methanol (30 ml) and 1N NaOH (15 ml) at ambient temperature for 1 hour. The mixture was diluted with brine (50 ml), washed with ethyl acetate, acidified with conc. HCl and extracted with ethyl acetate (2 × 50 ml). The second ethyl acetate extract was washed with brine, dried ($Na_2SO_4$) and reduced to dryness. The resulting oil was dissolved in anhydrous THF (20 ml) and added to a suspension of potassium hydride (1.56 g) in THF (20 ml) under nitrogen at −40°C. DMSO (50 µl) was added and the mixture was warmed to 0°C for 1/2 hr., cooled to −40° and then treated with methyl iodide (3.7 ml). After 1/2 hour at −40°C, the mixture was stirred at ambient temperature for 18 hours, then poured onto an excess of ice-conc. HCl mixture and was extracted with ethyl acetate. The organic

extract was washed with brine, dried ($Na_2SO_4$) and reduced to dryness. The resulting oil was dissolved in anhydrous methanol, saturated with HCl gas, and after 1 hour reduced to dryness to yield an oil which was purified by chromatography on silica gel to yield the title compound as an oil.

NMR ($CDCl_3$): 1.46 (6H, s), 2.78 (2H, s), 3.60 (3H, s), 3.82 (3H, s), 6.88 (2H, d), 7.80 (2H, d).

C: Preparation of Methyl 4-hydroxy-gamma-oxo-beta,beta-dimethylbenzenebutanoate

The product from Step B, (1.72 g) in $CH_2Cl_2$ (10 ml) at −78°C, was treated with boron tribromide (22 ml of a 1M solution in $CH_2Cl_2$). After 1/2 hour at −78°C the mixture was stirred at ambient temperature for 18 hours. The mixture was cooled to 0°C treated with methanol (5 ml) then washed with water, brine, dried over $Na_2SO_4$ and reduced to dryness to yield an oil which was purified by chromatography on silica gel to provide the title compound as an oil.

NMR ($CDCl_3$): 1.45 (6H, s), 2.78 (2H, s), 3.60 (3H, s), 6.80 (2H, d), 7.67 (2H, d).

D. Preparation of Methyl 4-(3-(4-acetyl-3-hydroxy-2-propylphenoxy)propyloxy)-gamma-oxo-beta,beta-dimethylbenzenebutanoate

A mixture of the product from Step C (250 mg), 4-(3-bromopropyl)-2-hydroxy-3-propylacetophenone (315 mg), and potassium carbonate (410 mg) in methyl ethyl ketone (10 ml) was refluxed under $N_2$ for 4 hours then stirred at ambient temperature for 18 hours. The mixture was filtered, washed with brine, evaporated to dryness and the resulting oil was purified by chromatography on silica gel to provide the title compound as an oil.

NMR ($CDCl_3$) 0.90 (3H, t), 1.43 (6H, s), 1.52 (2H, m), 2.30 (2H, m), 2.50 (3H, s), 2.55 (2H, m), 2.75 (2H, s), 3.57 (3H, s), 4.18 (4H, m), 6.41 (1H, d), 6.88 (2H, d), 7.53 (1H, d), 7.72 (2H, d).

E: Preparation of 4-(3-(4-acetyl-3-hydroxy-2-propylphenoxy)propyloxy)-gamma-oxo-beta,beta-dimethylbenzenebutanoic acid

The ester from Step 4 (740 mg) in methanol (1 ml) and THF (10 ml) was treated with 1N NaOH (4 ml) at ambient temperature for 2 hours. The mixture was acidified with conc. HCl, diluted with brine, extracted with ethyl acetate and the organic extracts were washed with brine, dried ($Na_2SO_4$) and evaporated to dryness to yield an oil which was purified by chromatography on silica gel to yield the title compound as a viscous oil.

NMR ($CDCl_3$): 0.89 (3H, 3t), 1.40 (8H, s and m), 2.30 (2H, m), 2.50 (3H, s), 2.5—2.8 (4H, m), 4.22 (4H, m), 6.47 (1H, d), 6.90 (2H, d), 7.49 (3H, m), 12.70 (1H, s).

## Example 71

### D,L-Sodium 4-(3-(4-acetyl-3-hydroxy-2-propylphenoxy)propyloxy)-gamma-hydroxy-beta,beta-dimethylbenzenebutanoate

A: Preparation of 4-hydroxy-gamma-hydroxy-beta,beta-dimethylbenzenebutanoic acid gamma lactone

The phenol from Example 70, Step C, (1.19 g) in dioxane (5 ml) was treated with sodium borohydride (190 mg) and the mixture was stirred 18 hours at ambient temperature. The solution was poured into 1N HCl (10 ml) and extracted with ethyl acetate. The extracts were washed with brine, dried ($Na_2SO_4$) and concentrated to dryness. The residue was recrystallized from chloroform-hexane to provide the title compound, m.p. 157—158°C.

B: Preparation of 4-(3-(4-acetyl-3-hydroxy-2-propylphenoxy)-propoxy)-gamma-hydroxy-beta,beta-dimethylbenzenebutanoic acid gamma lactone

The lactone from Step A (85 mg), 4-(3-bromo-propyloxy)-3-propyl-2-hydroxyacetophenone (160 mg) and potassium carbonate (210 mg) were refluxed together in methyl ethyl ketone (5 ml) under $N_2$ atmosphere for 8 hours. The mixture was cooled, diluted with ethyl acetate, washed with brine, dried ($Na_2SO_4$) and evaporated to an oil which was purified by chromatography on silica gel to provide the title compound as an oil.

NMR ($CDCl_3$): 0.68 (3H, s), 0.90 (3H, t), 1.22 (3H, s), 1.52 (2H, m), 2.30 (2H, m), 2.47 (2H, m), 2.53 (3H, s), 2.60 (2H, m), 4.18 (4H, m), 5.08 (1H, s), 6.47 (1H, d), 6.87 (2H, d), 7.18 (2H, d), 7.60 (1H, d), 12.72 (1H, s).

C. Preparation of D,L-sodium 4-(3-(4-acetyl-3-hydroxy-2-propylphenoxy)propyloxy)-gamma-hydroxy-beta,beta-dimethylbenzenebutanoate

The product from Step B (950 mg) was stirred in methanol (2 ml), THF (20 ml) and 1N NaOH (4.5 ml) for 2.5 hours. The mixture was concentrated to near dryness, taken up in water (10 ml) and applied to a 20 cm × 40 cm column of XAD-8 resin. After standing 1 hour the column was washed with water until the effluent pH was neutral. Methanol (50 ml) was applied to the column and the effluent was concentrated to dryness and the residue was triturated with ether to provide the title compound as a powder, m.p. 101—103°.

Analysis, *calculated*: $C_{26}H_{33}O_7N_2$: C, 64.99; H, 6.92.
*Observed*: C, 65.18; H, 7.04.

Example 72

4-(3-(4-acetyl-3-hydroxy-2-propylphenoxy)propylthio)-gamma-oxo-beta,beta-dimethylbenzenebutanoic acid

A: Preparation of 1(4-methylthiophenyl)-1-propane

To a mixture of thioanisole (5.0 g) and propionyl chloride (3.9 ml) at 0°C in dichloroethane (80 ml) was added $AlCl_3$ (6.4 g) in portions. The mixture was stirred 12 hours at ambient temperature, poured into water (200 ml) and conc. HCl (2 ml), then extracted with $CH_2Cl_2$. The extract was reduced to dryness to provide the title compound m.p. 60—61°C after purification by chromatography on silica gel.

B. Preparation of methyl 4-methylthio-gamma-oxo-beta-methylbenzenebutyrate

The ketone from Step A (180 mg) in THF (2 ml) was added to a solution of lithium diisopropylamide (1.1 mmole) in THF (3 ml) at −78° under $N_2$ atmosphere. After 15 minutes methyl bromoacetate (100 µl) was added and the mixture was warmed to ambient temperature and stirred 12 hours. The mixture was poured into water (15 ml) and conc. HCl (2 ml), extracted with $CH_2Cl_2$ and the extracts were dried ($Na_2SO_4$) evaporated to dryness and the residue purified by chromatography on silica gel to provide the title compound as an oil.

NMR ($CDCl_3$): 1.2 (3H, d), 2.55 (3H, s), 2.3—3.1 (12H, m), 3.65 (3H, s), 3.95 (1H, m), 7.3 (2H, d), 7.95 (2H, d).

C. Preparation of 4-methylthio-gamma-oxo-beta-methylbenzenebutanoic acid

The ester from Step B (1.07 g) was saponified with 1N NaOH (5 ml) and methanol (5 ml) and THF (5 ml) to provide after acidification, extraction into $CH_2Cl_2$ and evaporation to dryness the title compound, m.p. 69—71°C.

D. Preparation of Methyl 4-methylthio-gamma-oxo-beta,beta-dimethylbenzenebutyrate

The acid from Step C (3.03 g) in THF (10 ml) was added to a suspension of KH (1.9 g) in THF (50 ml) at −40° under argon. DMSO (50 µl) was added, the mixture was stirred at −5°C until gas evolution ceased, then recooled to −40°C followed by addition of methyl iodide (1.8 ml). After 0.5 hour at −40° and 12 hours at ambient temperature, the mixture was diluted with water, acidified with HCl and extracted with $CH_2Cl_2$. The organic extracts were dried ($Na_2SO_4$) and evaporated to an oil which was treated with excess diazomethane in ether. After concentration the residue was purified by chromatography on silica gel to provide the title compound.

NMR ($CDCl_3$): 1.40 (6H, s), 2.55 (3H, s), 2.75 (2H, s), 3.67 (3H, s), 7.23 (2H, d), 7.57 (2H, d).

E: Preparation of methyl 4-(3-(4-acetyl-3-hydroxy-2-propylphenoxy)propylthio-gamma-oxo-beta,beta-dimethylbenzenebutyrate

The ester from Step D (0.71 g) was treated in $CH_2Cl_2$ (10 ml) at 0°C with m-chloroperbenzoic acid (0.57 g) for 10 minutes, $Ca(OH)_2$ in excess was added and after 10 minutes at ambient temperature the mixture was filtered and evaporated to dryness. The residue was refluxed with trifluoroacetic anhydride (7 ml) for 20 minutes then concentrated to dryness. The residue was mixed with 2-hydroxy-3-propyl-4-(3-bromopropyloxy)acetophenone (0.945 g) and $K_2CO_3$ (1.2 g) in methyl ethyl ketone (10 ml) and water (50 µl) and the mixture was stirred at 20°C for 15 hours. The mixture was concentrated, partitioned between dilute HCl and $CH_2Cl_2$. The organic extracts were concentrated and purified by chromatography on silica gel to provide the title compound.

NMR ($CDCl_3$): 1.40 (6H, s), 2.77 (2H, s), 7.13 (2H, d), 7.37 (2H, d), 12.75 (1H, s).

F: Preparation of 4-(3-(4-acetyl-3-hydroxy-2-propylphenoxy)propylthio)-gamma-oxo-beta,beta-dimethylbenzenebutanoic acid

The ester from Step E (0.984 g) was saponified, acidified and the product purified by chromatography on silica gel to provide the title compound.

| | | | |
|---|---|---|---|
| Analysis, *calculated*: | C, 66.07; | H, 6.83; | S, 6.79. |
| *Observed*: | C, 66.14; | H, 6.86; | S, 6.51. |

Example 73

Sodium 4-(3-(4-acetyl-3-hydroxy-2-propylphenoxy)propylthio)-gamma-hydroxy-beta,beta-dimethylbenzenebutyrate

A: Preparation of methyl 4(3-bromopropylthio)-gamma-oxo-beta,beta-dimethylbenzenebutyrate

Following the procedure described in Example 72, Step E but substituting three equivalents of dibromopropane in place of 4(3-bromopropyloxy) 2-hydroxy-3-propyl acetophenone was obtained the title compound as an oil.

NMR ($CDCl_3$): 1.40 (6H, s), 2.0—2.3 (2H, m), 2.77 (2H, s), 3.13 (2H, t), 3.50 (2H, t), 3.67 (3H, s), 7.23 (2H, d), 7.57 (2H, d).

B: Preparation of 4-(3-bromopropylthio)gamma-hydroxy-beta,beta-dimethylbenzenebutanoic acid gamma lactone

The ester from Step A (1.4 g) in methanol (10 ml) was treated at −15°C with ceric chloride (2 mg) and $NaBH_4$ (100 mg) for 1 hour. The mixture was diluted with water, (100 ml) acidified with 1N HCl and extracted with $CH_2Cl_2$. The extracts were left for 2 days at room temperature, concentrated and purified by chromatography on silica gel to provide the title compound as an oil.

NMR ($CDCl_3$): 0.20 (3H, s), 1.27 (3H, s), 2.0—2.3 (2H, m), 2.37 (1H, d), 2.60 (1H, d), 3.07 (2H, t), 3.50 (2H, t), 5.10 (1H, s), 7.13 (2H, d), 7.33 (2H, d).

C: Preparation of 4-(3-(4-acetyl-3-hydroxy-2-propylphenoxy)propylthio-gamma-hydroxy-beta,beta-dimethylbenzenebutanoic acid gamma lactone

The lactone from Step B (0.95 g) and 2,4-dihydroxy-3-propylacetophenone (0.54 g) were placed with $K_2CO_3$ (1.2 g) in methyl ethyl ketone (10 ml) for 4 hours. The mixture was filtered, diluted with $CH_2Cl_2$, washed with water, dried ($Na_2SO_4$) concentrated and chromatographed on silica gel to provide the title compound as an oil. Mass spectrum m/e 456($M^+$).

D. Preparation of Sodium 4-(3-(4-acetyl-3-hydroxy-2-propylphenoxy)propylthio-gamma-hydroxy-beta,beta-dimethylbenzenebutyrate

The lactone from Step C (0.936 g) was saponified with 1N NaOH (4 ml) THF (4 ml) and MeOH (4 ml) for 45 minutes at ambient temperature. The mixture was concentrated to near dryness and then purified on XAD—8 resin to provide the title compound as a foam.

| | | | |
|---|---|---|---|
| Analysis, *calculated*: | C, 62.88; | H, 6.70; | S, 6.46. |
| *Observed*: | C, 62.71; | H, 6.81; | S, 6.46. |

Example 74

Resolution of Sodium (beta S*, gamma R*)-4-(3-(4-acetyl-3-hydroxy-2-propylphenoxy)propylthio-gamma-hydroxy-beta-methylbenzenebutanoate into the (+)isomer and the (−)isomer

A: Preparation of methyl (beta S, gamma R)-4-(3-(4-acetyl-3-hydroxy-2-propylphenoxy)propylthio)-gamma-(d-(alpha-methoxy)phenylacetoxy)-beta-methyl-benzenebutanoate and methyl (beta R, gamma S)-4(3-(4-acetyl-3-hydroxy-2-propylphenoxy)propylthio)-gamma-(d-(alpha-methoxy)phenylacetoxy)beta-methyl-benzenebutanoate

To the lactone (360 mg) from Example 75 below in THF (4 ml) and MeOH (1.5 ml) was added 1N NaOh (2 ml). After 30 minutes at room temperature the solution was evaporated and ethyl acetate (20 ml) was added. The mixture was cooled to 0° and acidified with HCl (1N). The ethyl acetate layer was separated and the aqueous layer was re-extracted with ethyl acetate. The combined organic layers were dried ($Na_2SO_4$) and evaporated to give crude hydroxy acid. To the crude acid in ethyl acetate was added excess $CH_2N_2$. The reaction mixture was stirred 10 minutes at room temperature and evaporated. To the residue was added THF (10 ml), $Et_3N$ (.6 ml), DCC (500 mg), DMAP (5 mg) and d-α-methoxy mandelic acid (500 mg). The reaction mixture was stirred at room temperature 2 hours and evaporated. The residue was passed through a short column of $SiO_2$ (~20 g) using 30% ethyl acetate in hexane as eluant. The combined product fractions were evaporated. Purification of the residue using HPLC using 20% ethyl acetate in hexane on Waters μ-porasil column afforded two isomers.

*Less Polar Isomer*: p.m.r. ($CDCl_3$): 0.7 (d, 3H), 1.0 (t, 3H), 1.6 (m, 2H), 1.8—2.2 (m, 4H), 2.6 (s, 3H), 2.7 (t, 2H), 3.1 (t, 2H), 3.3 (s, 3H), 3.5 (s, 3H), 4.1 (t, 2H), 4.7 (s, 1H), 5.6 (d, 1H), 6.3 (d, 1H), 7.0—7.4 (m, 9H), 7.6 (d, 1H), 12.8 (s, 1H).

*More Polar Isomer*: p.m.r. ($CDCl_3$): .9 (d, 3H), 1.5 (m, 2H), 2.0—2.5 (m, 4H), 2.6 (s, 3H), 2.65 (t, 2H), 3.1 (t, 2H), 3.4 (s, 3H), (s, 3H), 3.65 (s, 3H), 4.1 (t, 2H), 4.7 (s, 1H), 5.6 (d, 1H), 6.3 (d, 1H), 7.0 (m, 2H), 7.4 (s, 5H), 7.6 (d, 1H), 12.8 (s, 1H).

B: Preparation of the (+)-isomer of Sodium (beta S*, gamma R*)-4-(3-(4-acetyl-3-hydroxy-2-propylphenoxy)propylthio-gamma-hydroxy-beta-methylbenzenebutanoate

A solution of least polar isomer from Step A (200 mg) in MeOH (5 ml) THF (5 ml) and 1N NaOH (1 ml) was stirred at room temperature. After 2 hours 1N NaOH (5 ml) was added. After 4 hours the reaction mixture was evaporated and redissolved in $H_2O$ (2 ml). The solution was applied to an XAD column (30 ml resin). The column was washed with $H_2O$ (200 ml) and the product was then eluted with MeOH (150 ml). Evaporation of the MeOH afforded the title compound as an oil.

$[\alpha]_D$ + 9.5° (c=2.0, MeOH).

C: Preparation of the (−)-isomer of Sodium (beta S*, gamma R*)-4-(3-(4-acetyl-3-hydroxy-2-propylphenoxy)propylthio-gamma-hydroxy-beta-methylbenzenebutanoate

When the same procedure as described for the least polar isomer was applied to the more polar isomer from Step A (155 mg) there was obtained the title compound as an oil.

$[\alpha]_D$ −9.2° (c=2.0, MeOH).

Example 75

(Beta S*, gamma R*)-4-(3-(4-acetyl-3-hydroxy-2-propylphenoxy)propylthio)gamma-hydroxy-beta-methylbenzenebutanoic acid gamma lactone

A: Preparation of 4-methoxygamma-oxobenzenebutanoic Acid

Anisole (70.0 g) and succinic anhydride (65.0 g) were dissolved in 1,2-dichloroethane (1 liter) and the mixture was cooled to 0°C. To the resulting suspension there were added, in portions, $AlCl_3$ (172 g) and the resulting mixture was stirred with a mechanical stirrer for 1 hour. The mixture was then poured into a mixture of ice and water (about 1 liter) containing 50 ml of concentrated HCl. The resulting white solid was collected by filtration, washed with water and air dried to yield the title compound, m.p. 145—147°C.

B: Preparation of 4-hydroxy-gamma-oxobenzenebutanoic acid, methyl ester

A mixture of the compound from Step A (77.3 g), 48% HBr (310 ml), and acetic acid (620 ml) was heated under reflux for 18 hours. The resulting mixture was cooled to room temperature and poured into 3 liters of water. The resulting solution was extracted with ethyl acetate (3 × 500 ml). The combined organic layers were washed with water (4 × 200 ml), dried over $Na_2SO_4$, the solvents were removed by evaporation and the residue was dissolved in 500 ml of a mixture of 10 parts by weight of concentrated hydrochloric acid and 90 parts by weight of methanol. After 1 hour at room temperature the volatile components were removed by evaporation *in vacuo*. The resulting residue was triturated with hexane to yield the title compound, m.p. 115—116°.

C: Preparation of 4-dimethylthiocarbamoyloxy-gamma-oxobenzenebutanoic acid, methyl ester

A solution of the product from Step B, 25 g, in anhydrous dimethylformamide (DMF) (300 ml) was cooled to 0°- and 99% NaH, 3.46 g, was added in two portions. The mixture was stirred for 1 hour at 0° then dimethylthiocarbamoyl chloride, 19.3 g, was added and the mixture heated at 90° under a $N_2$ atmosphere for 1.5 hours. The mixture was cooled to room temperature and diluted with water to 1,200 mL. The resulting solution was then extracted with ethyl acetate (3 × 50 ml). The combined organic layers were washed with brine and then dried over $Na_2SO_4$ and evaporated to dryness *in vacuo* to yield a residue which was purified by chromatography on silica gel to yield the title compound, m.p. 62—64°.

D: Preparation of 4-dimethylcarbamoylthio-gamma-oxobenzenebutanoic acid, methyl ester

The compound from Step C, 29.6 g, was heated at 200° for 10 hours under an $N_2$ atmosphere. The mixture was cooled to room temperature, dissolved in methylene chloride and purified by chromatography on silica gel to provide the title compound, m.p. 98—100°.

E: Preparation of methyl 4-dimethylcarbamoylthio-gamma-oxo-beta-methylbenzenebutanoate

The compound from Step D, (10.0 g, 33.9 mmoles) was dissolved in THF (100 ml) and added dropwise to a stirred mixture of potassium hydride (1.6 g, 40.7 mmoles) in THF (10 ml) at −45°C. The reaction mixture was warmed to 0° to initiate the reaction, then cooled to −45°C. After 30 minutes, the reaction mixture was warmed to 0°C, and stirred for 10 minutes. A solution of methyl iodide (3.17 ml, 50.9 mmoles) in THF (10 ml) was added slowly to the cold solution. The reaction mixture was diluted with ether, washed with water, brine, dried and evaporated to yield an oil which was purified by HPLC to yield the title compound.

| | | | | |
|---|---|---|---|---|
| Analysis, *calculated*: | C, 58.23; | H, 6.19; | N, 4.53; | S, 10.36. |
| *Observed*: | C, 58.43; | H, 6.31; | N, 4.76; | S, 10.39. |

F: Preparation of (beta S*, gamma R*) 4-dimethylcarbamoylthio-gamma-hydroxy-beta-methylbenzenebutanoic acid gamma lactone (cis isomer) and (beta R*, gamma R*) 4-dimethylcarbamoylthio-gamma-hydroxy-beta-methylbenzenebutanoic acid gamma lactone (trans isomer)

To a solution of the compound from Step E aboe (10.4 g, 33.7 mmoles) in methanol (150 ml) was added cesium chloride monohydrate (50 mg). The solution was cooled to −20° and sodium borohydride (640 mg, 16.8 mmoles) was added in portions. The reaction mixture stirred at −20° for two hours. 2N NaOH (50 ml) was added and the mixture stirred at room temperature for one hour. The mixture was diluted with water (500 ml), cooled to 0°, acidified with concentrated HCl and extracted with ethyl acetate (3×). The combined organic extracts were washed with brine, dried and concentrated to an oil. The oil was dissolved in methylene chloride (150 ml) and trifluoroacetic acid (1 ml) was added. The reaction mixture stirred at room temperature for one hour. The reaction mixture was evaporated to an oil which crystallized on standing. The solid was purified by HPLC to afford pure *cis* (m.p. 116—118°) and pure *trans* (m.p. 74—76°) isomers of the title compound.

G: Preparation of (beta S*, gamma R*)-4-(3-(4-acetyl-3-hydroxy-2-propylphenoxy)propylthio)-gamma-hydroxy-beta-methylbenzenebutanoic acid gamma lactone

The *cis*-isomer of the compound of Step F above (4.50 g, 1.1 mmoles) was suspended in methanol (160 ml). To the suspension was added 2N NaOH (80 ml, 160 mmoles) and the mixture was heated at reflux for five hours. The mixture was cooled to room temperature and 4-(3-bromopropoxy)-3-propyl-2-hydroxyacetophenone (7.61 g, 24.2 mmoles) was added. The mixture stirred at room temperature for three hours, and was then heated at reflux for two hours. The reaction mixture was cooled to room temperature,

diluted with water (500 ml), acidified with concentrated HCl and extracted with ethyl acetate (2×). The organic extracts were washed with brine, dried and concentrated to an oil. The oil was dissolved in methylene chloride (100 ml) and trifluoroacetic acid (1 ml) was added. The mixture stirred at room temperature for one hour. The solution was evaporated to an oil which was purified by HPLC to yield the title compound as a white solid, m.p. 92—94°.

| | | | |
|---|---|---|---|
| Analysis, *calculated*: | C, 67.84; | H, 6.83; | S, 7.25. |
| *Observed*: | C, 67.75; | H, 6.84; | S, 7.47. |

Example 76

(Beta S*, gamma R*) 4-(3-(4-acetyl-3-hydroxy-2-propylphenoxy)propylsulfonyl)-gamma-hydroxy-beta-methylbenzenebutanoic acid gamma lactone

The compound of Example 75, Step G (550 mg, 1.24 mmoles) was dissolved in methylene chloride (5 ml) and a solution of m-chloroperbenzoic acid (606 mg, 2.99 mmoles) in methylene chloride (5 ml) was added. The mixture stirred at room temperature for ten minutes and calcium hydroxide (440 mg, 5.95 mmoles) was added. The mixture stirred at room temperature for thirty minutes. The mixture was filtered, the solid washed with methylene chloride and the filtrate evaporated to yield an oil. Silica gel chromatography of the oil afforded the title compound as a white foam.

NMR (ppm) ($CDCl_3$): 12.65 (1H, s), 8.0 (2H, d), 7.4—7.6 (3H, t), 6.35 (1H, d), 5.65 (1H, d), 4.1 (2H, t), 3.3 (2H, t), 2.7—3.1 (2H, m), 2.55 (3H, s), 2.1—2.7 (5H, m), 1.1—1.6 (2H, m), 0.9 (3H, t), 0.7 (3H, d).

Example 77

(Beta S*, gamma R*) solium 4-(3-(4-acetyl-3-hydroxy-2-propylphenoxy)propylsulfonyl)-gamma-hydroxy-beta-methylenzenebutanoate

The compound of Example 76 (543 mg, 1.15 mmoles) was dissolved in THF (17 ml) and 1N NaOH (1.7 ml, 1.72 mmoles) was added. The mixture stirred for twelve hours at room temperature and was then concentrated to dryness. The residue was purified on XAD—8 resin to afford the title compound as a beige foam.

| | | | |
|---|---|---|---|
| Analysis, *calculated*: | C, 58.35; | H, 6.07; | S, 6.23. |
| *Observed*: | C, 58.28; | H, 6.08; | S, 6.13. |

Example 78

(Beta S*, gamma R*)-sodium 4-(3-(4-acetyl-3-hydroxy-2-propylphenoxy)propylthio)-gamma-hydroxy-beta-methylbenzenebutanoate

Following the procedure of Example 77, but substituting an equivalent amount of the compound of Example 75, Step G, for the compound of Example 76, there was obtained the title compound as a beige foam.

| | | | |
|---|---|---|---|
| Analysis, *calculated*: | C, 62.22; | H, 6.48; | S, 6.64. |
| *Observed*: | C, 62.34; | H, 6.23; | S, 6.74. |

Example 79

(Beta R*, gamma R*) 4-(3-(4-acetyl-3-hydroxy-2-propylphenoxy-propylthio)-gamma-hydroxy-beta-methylbenzenebutanoic acid gamma lactone

Following the procedure of Example 75, Step G, but substituting the *trans*-lactone of Step F for the *cis*-lactone of Step F, there was obtained the title compound as an oil.

NMR (ppm) ($CDCl_3$): 12.75 (1H, s), 7.6 (1H, d), 7.2—7.5 (4H, dd), 6.4 (1H, d), 4.9 (1H, d), 4.15 (2H, t), 3.15 (2H, t), 2.6 (3H, s), 2.0—3.0 (7H, m), 1.3—1.8 (2H, m), 1.2 (2H, d), 0.95 (3H, t).

Example 80

(Beta R*, gamma R*) 4-(3-(4-acetyl-3-hydroxy-2-propylphenoxy)propylsulfonyl)-gamma-hydroxy-beta-methylbenzenebutanoic acid gamma lactone

Following the procedure of Example 76, but substituting an equivalent amount of the compound of Example 79, for the compound of Example 75, there was obtained the title compound as a white foam.

NMR (ppm) ($CDCl_3$): 12.75 (1H, s), 8.05 (2H, d), 7.6 (3H, d), 6.4 (1H, d), 5.05 (1H, d), 4.15 (2H, t), 3.4 (2H, t), 2.65 (3H, s), 2.1—2.9 (7H, m), 1.25—1.7 (2H, m), 1.25 (3H, d), 0.9 (3H, t).

Example 81

Sodium (beta R*, gamma R*) 4-(3-(4-acetyl-3-hydroxy-2-propylphenoxy)propylsulfonyl)-gamma-hydroxy-beta-methylbenzenebutanoate

Following the procedure of Example 77, but substituting an equivalent amount of the compound of Example 80 for the compound of Example 76, there was obtained the title compound as a beige foam.

| | | | |
|---|---|---|---|
| Analysis, *calculated*: | C, 58.28; | ~~H, 6.08;~~ | S, 6.13. |
| *Observed*: | C, 58.10; | H, 6.07; | S, 6.37. |

Example 82

Sodium (beta R*, gamma R*)-4 (3-(4-acetyl-3-hydroxy-2-propylphenoxy)propylthio)-gamma-hydroxy-beta-methylbensenebutanoate

Following the procedure of Example 78, but substituting an equivalent amount of the compound of Example 79 for the compound of Example 75, Step G, there was obtained the title compound as a beige foam.

| | | | |
|---|---|---|---|
| Analysis, *calculated*: | C, 62.34; | H, 6.23; | S, 6.74. |
| *Observed*: | C, 62.40; | H, 6.30; | S, 6.87. |

Example 83

(Alpha S*, beta R*, gamma R*)-4-(3-(4-acetyl-3-hydroxy-2-propylphenoxy)propylthio)-gamma-hydroxy-alpha, beta-dimethyl-benzenebutanoic acid gamma lactone

A: (Alpha S*, beta R*) 4-(3-bromopropylthio)-gamma-oxo-alpha,beta-dimethyl-benzenebutanoic acid

3-Bromopropylthiobenzene (4.62 g, 20.0 mmoles) and meso-2,3-dimethyl succinic anhydride (2.56 g, 20.0 mmoles) were dissolved in dichloroethane (100 ml) and cooled to 0°. Aluminum chloride (5.32 g, 40.0 mmoles) was added to the solution and the reaction mixture stirred at room temperature for twelve hours. The mixture was poured into ice-water (300 ml) and acidified with 6N HCl (30 ml). Methylene chloride (200 ml) was added and the mixture was stirred for two hours. The organic layer was collected. The aqueous layer was extracted with methylene chloride and the combined organics were washed with brine, dried ($Na_2SO_4$) and concentrated, to afford the title compound.

NMR (ppm) ($CDCl_3$): 1.23 (3H, d), 1.30 (3H, d), 2.23 (2H, m), 2.8—3.3 (3H, m), 3.4—3.8 (3H, m), 7.37 (2H, d), 7.93 (2H, d), 10.3 (1H, broad).

B: (Alpha S*, beta R*, gamma R*)-4(3-bromopropylthio)-gamma-hydroxy-alpha, beta-dimethylbenzenebutanoic acid gamma lactone

To a solution of the compound of Step A above (5.2 g, 14.7 mmoles) in dioxane (20 ml) and water (5 ml) at 0° was added 1N NaOH (15 ml). Sodium borohydride (0.6 g, 15.0 mmoles) was added and the mixture stirred at room temperature for one hour. Cesium chloride (20 mg) was added and the mixture stirred for ten hours. The reaction mixture was acidified with concentrated HCl and extracted with ether. The organic extracts were concentrated, dissolved in methylene chloride (20 ml) and trifluoroacetic acid (2 drops) was added. The solution stirred at room temperature for thirty minutes, was washed with brine and dried ($Na_2SO_4$). The resulting lactones were separated by HPLC to yield (a) a less polar lactone the (alpha R*, beta R*, gamma R.*) isomer of the title compound and (b) a more polar lactone. NMR analysis confirmed the more polar lactone product (b) as the title compound.

NMR (ppm) ($CDCl_3$): 0.53 (3H, d), 1.23 (3H, d), 2.17 (2H, m), 2.67—3.17 (4H, m), 3.53 (2H, t), 5.50 (1H, d, J=5Hz), 7.23 (2H, d), 7.40 (2H, d).

C: Preparation of sodium (alpha S*, beta R*, gamma R*)-4-(3-bromopropylthio)-gamma-hydroxy-alpha, beta-dimethylbenzenebutanoate

The more polar lactone of Step B above (822 mg, 2.40 mmoles) was dissolved in THF (20 ml) and treated with 2N NaOH (1.3 ml, 2.6 mmoles) at room temperature for fifteen hours. The solvent was removed *in vacuo*. The residue was dissolved in methanol and again concentrated *in vacuo* to yield the title compound as a foam. This compound was used without further purification or characterization in the next step.

D: (Alpha S*, beta R*, gamma R*)-4-(3-(4-acetyl-3-hydroxy-2-propylphenoxy)propylthio)-gamma-hydroxy-alpha, beta-dimethylbenzenebutanoic acid gamma lactone

2,4-Dihydroxy-3-propylacetophenone (450 mg, 2.32 mmoles) the compound of Step C above (890 mg, 2.32 mmoles) and potassium carbonate (1.09 g, 7.24 mmoles) were refluxed in methyl ethyl ketone (30 ml) for four hours. The mixture was cooled to room temperature and was poured into ice-water. The mixture was acidified with 6N HCl and extracted with methylene chloride. The combined extracts were washed with brine and concentrated *in vacuo.* The residue was dissolved in methylene chloride (20 ml) and trifluoroacetic acid (5 drops) was added. After five minutes the solution was washed with water, 0.1N NaOH and brine. The organic layer was dried ($Na_2SO_4$) and concentrated *in vacuo*. The residue was purified by chromatography on silica gel to afford the title compound.

NMR (ppm) ($CDCl_3$): 0.57 (3H, d), 0.93 (3H, t), 1.23 (3H, d), 1.37—1.67 (2H, m), 2.0—2.3 (2H, m), 2.57 (3H, s), 2.5—3.0 (4H, m), 3.13 (2H, t), 4.13 (2H, t), 5.45 (1H, d, *J*=5Hz), 6.37 (1H, d, J=9Hz), 7.17 (2H, d, J=8Hz), 7.33 (2H, d, J=8Hz), 7.53 (1H, d, J=9Hz), 12.70 (1H, s).

Example 84

Sodium (alpha S*, beta R*, gamma R*)-4(3-(4-acetyl-3-hydroxy-2-propylphenoxy)propylthio)-gamma-hydroxy-alpha, beta-dimethylbenzenebutanoate

The lactone of Example 83, Step D (487 mg, 1.067 mmoles) was dissolved in THF (10 ml) and methanol (2 ml). To this solution was added 1N NaOH (2 ml) and the reaction mixture stirred at room temperature for

twenty one hours. The mixture was concentrated *in vacuo* and the residue purified by chromatography on XAD—8 resin to yiels the title compound.

Analysis, *calculated*: C, 62.88; H, 6.70; S, 6.46.
*Observed*: C, 63.02; H, 6.86; S, 6.37.

Example 85

D,L-4-(3-(4-acetyl-3-hydroxy-2-propylphenoxy)-propylthio)-gamma-hydroxybenzenebutanoic acid gamma lactone

A: Preparation of 4-mercapato-gamma-oxobenzenebutanoic acid, methyl ester

Sodium (280 mg, 12.18 mmoles) was dissolved in anhydrous methanol (50 ml). To the resulting solution was added the compound of Example 75, Step D (5.0 g). the mixture was stirred at room temperature for twelve hours and poured into a mixture of water (30 ml) and concentrated HCl (7 ml). The resulting yellow solid was collected by filtration, washed with water and dried to afford the title compound, m.p. 83—84°.

B: Preparation of 4-(3-(4-Acetyl-3-hydroxy-2-propylphenoxy)propylthio)-gamma-oxobenzenebutanoic acid

2-Hydroxy-3-propyl-4-(3-bromopropyloxy)acetophenone (0.76 g., 2.4 mmoles) and the compound of Step A above (0.6 g, 2.4 mmoles) were dissolved in THF (50 ml) and heated at reflux for forty-eight hours in the presence of 1.0 equivalent of potassium carbonate. the reaction mixture was filtered and concentrated *in vacuo*. The residue was purified by chromatography on silica gel and saponified with potassium hydroxide (1.5 equivalents) in a mixture of methanol and water (25 ml, 10:1). The reaction mixture was concentrated *in vacuo*, the residue dissolved in water and acidified with citric acid. The aqueous phase was extracted with ethyl acetate. The organic phase was washed with brine, dried ($Na_2SO_4$) and evaporated to dryness. The residue was triturated with hexane and the solid collected by filtration to afford the title compound.

Analysis, *calculated*: C, 64.98; H, 6.14; S, 7.23.
*Observed*: C, 65.09; H, 6.09; S 7.28.

C: Preparation of D,L-4-(3-(4-acetyl-3-hydroxy-2-propylphenoxy)propylthio)-gamma-hydroxybenzenebutanoic acid gamma lactone

The compound of Step B above (4.0 g, 8.99 mmoles) was dissolved in a mixture of THF and ethanol (110 ml, 10:1). To this solution was added sodium borohydride (440 mg, 11.63 mmoles). The reaction mixture stirred for twenty-four hours and was concentrated *in vacuo*. The residue was treated with chloroform (40 ml) and acidified with trifluoroacetic acid. After two hours the reaction mixture was concentrated *in vacuo* and the residue purified by HPLC to afford the title compound.

Analysis, *calculated*: C, 67.27; H, 6.59; S, 7.41.
*Observed*: C, 67.49; H, 6.90; S,7.81.

Example 86

D,L-4-(3-(4-acetyl-3-hydroxy-2-propylphenoxy)propylsulfonyl)-gamma-hydroxybenzenebutanoic acid gamma lactone

A: Preparation of 4-(3-(4-acetyl-3-hydroxy-2-propylphenoxy)propylsulfonyl)-gamma-oxo-benzenebutenoic acid

1.83 g (4.0 mmole) of the ester prepared as in the first part of Example 85 Step B, before saponification, was dissolved in methylene chloride (50 ml) and treated at room temperature with m-chloroperbenzoic acid (2.5 equivalents) for two hours. The reaction mixture was concentrated *in vacuo* and purified by chromatography on silica gel to yield the methyl ester of the title compound, which, upon saponification with NaOH in methanol, yielded the title compound.

Analysis, *calculated*: C, 60.48; H, 5.92; S, 6.72.
*Observed*: C, 60.51; H, 5.90; S, 5.48.

B: Preparation of D,L-4-(3-(4-acetyl-3-hydroxy-2-propylphenoxy)propylsulfonyl)-gamma-hydroxybenzenebutanoic acid gamma lactone

The compound of Step A above (5.0 g, 10.5 mmoles) was dissolved in a mixture of THF-ethanol (105 ml, 20:1). To this solution was added sodium borohydride (440 mg, 11.63 mmoles) and the reaction mixture stirred at room temperature for 16 hours. The mixture was concentrated *in vacuo* and dissolved in chloroform (100 ml) containing trifluoroacetic acid (2 ml). This solution stirred at room temperature for two hours and was concentrated *in vacuo*. The residue was purified by HPLC to afford the title compound, m.p. 131—133°.

Analysis, *calculated*: C, 62.59; H, 6.13; S, 6.96.
*Observed*: C, 62.62; H, 6.09; S, 6.74.

Using the above described methodology and starting with the appropriately substituted 2,4-dihydroxyacetophenone, the following compounds are also prepared:

Example 87
4-((3-(4-acetyl-6-fluoro-3-hydroxy-2-propylphenoxy)propyl)thio)-gamma-oxobenzenebutanoic acid

Example 88
4-((3-(4-acetyl-6-chloro-3-hydroxy-2-propylphenoxy)propyl)thio-gamma-oxobenzenebutanoic acid

Example 89
4-(3-(4-acetyl-6-chloro-3-hydroxy-2-propylphenoxy)propylsulfonyl)-gamma-oxobenzenebutanoic acid

Example 90
4-((3-(4-acetyl-6-bromo-3-hydroxy-2-propylphenoxy)propyl)thio)-gamma-oxobenzenebutanoic acid

Example 91
4-(3-(4-acetyl-6-bromo-3-hydroxy-propylphenoxy)propylsulfonyl)-gamma-oxobenzenebutanoic acid

Example 92
4-((3-(4-acetyl-3-hydroxy-6-methyl-2-propylphenoxy)propyl)thio)-gamma-oxobenzenebutanoic acid

Example 93
(Beta S*, gamma R*) 4-(3-(4-acetyl-6-fluoro-3-hydroxy-2-propylphenoxy)propylthio-gamma-hydroxy-beta-methylbenzenebutanoic acid

Example 94
(Beta S*, gamma R*) 4-(3-(4-acetyl-5-chloro-3-hydroxy-2-propylphenoxy)propylthio)-gamma-hydroxy-beta-methylbenzenebutanoic acid

Example 95
(Beta S*, gamma R*) 4-(3-(4-acetyl-3-hydroxy-6-methyl-2-propylphenoxy)propylthio)-gamma-hydroxy-beta-methylbenzenebutanoic acid

Example 96
(Beta S*, gamma R*) 4-(3-(4-acetyl-6-ethyl-3-hydroxy-2-propylphenoxy)propylthio)gamma-hydroxy-beta-methylbenzenebutanoic acid

Example 97
4-(3-(4-acetyl-6-fluoro-3-hydroxy-2-propylphenoxy)propyloxy)-gamma-hydroxy-beta,beta-dimethylbenzenebutanoic acid

Example 98
4-(3-(4-acetyl-6-chloro-3-hydroxy-2-propylphenoxy)propyloxy)-gamma-hydroxy-beta,beta-dimethylbenzenebutanoic acid

Example 99
4-(3-(4-acetyl-3-hydroxy-6-methyl-2-propylphenoxy)propyloxy)-gamma-hydroxy-beta,beta,dimethylbenzenebutanoic acid

Example 100
4-(3-(4-acetyl-6-fluoro-3-hydroxy-2-propylphenoxy)propylthio)-gamma-hydroxy-beta,beta-dimethylbenzenebutanoic acid

Example 101
4-(3-(4-acetyl-5-chloro-3-hydroxy-2-propylphenoxy)propylthio)-gamma-hydroxy-beta,beta-dimethylbenzenebutanoic acid

Example 102
4-(3-(4-acetyl-3-hydroxy-6-methyl-2-propylphenoxy)propylthio)-gamma-hydroxy-beta,beta-dimethylbenzenebutanoic acid

Example 103
Sodium (beta S*, gamma R*) 4-(3-(4-acetyl-3-hydroxy-2-propylphenoxy)propyloxy)gamma-hydroxy-beta-methylbenzenebutanoate

A: Preparation of methyl 4-hydroxy-gamma-oxo-beta-methylbenzenebutanoate

A mixture of the ester from Example 70, Step A (8.15 g), 48% HBr (40 ml) and acetic acid (80 ml) was refluxed for 4 days, then poured into water (250 ml) and extracted with ethyl acetate. The extracts were washed with water and dried ($Na_2SO_4$) and evaporated to an oil which was treated with 150 ml of a mixture

of 10 parts by weight of concentrated hydrochloric acid and 90 parts by weight of methanol for 18 hours. the volatiles were removed by evaporation and the residue was taken up in $CH_2Cl_2$, washed with water, brine, dried, evaporated to dryness then purified by chromatography on silica gel to provide the title compound, m.p. 89—91°C.

B: Preparation of methyl 4(3-bromopropoxy)-gamma-oxo-beta-methylbenzenebutanoate

The mixture of the phenol from Step A (4.0 g) 1,3-dibromopropane (18.2 ml), potassium carbonate (7.5 g) and methyl ethyl ketone (100 ml) was refluxed for 1 hour. The mixture was filtered, concentrated *in vacuo* and the residue was purified by chromatography on silica gel to provide the title compound as an oil.

C: Preparation of (beta S*, gamma S*) and (beta S*, gamma R*) 4-(3-bromopropyloxy)-gamma-hydroxy-beta-methylbenzenebutanoic acid gamma lactones

The ketone from Step B (5.0 g) in methanol (70 ml) was treated with ceric chloride monohydrate (50 mg) and the mixture was cooled to −20°C followed by addition of sodium borohydride (277 mg). After stirring 2 hours at −20°C the mixture was warmed to ambient temperature, diluted with water (150 ml) and 1N HCl (10 ml), then extracted with $CH_2Cl_2$. The extracts were washed with brine and evaporated to an oil which was treated in methanol (50 ml) and 2N NaOH (22 ml) for 1 hour. The mixture was diluted with water (750 ml), acidified, and extracted with EtOAc. The organic extracts were washed with brine, dried ($Na_2SO_4$) evaporated to an oil which was dissolved in dichloromethane (100 ml) and trifluoroacetic acid (1 ml). After 30 minutes the mixture was evaporated to dryness and the residue was purified by chromatography on silica gel to provide the (beta S*, gamma S*)-isomer of the title compound (less polar).

NMR ($CDCl_3$): 1.15 (3H, d), 2.1—2.9 (5H, m), 3.55 (2H, t), 4.1 (2H, t), 4.85 (1H, d), 6.9 (2H, d), 7.25 (2H, d), and the (beta S*, gamma R*) isomer of the the title compound (more polar), NMR ($CDCl_3$): 0.7 (3H, d), 2.2—2.9 (5H, m), 3.6 (2H, t), 4.1 (2H, s), 5.5 (1H, d), 6.9 (2H, d), 7.1 (2H, d).

D: Preparation of (Beta S*, gamma R*) 4-(3-(4-acetyl-3-hydroxy-2-propylphenoxy)propyloxy)-gamma-hydroxy-beta-methylbenzenebutanoic acid gamma lactone

The more polar isomer from Step C (1.2 g) and 2,4-dihydroxy-3-propylacetophenone (1.19 g) was refluxed in methyl ethyl ketone (20 ml) and potassium carbonate (2 g) for 12 hours. The mixture was filtered, concentrated and purified by chromatography on silica gel to provide the title compound as an oil.

NMR ($CDCl_3$): 0.7 (3H, d), 0.9 (3H, t), 1.5 (2H, m), 2.1—2.9 (7H, m), 2.5 (3H, s), 4.05—4.3 (4H, m), 5.5 (1H, d), 6.4 (1H, d), 6.85 (2H, d), 7.1 (2H, d), 7.55 (2H, d), 12.65 (1H, s).

E: Preparation of Sodium (Beta S*, gamma R*) 4-(3-(4-acetyl-3-hydroxy-2-propylphenoxy)propyloxy)-gamma-hydroxy-beta-methylbenzenebutanoate

The lactone from Step D (1.19 g) was saponified in 1N NaOH (4 ml), THF (15 ml) and methanol (2 ml) for 12 hours. The mixture was concentrated *in vacuo* and the residue in water was purified on XAD—8 resin to provide the title compound as a foam.

| | | |
|---|---|---|
| Analysis, *calculated*: | C, 64.36; | H, 6.70. |
| *Observed*: | C, 64.26; | H, 6.93. |

Using the above described methodologies, the following compounds are also prepared:

Example 104

4-(3-(4-acetyl-3-hydroxy-2-propylphenoxy)propylthio)-3-propyl-gamma-oxo-benzenebutanoic acid, m.p. 148—149°.

| | | | |
|---|---|---|---|
| Analysis, *calculated*: | C, 66.64; | H, 7.04; | S, 6.59. |
| *Observed*: | C, 66.89; | H, 7.22; | S, 6.25. |

Example 105

4-(3-(4-acetic-3-hydoxy-2-propylphenoxy)propylsulfonyl)-3-propyl-gamma-oxo-benzenebutanoic acid, m.p. 156—158°C.

| | | | |
|---|---|---|---|
| Analysis, *calculated*: | C, 62.53; | H, 6.61; | S, 6.18. |
| *Observed*: | C, 62.35; | H, 6.62; | S, 5.99. |

Example 106

(Beta R*, gamma S*) sodium 4(3(4-acetyl-3-hydroxy-2-propylphenoxy)propylthio)-3-fluoro-gamma-hydroxy-beta-methylbenzenebutanoate

Example 107

(Beta R*, gamma S*) sodium 4-(3-(4-acetyl-3-hydroxy-2-propylphenoxy)propylthio)-3-bromo-gamma-hydroxy-beta-methylbenzenebutanoate

Example 108

(Beta R*, gamma S*) sodium 4-(3-(4-acetyl-3-hydroxy-2-propylphenoxy)propylthio)-3-chloro-gamma-hydroxy-beta-methylbenzenebutanoate

Example 109
4-(3-(4-acetyl-3-hydroxy-2-propylphenoxy)propylsulfonyl)-gamma-oxo-beta,beta-dimethylbenzenebutanoic acid

Example 110
D,L-Sodium 4-(3-(4-acetyl-3-hydroxy-2-propylphenoxy)propylsulfonyl)-gamma-hydroxy-beta,beta-dimethylbenzenebutanoate

Example 111
4-(3-(4-acetyl-3-hydroxy-2-propylphenoxy)propylthio)-3-propyl-gamma-oxo-beta,beta-dimethylbenzenebutanoic acid

Example 112
D,L Sodium 4-(3-(4-acetyl-3-hydroxy-2-propylphenoxy)propylthio)-3-propyl-gamma-hydroxy-beta,beta-dimethylbenzenebutanoate

Example 113
Sodium 4-(3-(4-acetyl-3-hydroxy-2-propylphenoxy)propylthio)-3-fluoro-gamma-oxo-beta,beta-dimethylbenzenebutanoate

Example 114
D,L Sodium 4-(3-(4-acetyl-3-hydroxy-2-propylphenoxy)propylthio)-3-fluoro-gamma-hydroxy-beta,beta-dimethylbenzenebutanoate

Example 115
4-(3-(4-acetyl-3-hydroxy-2-propylphenoxy)propylthio)-3-chloro-gamma-oxo-beta,beta-dimethylbenzenebutanoic acid

Example 116
D,L-Sodium 4-(3-(4-acetyl-3-hydroxy-2-propyphenoxy)propylthio)-3-chloro-gamma-hydroxy-beta,beta-dimethylbenzenebutanoate

Example 117
D,L-Sodium 4-(3-(4-acetyl-3-hydroxy-2-propylphenoxy)propylthio)-3-bromo-gamma-hydroxy-beta,beta-dimethylbenzenebutanoate

Example 118
D,L-Sodium 4-(3-(4-acetyl-3-hydroxy-2-propylphenoxy)propyloxy)-3-fluoro-gamma-hydroxy-beta,beta-dimethylbenzenebutanoate

Example 119
D,L-Sodium 4-(3-(4-acetyl-3-hydroxy-2-propylphenoxy)propyloxy)-3-bromo-gamma-hydroxy-beta,beta-dimethylbenzenebutanoate

Example 120
D,L-Sodium 4-(3-(4-acetyl-3-hydroxy-2-propylphenoxy)propyloxy)-3-chloro-gamma-hydroxy-beta,beta-dimethylbenzenebutanoate

Example 121
4-(3-(4-(acetyl-3-hydroxy-2-propylphenoxy)propyloxy-3-fluoro-gamma-oxo-beta,beta-dimethylbenzenebutanoic acid

Example 122
4-(3-(4-acetyl-2-hydroxy-2-propylphenoxy)propyloxy)-3-chloro-gamma-oxo-beta,beta-dimethylbenzenebutanoic acid

Example 123
4-(3-(4-acetyl-3-hydroxy-2-propylphenoxy)propyloxy)-3-bromo-gamma-oxo-beta,beta-dimethylbenzenebutanoic acid

Example 124
4-(3-(4-acetyl-3-hydroxy-2-propylphenoxy)propyloxy)-3-propyl-gamma-oxo-beta,beta-dimethylbenzenebutanoic acid

Example 125
Sodium-(Beta R*, gamma S*) 3-(3-(4-acetyl-3-hydroxy-2-propylphenoxy)propylthio)-gamma-hydroxy-beta-methylbenzenebutanoate

Example 126
D,L-sodium 3-(3-(4-acetyl-3-hydroxy-2-propylphenoxy)propylthio)-gamma-hydroxy-beta,beta-dimethylbenzenebutanoate

Example 127
D,L-sodium 3-(3-(4-acetyl-3-hydroxy-2-propylphenoxy)propyloxy)-gamma-hydroxy-beta,beta-dimethylbenzenebutanoate

Example 128
3-(3-(4-acetyl-3-hydroxy-2-propylphenoxy)propylthio)-gamma-oxobenzenebutanoic acid

Example 129
3-(3-(4-acetyl-3-hydroxy-2-propylphenoxy)propylthio)-gamma-oxo-beta,beta,dimethylbenzenebutanoic acid

Example 130
3-(3-(3-acetyl-3-hydroxy-2-paropylphenoxy)propyloxy)-gamma-oxo-beta,beta-dimethylbenzenebutanoic acid

Example 131
Beta R*, gamma S*) sodium 4-(3-(4-acetyl-3-hydroxy-2-propylphenoxy)propylthio)-gamma-hydroxy-beta-methylbenzenebutanoate-S-oxide

Example 132
(Beta R*, gamma S*) 4-(3-(4-acetyl-3-hydroxy-2-propylphenoxy)propylthio)-3-propyl-gamma-hydroxy-beta-methylbenzenebutanoic acid

Example 133
4-(3-(4-acetyl-3-hydroxy-2-propylphenoxy)-propylsufonyl)-gamma-oxo-beta,beta-dimethylbenzenebutanoic acid

Example 134
D,L-4-(3-(4-acetyl-3-hydroxy-2-propylphenoxy)propylsulfonyl)-gamma-hydroxy-beta,beta-dimethylbenzenebutanoic acid

Example 135
D,L-4-(3-(4-acetyl-3-hydroxy-2-propylphenoxy)propylthio)-3-propyl-gamma-oxo-beta,beta-dimethylbenzenebutanoic acid

Example 136
4-(3-(4-acetyl-3-hydroxy-2-propylphenoxy)propylthio)-3-fluoro-gamma-oxo-beta,beta-dimethylbenzenebutanoic acid

Example 137
D,L-4-(3-(4-acetyl-3-hydroxy-2-propylphenoxy)propylthio)-3-fluoro-gamma-hydroxy-beta,beta-dimethyl-benzenebutanoic acid

Example 138
(3R*, 4S*) 4-[4-[3-(4-acetyl-3-hydroxy-2-propylphenoxy)propylthio]-3-fluorophenyl]-3-methyl-gamma-butyrolactone

Example 139
(3R*, 4S*) 4-[4-[3-(4-acetyl-3-hydroxy-2-propylphenoxy)propylthio]-3-chlorophenyl]3-methyl-gamma-butyrolactone

Example 140
(3R*, 4S*) 4-[4-[3-(4-acetyl-3-hydroxy-2-propylphenoxy)propylthio]-3-bromophenyl]-3-methyl-gamma-butyrolactone

Example 141

(3R*, 4R*) 4-[4-[3-(4-acetyl-3-hydroxy-2-propylphenoxy)propylthio]-3-fluorophenyl]-3-methyl-gamma-butyrolactone

Example 142

(3R*, 4R*) 4-[4-[3-(4-acetyl-3-hydroxy-2-propylphenoxy)propylthio]-3-bromophenyl]-3-methyl-gamma-butyrolactone

Example 143

(3R*, 4R*) 4-[4-[3-(4-acetyl-3-hydroxy-2-propylphenoxy)propylthio]-3-chlorophenyl]-3-methyl-gamma-butyrolactone

Example 144

(3R*, 4S*) 4-[4-[3-(4-acetyl-3-hydroxy-2-propylphenoxy)propyloxy]-phenyl]-3-methyl-butyrolactone

Example 145

(Beta R*, gamma S*) 4-(3-(4-acetyl-3-hydroxy-2-propylphenoxy)propyloxy)-3-propyl-gamma-hydroxy-beta-methylbenzenebutanoic acid

Example 146

4-(3-(4-acetyl-3-hydroxy-2-propylphenylthio)propyloxy)-3-fluoro-gamma-oxo-benzenebutanoic acid m.p. 133—134°.

Example 147

4-(3-(4-acetyl-3-hydroxy-2-propylphenylthio)propyloxy)-3-fluoro-gamma-oxo-benzenebutanoic acid S-oxide m.p. 156—157°

Example 148

4-(3-(4-acetyl-3-hydroxy-2-propylphenylsulfonyl)propyloxy)-3-fluoro-gamma-oxo-benzenebutanoic acid m.p.182—184°

Example 149

4-(3-(4-acetyl-3-hydroxy-2-propylphenylthio)propylthio)-gamma-oxo-benzenebutanoic acid m.p. 87—88.5°.

Example 150

4-(3-(4-acetyl-3-hydroxy-2-propylphenylthio)propylsulfonyl)-gamma-oxo-benzenebutanoic acid m.p. 135—138°.

It is understood that for those compounds which contain asymmetric centers, the present invention includes the racemic mixture as well as the individual resolved optical isomers.

**Claims for the Contracting States: BE CH DE FR GB IT LI LU NL SE**

1. A compound having either of the following Formulae:

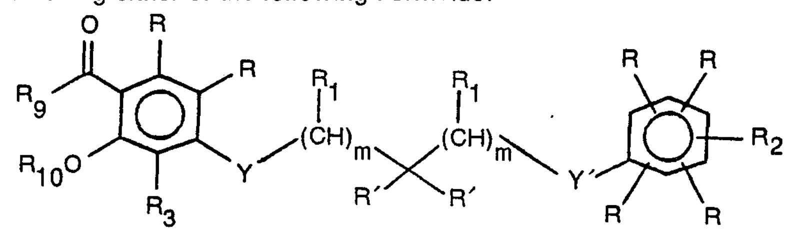

and

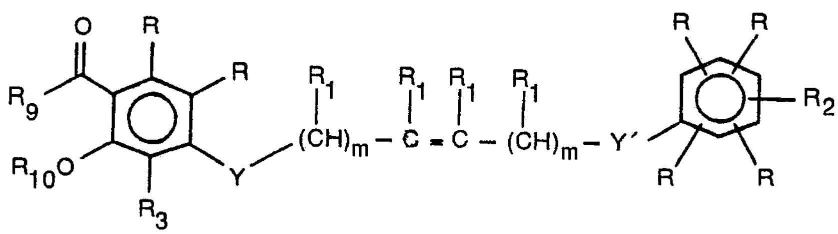

wherein

each R is independently H, OH, alkyl of 1 to 6 carbon atoms which may be straight chain or branched; alkenyl of 2 to 6 carbon atoms which may be straight chain or branched; trifluoromethyl; alkoxy of 1 to 6 carbon atoms which may be straight chain or branched; SH; thioalkyl of 1 to 6 carbon atoms which may be

straight chain or branched; phenyl; phenyl substituted by alkyl of 1 to 3 carbon atoms or by halogen; benzyl; phenalkyl with from 2 to 4 alkyl carbon atoms; halogen, amino; $N(R_4)_2$ wherein $R_4$ is H or alkyl of 1 to 6 carbon atoms which may be straight chain or branched; $COOR_4$; $CH_2OR_4$; formyl; CN; trifluoromethylthio; or nitro;

each R′ is independently $R_4$; $OR_4$; $COOR_4$; $N(R_4)_2$; $SR_4$; $CH_2OR_4$; CHO; or together R′ and R′ are O, $CH_2$ or

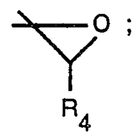

Y is oxygen, sulfur, sulfoxide, sulfone;

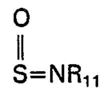

wherein $R_{11}$ is alkyl or 1—4 carbon atoms which may be straight chain or branched; $NR_{12}$ wherein $R_{12}$ is H, alkyl of 1—4 carbon atoms, which may be straight chain or branched, or

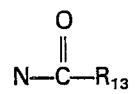

wherein $R_{13}$ is alkyl or 1—4 carbon atoms which may be straight chain or branched, alkoxy of 1—4 carbon atoms, which may be straight chain or branched; or N—CN;

Y′ is Y, $—CH_2—$ or

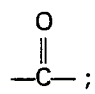

each $R_1$ is independently hydrogen or alkyl of 1—3 carbon atoms;

each m is independently an integer from 0—6; and $R_2$ is

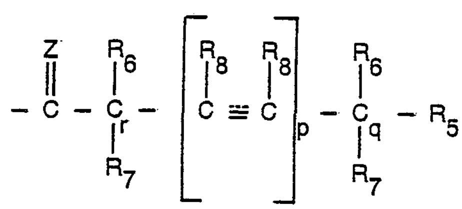

wherein

Z is O, S, $CH_2$, H and OH, alkenyl of 1—4 carbons; or $N—R_{14}$ wherein $R_{14}$ is OH, alkyl or alkoxy of 1 to 6 carbon atoms, perhaloalkyl of 1 to 6 carbon atoms, phenyl or phenyl substituted by alkyl or alkoxy groups of 1 to 3 carbon atoms, halogen, hydroxy, haloalkyl, COOH, CN, formyl or acyl of 1 to 6 carbon atoms;

each $R_6$ is independently H or alkyl of 1—4 carbons;

each $R_7$ is independently H, OH, or alkyl of 1—4 carbons;

each $R_8$ is independently H, or alkyl of 1—4 carbons, and is absent when a triple bond is present;

$R_5$ is $COOR_4$; $CH_2OH$; CHO; tetrazole; $NHSO_2R_{14}$; $CONHSO_2R_{14}$; hydroxymethylketone; CN; $CON(R_7)_2$; a monocyclic or bicyclic heterocyclic ring containing an acidic hydroxyl group; or $COOR_{15}$ where $R_{15}$ is:

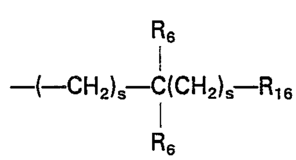

wherein each s is independently 0—3; $R_{16}$ is

A) a monocyclic or bicyclic heterocyclic radical containing from 3 to 12 nuclear carbon atoms and 1 to 2 nuclear heteroatoms selected from N and S with at least one being N, and with each ring in the heterocyclic radical being formed of 5 or 6 atoms, or

B) the radical $W—R_{17}$ wherein W is O, S or NH and $R_{17}$ contains up to 21 carbon atoms and is (1) a hydrocarbon radical (2) an acyl radical of an organic acyclic or monocyclic carboxylic acid containing not more than 1 heteroatom in the ring;

r and q are each independently 0—20 provided that the total of r and q does not exceed 20; and p is 0 or 1;

$R_3$ is alkyl of 1 to 6 carbon atoms which may be straight chain or branched; or alkenyl of 3 to 6 carbon atoms which may be straight chain or branched;

$R_9$ is alkyl of 1 to 6 carbon atoms which may be straight chain or branched; alkoxy of 1 to 6 carbon atoms which may be straight chain or branched; or $(CH_2)_rR_5$;

$R_{10}$ is H; alkyl of 1 to 6 carbon atoms which may be straight chain or branched;

$$R_4\overset{\displaystyle O}{\overset{\|}{C}}— \text{ or } R_4OCH_2— ;$$

or a compound that is a lactone thereof when, in $R_2$, Z is H and OH or $R_7$ is OH and $R_5$ is carboxy, or a compound that is a pharmaceutically acceptable salt or acid-addition salt thereof.

2. A compound as claimed in Claim 1 in which Y is oxygen and Y′ is oxygen, sulfur, sulfoxide, sulfone, amino or cyanoamido.

3. A compound as claimed in Claim 1 or 2 in which each m is 1.

4. 4-((3-(4-Acetyl-3-hydroxy-2-propylphenoxy)-propyl)thio)-gamma-oxobenzenebutanoic acid.

5. 4-[3-(4-Acetyl-3-hydroxy-2-propylphenoxy)propylsulfonyl]-gamma-oxobenzene butanoic acid.

6. (Beta S*, gamma R*) 4-(3-(4-acetyl-3-hydroxy-2-propylphenoxy)propylthio)-gamma-hydroxy-beta-methylbenzenebutanoic acid sodium salt.

7. (Beta S*, gamma R*) 4-(3-(4-acetyl-3-hydroxy-2-propylphenoxy)propyloxy)-gamma-hydroxy-beta-methyl-benzenebutanoic acid sodium salt.

8. A compound as claimed in Claim 1 having the name:

4-(3-(4-Acetyl-3-hydroxy-2-propylphenoxy)propoxy)3-fluoro-gamma-oxobenzenebutanoic acid,

4-(3-(4-Acetyl-3-hydroxy-2-propylphenoxy)propoxy)-gamma-oxobenzenebutanoic acid,

4-((3-(4-Acetyl-3-hydroxy-2-propylphenoxy)-2-hydroxypropyl)thio)-2-fluoro-gamma-oxobenzenebutanoic acid,

4-((3-(4-Acetyl-3-hydroxy-2-propylphenoxy)2-hydroxypropyl)thio)-2-hydroxy-gamma-oxobenzenebutanoic acid,

4-((3-(4-Acetyl-3-hydroxy-2-propylphenoxy)propyl)thio)-2-hydroxy-gamma-oxobenzenebutanoic acid,

4-((3-(4-Acetyl-3-hydroxy-2-propylphenoxy)-2-hydroxypropyl)thio)-2-methoxy-gamma-oxobenzenebutanoic acid,

4-((3-(4-Acetyl-3-hydroxy-2-propylphenoxy)propyl)thio)-3-fluoro-gamma-oxobenzenebutanoic acid,

4-((3-(4-Acetyl-3-hydroxy-2-propylphenoxy)propyl)thio)-2-fluoro-gamma-oxobenzenebutanoic acid,

4-(3-(4-Acetyl-3-hydroxy-2-propylphenoxy)propoxy)-3-fluoro-gamma-oxobenzenehexanoic acid,

4-((3-(4-Acetyl-3-hydroxy-2-propylphenoxy)propoxy-2-fluoro-gamma-oxobenzenebutanoic acid,

4-(3-(4-Acetyl-3-hydroxy-2-propylphenoxy)propoxy)-gamma-oxobenzenebutanoic acid methyl ester,

4-(3-(4-Acetyl-3-hydroxy-2-propylphenoxy)propoxy)-3-methyl-gamma-oxobenzenebutanoic acid,

4-(3-(4-Acetyl-3-hydroxy-2-propylphenoxy)propoxy)-2-chloro-gamma-oxobenzenebutanoic acid,

4-(4-(4-Acetyl-3-hydroxy-2-propylphenoxy)-2-butenoxy)-3-fluoro-gamma-oxobenzenebutanoic acid,

4-(3-(4-Acetyl-3-hydroxy-2-propylphenoxy)-2-hydroxypropylthio)-3-fluoro-gamma-oxobenzenebutanoic acid-S-oxide,

4-(3-(4-Acetyl-3-hydroxy-2-propylphenoxy)propylthio)-3-fluoro-gamma-oxobenzenebutanoic acid-S-oxide,

4-(3-(4-Acetyl-3-hydroxy-2-propylphenoxy)propylthio)-2-fluoro-gamma-oxobenzenebutanoic acid-S-oxide,

4-(3-(4-Acetyl-3-hydroxy-2-propylphenoxy)-2-hydroxypropylthio)-2-fluoro-gamma-oxobenzenebutanoic acid-S-oxide,

4-(3-(4-Acetyl-3-hydroxy-2-propylphenoxy)propylsulfonyl)-2-fluoro-gamma-oxobenzenebutanoic acid,

4-(3-(4-Acetyl-3-hydroxy-2-propylphenoxy)-2-hydroxypropylsulfonyl)-2-fluoro-gamma-oxobenzenebutanoic acid,

4-(3-(4-Acetyl-3-hydroxy-2-propylphenoxy)propylsulfonyl)-3-fluoro-gamma-oxobenzenebutanoic acid,

4-(3-(4-Acetyl-3-hydroxy-2-propylphenoxy)-2-hydroxypropylsulfonyl)-3-fluoro-gamma-oxobenzenebutanoic acid,

4-(3-(4-Acetyl-3-hydroxy-2-propylphenoxy)-2-hydroxypropylsulfonyl)-gamma-oxobenzenebutanoic acid,

4-(3-(4-Acetyl-3-hydroxy-2-propylphenoxy)propoxy)-3-chloro-gamma-oxobenzenebutanoic acid,

4-(3-(4-Acetyl-3-hydroxy-2-propylphenoxy)-2-hydroxypropoxy)-3-fluoro-gamma-oxobenzenebutanoic acid, sodium salt, monohydrate,

4-(3-(4-Acetyl-3-hydroxy-2-propylphenoxy)propoxy)-alpha,alpha-dimethyl-gamma-oxobenzenebutanoic acid,

4-(3-(4-Acetyl-3-hydroxy-2-propylphenoxy)propoxy)-beta-methyl-gamma-oxobenzenebutanoic acid,

4-(3-(4-Acetyl-3-hydroxy-2-propylphenoxy)-2-methylidenylpropoxy)-gamma-oxobenzenebutanoic acid,

4-(3-(4-Acetyl-6-chloro-3-hydroxy-2-propylphenoxy)-2-methylidenepropoxy)-gamma-oxobenzenebutanoic acid,

4-(3-(4-Acetyl-3-hydroxy-2-propylphenoxy)-2-methylidenepropylthio)-gamma-oxobenzenebutanoic acid,

4-(3-(4-Acetyl-3-hydroxy-2-propylphenoxy)-2-methylidenepropoxy)-3-fluoro-gamma-oxobenzenebutanoic acid,

4-(3-(4-Acetyl-3-hydroxy-2-propylphenoxy)propylthio)-gamma-oxobenzenebutyronitrile,

5-(3(4-(3-(4-Acetyl-3-hydroxy-2-propylphenoxy)propylthio)phenyl)-3-oxopropyl)-1H-tetrazole,

4(3(4-Acetyl-3-hydroxy-2-propylphenoxy)propylsulfonyl)-gamma-oxobenzenebutyronitrile,

5-(3-(4-(3-(4-Acetyl-3-hydroxy-2-propylphenoxy)propylsulfonylphenyl)-3-oxopropyl)-1H-tetrazole

4-(3-(4-Acetyl-3-hydroxy-2-propylphenoxy)propylthio)-epsilon-oxobenzenehexanoic acid,

4(3-(4-Acetyl-3-hydroxy-2-propylphenoxy)propylsulfonyl)-epsilon-oxobenzenehexanoic acid,

4-(3-(4-Acetyl-3-hydroxy-2-propylphenoxy)-propylthio)-delta-oxobenzenebutanol,

4(3-(4-Acetyl-3-hydroxy-2-propylphenoxy)propylsulfonyl)-delta-oxobenzenebutanol,

4-(3-(4-Acetyl-3-hydroxy-2-propylphenoxy)propyl-thio-beta epsilon-dioxo-benzenepentanol,

4-(3-(4-Acetyl-3-acetoxy-2-propylphenoxy)propylthio)-gamma-oxobenzenebutanoic acid,

4-(3-(4-Acetyl-3-acetoxy-2-propylphenoxy)-propylsulfonyl)-gamma-oxobenzenebutanoic acid,

4-(3-(4-Acetyl-3-hydroxy-2-propylphenoxy)-propylsulfonyl-gamma-oxobenzenebutyramide,

N-Methylsulfonyl 4-(3-(4-acetyl-3-hydroxy-2-propylphenoxy)propylthio-gamma-oxo-benzenebutyramide,

5(4-(3-(4-Acetyl-3-hydroxy-2-propylphenoxy)propylsulfonyl)phenyl)-2(3H)-furanone,

4-(3-(4-Acetyl-3-hydroxy-2-propylphenoxy)propyloxy)-gamma-oxo-beta,beta-dimethylbenzene-butanoic acid,

4-(3-(4-Acetyl-3-hydroxy-2-propylphenoxy)propyloxy)-gamma-hydroxy-beta,beta-dimethyl-benzenebutanoic acid, sodium salt,

4-(3-(4-Acetyl-3-hydroxy-2-propylphenoxy)-propylthio)-gamma-oxo-beta,beta-dimethylbenzene-butanoic acid,

4-(3-(4-Acetyl-3-hydroxy-2-propylphenoxy)propylthio)-gamma-hydroxy-beta,beta-dimethylbenzene-butanoic acid sodium salt,

(+) Isomer of (beta S*, gamma R*)-4-(3-(4-acetyl-3-hydroxy-2-propylphenoxy)propylthio)-gamma-hydroxy-beta-methylbenzenebutanoic acid sodium salt,

(−) Isomer of (beta S*, gamma R*)-4-(3-(4-acetyl-3-hydroxy-2-propylphenoxy)propylthio)-gamma-hydroxy-beta-methylbenzenebutanoic acid sodium salt,

(Beta S*, gamma R*) 4-(3-(4-acetyl-3-hydroxy-2-propylphenoxy)propylthio)-gamma-hydroxy-beta-methylbenzenebutanoic acid gamma lactone,

(Beta S*, gamma R*) 4-(3-(4-acetyl-3-hydroxy-2-propylphenoxy)propylsulfonyl)-gamma-hydroxy-beta-methylbenzenebutanoic acid gamma lactone,

(Beta S*, gamma R*) 4-(3-(4-acetyl-3-hydroxy-2-propylphenoxy)propylsulfonyl)-gamma-hydroxy-beta-methylbenzenebutanoic acid sodium salt,

(Beta R*, gamma R*) 4-(3-(4-acetyl-3-hydroxy-2-propylphenoxy)-propylsulfonyl)-gamma-hydroxy-beta-methylbenzenebutanoic acid sodium salt,

(Beta R*, gamma R*) 4-(3-(4-acetyl-3-hydroxy-2-propylphenoxy)propylthio)-gamma-hydroxy-beta-methylbenzenebutanoic acid sodium salt,

(Beta R*, gamma R*) 4-(3-(4-acetyl-3-hydroxy-2-propylphenoxy)propylsulfonyl)-gamma-hydroxy-beta-methylbenzenebutanoic acid gamma lactone,

(Beta R*, gamma R*)-(4-(3-(4-acetyl-3-hydroxy-2-propylphenoxy)propylthio)-gamma-hydroxy-beta-methylbenzenebutanoic acid gamma lactone,

(Beta S*, gamma R*)-4-(3-(4-acetyl-3-hydroxy-2-propylphenoxy)propylthio)-gamma-hydroxy-beta-methylbenzenebutanoic acid-S-oxide sodium salt,

(Alpha S*, beta R*, gamma R*)-4-(3-(4-acetyl-3-hydroxy-2-propylphenoxy)propylthio)-gamma-hydroxy-alpha,beta-dimethylbenzenebutanoic acid sodium salt,

4-(3-(4-Acetyl-3-hydroxy-2-propylphenoxy)propylthio)-gamma-hydroxybenzenebutanoic acid gamma lactone,

4-(3-(4-Acetyl-3-hydroxy-2-propylphenoxy)propylsulfonyl)-gamma-hydroxybenzenebutanoic acid gamma lactone,

4-((3-(4-Acetyl-6-fluoro-3-hydroxy-2-propylphenoxy)propyl)thio)-gamma-oxobenzenebutanoic acid,

4-((3-(4-Acetyl-6-chloro-3-hydroxy-2-propylphenoxy)propyl)thio)-gamma-oxobenzenebutanoic acid,

4-(3-(4-Acetyl-6-chloro-3-hydroxy-2-propylphenoxy)propylsulfonyl)-gamma-oxobenzenebutanoic acid,

4-((3-(4-Acetyl-6-bromo-3-hydroxy-2-propylphenoxy)propyl)thio)-gamma-oxobenzenbutanoic acid,

4-(3-(4-Acetyl-6-bromo-3-hydroxy-2-propylphenoxy)propylsulfonyl)-gamma-oxobenzenebutanoic acid,

4-((3-(4-Acetyl-3-hydroxy-6-methyl-2-propylphenoxy)propyl)thio)-gamma-oxobenzenebutanoic acid,

(Beta S*, gamma R*) 4-(3-(4-acetyl-6-fluoro-3-hydroxy-2-propylphenoxy)propylthio)-gamma-hydroxy-beta-methylbenzenebutanoic acid,

(Beta S*, gamma R*) 4-(3-(4-acetyl-5-chloro-3-hydroxy-2-propylphenoxy)propylthio)-gamma-hydroxy-beta-methylbenzenebutanoic acid,

(Beta S*, gamma R*) 4-(3-(4-acetyl-3-hydroxy-6-methyl-2-propylphenoxy)propylthio)-gamma-hydroxy-beta-methylbenzenebutanoic acid,

(Alpha S*, beta R*, gamma R*) 4-(3-(4-acetyl-3-hydroxy-2-propylphenoxy)propylthio)-gamma-hydroxy-alpha,beta-dimethylbenzenebutanoic acid gamma lactone,

(Beta S*, gamma R*) 4-(3-(4-acetyl-6-ethyl-3-hydroxy-2-propylphenoxy)propylthio)-gamma-hydroxy-beta-methylbenzenebutanoic acid,

4-(3-(4-Acetyl-6-fluoro-3-hydroxy-2-propylphenoxy)propyloxy)-gamma-hydroxy-beta,beta-dimethylbenzenebutanoic acid,

4-(3-(4-Acetyl-6-chloro-3-hydroxy-2-propylphenoxy)propyloxy)-gamma-hydroxy-beta,beta-dimethylbenzenebutanoic acid,

4-(3-(4-Acetyl-3-hydroxy-6-methyl-2-propylphenoxy)propyloxy)-gamma-hydroxy-beta,beta-dimethylbenzenebutanoic acid,

4-(3-(4-Acetyl-6-fluoro-3-hydroxy-2-propylphenoxy)propylthio)-gamma-hydroxy-beta,beta-dimethylbenzene-butanoic acid,

4-(3-(4-Acetyl-5-chloro-3-hydroxy-2-propylphenoxy)propylthio)-gamma-hydroxy-beta,beta-dimethylbenzenebutanoic acid,

4-(3-(4-Acetyl-3-hydroxy-6-methyl-2-propylphenoxy)propylthio)-gamma-hydroxy-beta,beta-dimethylbenzenebutanoic acid,

4-(3-(4-Acetyl-3-hydroxy-2-propylphenoxy)propylthio)-3-propyl-gamma-oxo-benzenebutanoic acid,

4-(3-(4-Acetyl-3-hydroxy-2-propylphenoxy)propylsufonyl)-3-propyl-gamma-oxobenzenebutanoic acid,

(Beta R*, gamma S*)-4-(3-(4-acetyl-3-hydroxy-2-propylphenoxy)propylthio)-3-fluoro-gamma-hydroxy-beta-methylbenzenebutanoic acid sodium salt,

(Beta R*, gamma S*) 4-(3-(4-acetyl-3-hydroxy-2-propylphenoxy)propylthio)-3-bromo-gamma-hydroxy-beta-methylbenzenebutanoic acid sodium salt,

(Beta R*, gamma S*) 4-(3-(4-acetyl-3-hydroxy-2-propylphenoxy)propylthio)-3-chloro-gamma-hydroxy-beta-methylbenzenebutanoic acid sodium salt,

4-(3-(4-Acetyl-3-hydroxy-2-propylphenoxy)-propylsulfonyl)-gammna-oxo-beta,beta-dimethylbenzenebutanoic acid,

4-(3-(4-Acetyl-3-hydroxy-2-propylphenoxy)propylsulfonyl)-gamma-hydroxy-beta,beta-dimethylbenzenebutanoic acid sodium salt,

4-(3-(4-Acetyl-3-hydroxy-2-propylphenoxy-propylthio)-3-propyl-gamma-oxo-beta,beta-dimethylbenzenebutanoic acid,

4-(3-(4-Acetyl-3-hydroxy-2-propylphenoxy)propylthio)-3-propyl-gamma-hydroxy-beta,beta-dimethylbenzenebutanoic acid sodium salt,

4-(3-(4-Acetyl-3-hydroxy-2-propylphenoxy)propylthio)-3-fluoro-gamma-oxo-beta,beta-dimethylbenzenebutanoic acid sodium salt,

4-(3-(4-Acetyl-3-hydroxy-2-propylphenoxy)propylthio)-3-fluoro-gamma-hydroxy-beta,beta-dimethylbenzenebutanoic acid sodium salt,

4-(3-(4-Acetyl-3-hydroxy-2-propylphenoxy)-propylthio)-3-chloro-gamma-oxo-beta,beta-dimethylbenzenebutanoic acid,

4-(3-(4-Acetyl-3-hydroxy-2-propylphenoxy)propylthio)-3-chloro-gamma-hydroxy-beta,beta-dimethylbenzenebutanoic acid sodium salt,

4-(3-(4-Acetyl-3-hydroxy-2-propylphenoxy)propylthio)-3-bromo-gamma-hydroxy-beta,beta-dimethylbenzenebutanoic acid sodium salt,

4-(3-(4-Acetyl-3-hydroxy-2-propylphenoxy)propyloxy)-3-fluoro-gamma-hydroxy-beta,beta-dimethylbenzenebutanoic acid sodium salt,

4-(3-(4-Acetyl-3-hydroxy-2-propylphenoxy)propyloxy)-3-bromo-gamma-hydroxy-beta,beta-dimethylbenzenebutanoic acid sodium salt,

4-(3-(4-Acetyl-3-hydroxy-2-propylphenoxy)propyloxy)-3-chloro-gamma-hydroxy-beta,beta-dimethylbenzenebutanoic acid sodium salt,

4-(3-(4-Acetyl-3-hydroxy-2-propylphenoxy)propyloxy-3-fluoro-gamma-oxo-beta,beta-dimethylbenzenebutanoic acid,

4-(3-(4-Acetyl-3-hydroxy-2-propylphenoxy)propyloxy)-3-chloro-gamma-oxo-beta,beta-dimethylbenzenebutanoic acid,

4-(3-(4-Acetyl-3-hydroxy-2-propylphenoxy)propyloxy)-3-bromo-gamma-oxo-beta,beta-dimethylbenzenebutanoic acid,

4-(3-(4-Acetyl-3-hydroxy-2-propylphenoxy)propyloxy)-3-propyl-gamma-oxo-beta,beta-dimethylbenzenebutanoic acid,

4-(3-(4-Acetyl-3-hydroxy-2-propylphenoxy)propylthio)-gamma-hydroxy-beta-methylbenzenebutanoic acid,

3-(3-(4-Acetyl-3-hydroxy-2-propylphenoxy)propylthio)-gamma-hydroxy-beta,beta-dimethylbenzenebutanoic acid sodium salt,

3-(3-(4-Acetyl-3-hydroxy-2-propylphenoxy)propyloxy)-gamma-hydroxy-beta,beta-dimethylbenzenebutanoic acid sodium salt,

3-(3-(4-Acetyl-3-hydroxy-2-propylphenoxy)propylthio)-gamma-oxobenzenebutanoic acid,

3-(3-(4-Acetyl-3-hydroxy-2-propylphenoxy)propylthio)-gamma-oxo-beta,beta-dimethylbenzenebutanoic acid,

3-(3-(4-Acetyl-3-hydroxy-2-propylphenoxy)propyloxy)-gamma-oxo-beta,beta-dimethylbenzenebutanoic acid,

(Beta R*, gamma S*) 4-(3-(4-acetyl-3-hydroxy-2-propylphenoxy)propylthio)-gamma-hydroxy-beta-methylbenzenebutanoic acid sodium salt,

4-(3-(4-Acetyl-3-hydroxy-2-propylphenoxy)propylsulfonyl)-3-propyl-gamma-oxobenzenebutanoic acid methyl ester,

4-(3-(4-Acetyl-3-hydroxy-2-propylphenoxy)propylthio)-3-propyl-gamma-oxobenzenebutanoic acid methyl ester,

(Beta R*, gamma S*) 4-(3-4-acetyl-3-hydroxy-2-propylphenoxy)propylthio)-3-propyl-gamma-hydroxy-beta-methylbenzenebutanoic acid,

4-(3-(4-Acetyl-3-hydroxy-2-propylphenoxy)propylsulfonyl)-gamma-oxo-beta,beta-dimethylbenzenebutanoic acid,

4-(3-(4-Acetyl-3-hydroxy-2-propylphenoxy)propylsulfonyl)-gamma-hydroxy-beta,beta-dimethylbenzenebutanoic acid,

4-(3-(4-Acetyl-3-hydroxy-2-propylphenoxy)propylthio)-3-propyl-gamma-oxo-beta,beta-dimethylbenzenebutanoic acid methyl ester,

4-(3-(4-Acetyl-3-hydroxy-2-propylphenoxy)propylthio)-3-fluoro-gamma-oxo-beta,beta-dimethylbenzenebutanoic acid,

4-(3-(4-Acetyl-3-hydroxy-2-propylphenoxy)propylthio)-3-fluoro-gamma-hydroxy-beta,beta-dimethylbenzenebutanoic acid,

(Beta R*, gamma S*) 4-(3-(4-acetyl-3-hydroxy-2-propylphenoxy)propylthio)-3-fluoro-gamma-hydroxy-beta-methylbenzenebutanoic acid gamma lactone,

(Beta S*, gamma R*) 4-(3-(4-acetyl-3-hydroxy-2-propylphenoxy)-propylthio)-3-chloro-gamma-hydroxy-beta-methylbenzenebutanoic acid gamma lactone,

(Beta S*, gamma R*) 4-(3-(4-acetyl-3-hydroxy-2-propylphenoxy)propylthio-3-bromo-gamma-hydroxy-beta-methylbenzenebutanoic acid gamma lactone,

(Beta S*, gamma R*) 4-(3-(4-acetyl-3-hydroxy-2-propylphenoxy)propylsulfonyl-3-fluoro-gamma-hydroxy-beta-methylbenzenebutanoic acid gamma lactone,

(Beta S*, gamma R*) 4-(3-(4-acetyl-3-hydroxy-2-propylphenoxypropoxy)-3-bromo-gamma-hydroxy-beta-methylbenzenebutanoic acid gamma lactone,

(Beta R* gamma S*) 4-(3-(4-acetyl-3-hydroxy-2-propylphenoxy)propylsulfonyl)-3-chloro-gamma-hydroxy-beta-methylbenzenebutanoic acid gamma lactone,

(Beta S*, gamma R*) 4-(3-(4-acetyl-3-hydroxy-2-propylphenoxy)propyloxy]-gamma-hydroxy-beta-methylbenzenebutanoic acid gamma lactone,

(Beta S*, gamma R*) 4-(3-(4-acetyl-3-hydroxy-2-propylphenoxy)propyloxy)-gamma-hydroxy-beta-methylbenzenebutanoic acid,

4-(3-(4-acetyl-3-hydroxy-2-propylphenylthio)propyloxy-3-fluoro-gamma-oxo-benzenebutanoic acid,

4-(3-(4-acetyl-3-hydroxy-2-propylphenylthio)propyloxy)-3-fluoro-gamma-oxo-benzenebutanoic acid-S-oxide,

4-(3-(4-acetyl-3-hydroxy-2-propylphenylsulfonyl)propyloxy)-3-fluoro-gamma-oxo-benzenebutanoic acid,

4-(3-(4-acetyl-3-hydroxy-2-propylphenylthio)-gamma-oxo-benzenebutanoic acid,

4-(3-(4-acetyl-3-hydroxy-2-propylthio)propylsulfonyl)-gamma-oxo-benzenebutanoic acid,

2-(3-(4-acetyl-3-hydroxy-2-propylphenoxy)propoxy)-3-fluoro-gamma-oxobenzenebutanoic acid,

2-(3-(4-acetyl-3-hydroxy-2-propylphenoxy)propoxy)-3-fluoro-epsilon-oxobenzenehexanoic acid,

4-(3-(4-acetyl-3-hydroxy-2-propylphenoxy)propoxy-2,3-dichlorogamma-oxobenzenebutanoic acid.

9. Prodrug derivatives of compounds as claimed in Claim 1 having the formula:

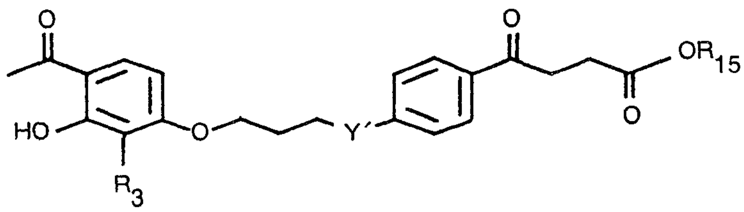

XV

in which Y′ and $OR_{15}$ are as follows:

| Y′ | $OR^{15}$ |
|---|---|
| S | 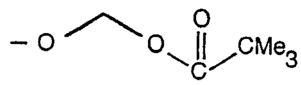 |
| $SO_2$ | 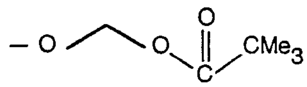 |
| S | 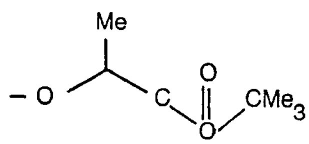 |
| $SO_2$ | 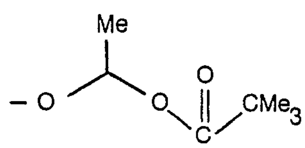 |
| S | 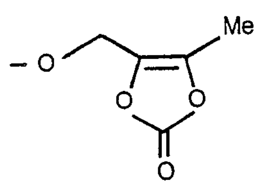 |
| $SO_2$ | 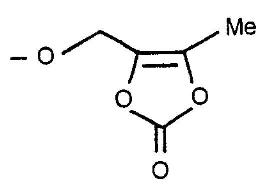 |

| Y′ | $OR^{15}$ |
|---|---|
| S | 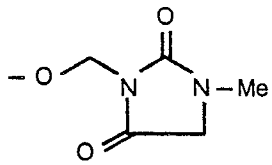 |
| $SO_2$ | 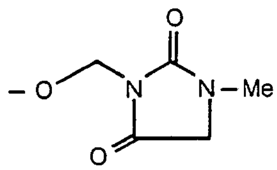 |
| S | 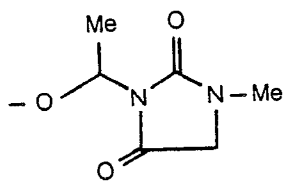 |
| $SO_2$ | 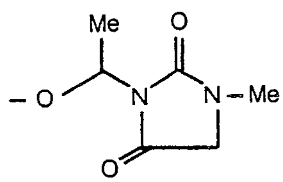 |
| S | 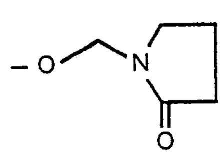 |
| $SO_2$ | 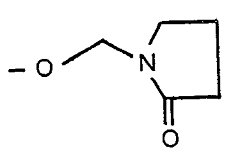 |

| Y′ | $OR^{15}$ |
| --- | --- |
| S | 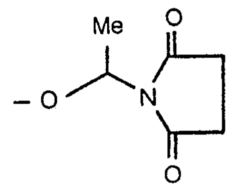 |
| $SO_2$ | 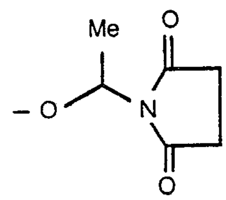 |
| S | 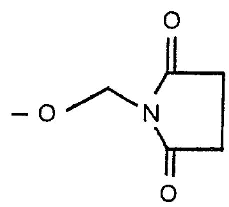 |
| $SO_2$ | 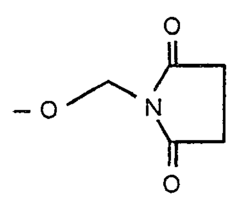 |

10. A pharmaceutical composition for antagonizing the leukotrienes in mammals, especially humans, containing an effective amount of a compound as claimed in Claim 1 and a pharmaceutically acceptable carrier.

11. A compound as claimed in Claim 1 for use in antagonizing leukotriene action in a mammal.

12. A method of preparing a compound as claimed in Claim 1 that comprises condensing a compound of the formula:

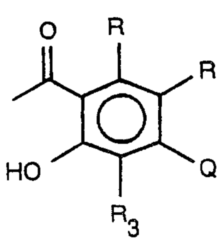

VII

where Q is 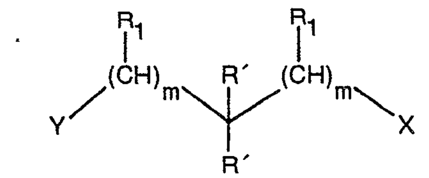 or

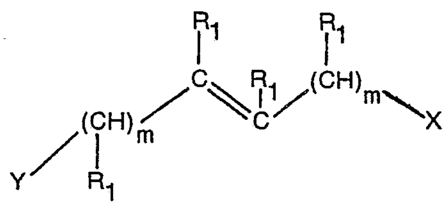 or 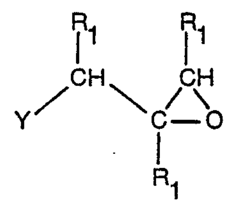

with a compound of the Formula

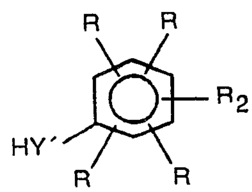

where R, $R_1$, $R_2$, $R_3$, R′, Y, Y′ and m have the meaning given in Claim 1, and X is halogen.

13. A method of preparing a compound as claimed in Claim 1 which comprises condensing a compound having one of the following Formulae

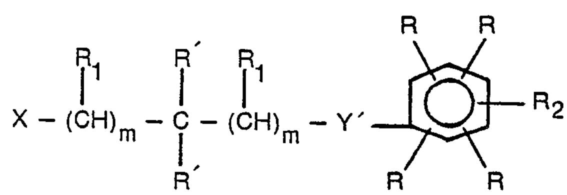

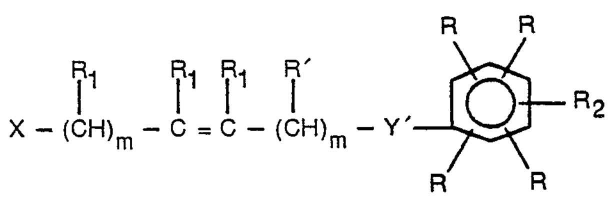

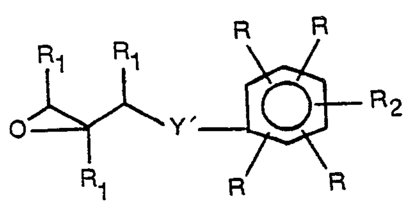

with a compound of the Formula

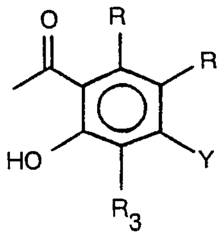

where R, $R_1$, $R_2$, $R_3$, $R^1$, Y, Y′ and m have the meaning given in Claim 1, and X is halogen.

14. A compound having one of the following Formulae

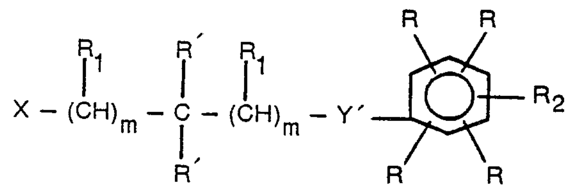

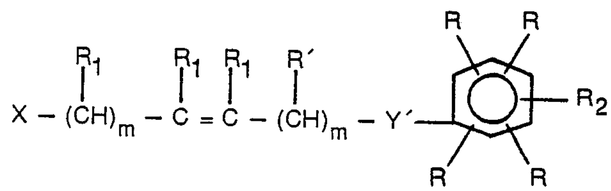

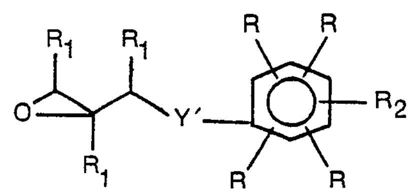

where Y′, R, $R_1$, $R_2$, R′ and m having the meaning given in Claim 1, and XC is halogen.

**Claims for the Contracting State: AT**

1. A method of preparing a compound having either of the following Formulae:

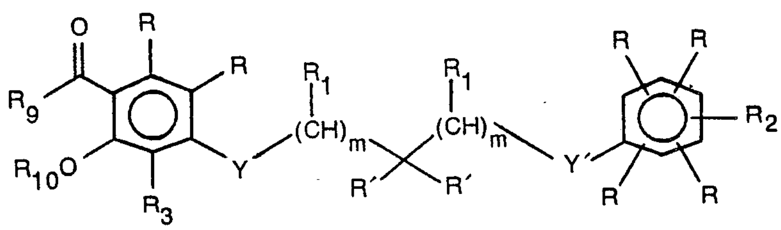

XI

and

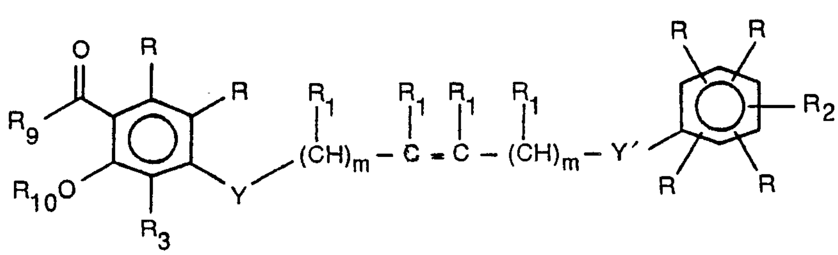

XII

where

each R is independently H, OH, alkyl of 1 to 6 carbon atoms which may be straight chain or branched; alkenyl of 2 to 6 carbon atoms which may be straight chain or branched; trifluoromethyl; alkoxy of 1 to 6 carbon atoms which may be straight chain or branched; SH; thioalkyl of 1 to 6 carbon atoms which may be straight chain or branched; phenyl; phenyl substituted by alkyl of 1 to 3 carbon atoms or by halogen; benzyl; phenalkyl with from 2 to 4 alkyl carbon atoms; halogen, amino; $N(R_4)_2$ wherein $R_4$ is H or alkyl of 1 to 6 carbon atoms which may be straight chain or branched; $COOR_4$; $CH_2OR_4$; formyl; CN; trifluoromethylthio; or nitro;

each R' is independently $R_4$; $OR_4$; $COOR_4$; $N(R_4)_2$; $SR_4$; $CH_2OR_4$; CHO; or together R' and R' are O, $CH_2$ or

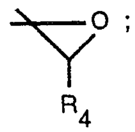

Y is oxygen, sulfur, sulfoxide, sulfone;

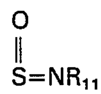

wherein $R_{11}$ is alkyl of 1—4 carbon atoms which may be straight chain or branched; $NR_{12}$ wherein $R_{12}$ is H, alkyl of 1—4 carbon atoms, which may be straight chain or branched, or

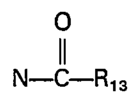

wherein $R_{13}$ is alkyl of 1—4 carbon atoms, which may be straight chain or branched, alkoxy of 1—4 carbon atoms, which may be straight chain or branched; or N—CN;

Y' is Y, —$CH_2$— or

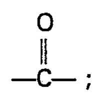

each $R_1$ is independently hydrogen or alkyl of 1—3 carbon atoms;

each m is independently an integer from 0—6; and $R_2$ is

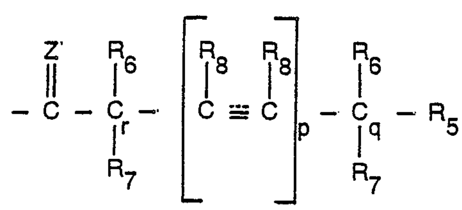

wherein

Z is O, S, $CH_2$, H and OH, alkenyl of 1—4 carbons; or N—$R_{14}$ wherein $R_{14}$ is OH, alkyl or alkoxy of 1 to 6 carbon atoms, perhaloalkyl of 1 to 6 carbon atoms, phenyl or phenyl substituted by alkyl or alkoxy groups of 1 to 3 carbon atoms, halogen, hydroxy, haloalkyl, COOH, CN, formyl or acyl of 1 to 6 carbon atoms;

each $R_6$ is independently H or alkyl of 1—4 carbons;

each $R_7$ is independently H, OH, or alkyl of 1—4 carbons;

each $R_8$ is independently H, or alkyl of 1—4 carbons, and is absent when a triple bond is present;

$R_5$ is $COOR_4$; $CH_2OH$; CHO; tetrazole; $NHSO_2R_{14}$; $CONHSO_2R_{14}$; hydroxymethylketone; CN; $CON(R_7)_2$; a monocyclic or bicyclic heterocyclic ring containing an acidic hydroxyl group; or $COOR_{15}$ where $R_{15}$ is:

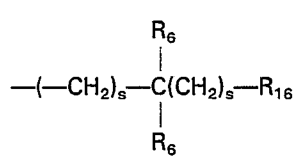

wherein each s is independently 0—3; $R_{16}$ is

A) a monocyclic or bicyclic heterocyclic radical containing from 3 to 12 nuclear carbon atoms and 1 to 2 nuclear heteroatoms selected from N and S with at least one being N, and with each ring in the heterocyclic radical being formed of 5 or 6 atoms, or

B) the radical W—$R_{17}$ wherein W is O, S or NH and $R_{17}$ contains up to 21 carbon atoms and is (1) a hydrocarbon radical (2) an acyl radical of an organic acyclic or monocyclic carboxylic acid containing not more than 1 heteroatom in the ring;

r and q are each independently 0—20 provided that the total of r and q does not exceed 20; and p is 0 or 1;

$R_3$ is alkyl of 1 to 6 carbon atoms which may be straight chain or branched; or alkenyl of 3 to 6 carbon atoms which may be straight chain or branched;

$R_9$ is alkyl of 1 to 6 carbon atoms which may be straight chain or branched; alkoxy of 1 to 6 carbon atoms which may be straight chain or branched; or $(CH_2)_rR_5$;

$R_{10}$ is H; alkyl of 1 to 6 carbon atoms which may be straight chain or branched;

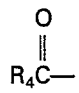 or $R_4OCH_2$— ;

or a compound that is a lactone thereof when, in $R_2$, Z is H and OH or $R_7$ is OH and $R_5$ is carboxy, or a compound that is a pharmaceutically acceptable salt or acid-addition salt thereof, that comprises condensing a compound of the formula:

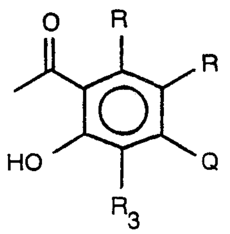

VII

where Q is 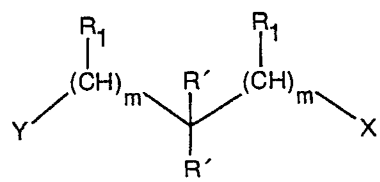 or

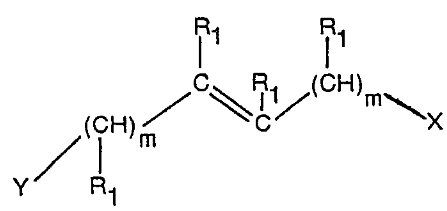 or 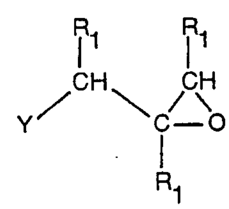

with a compound of the Formula

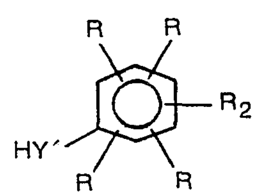

where R, $R_1$, $R_2$, $R_3$, R′, Y, Y′ and m are as defined and X is a halogen.

2. A method of preparing a compound having either of formula XI or XII in Claim 1, that comprises condensing a compound having one of the following Formulae

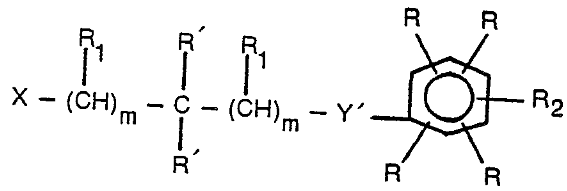

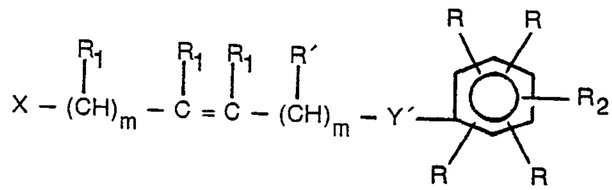

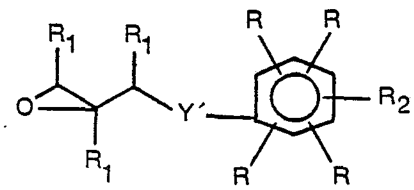

with a compound of the Formula

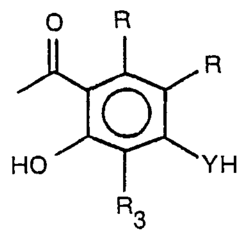

where R, $R_1$, $R_2$, $R_3$, $R^1$, Y, Y', *m* and X are as defined in Claim 1.

3. A method as claimed in Claim 1 or 2, as applied to the preparation of 4-((3-(4-acetyl-3-hydroxy-2-propylphenoxy)propyl)thio)-gamma-oxobenzenebutanoic acid.

4. A method as claimed in Claim 1 or 2, as applied to the preparation of 4-[3-(4-acetyl-3-hydroxy-2-propylphenoxy)propylsulfonyl)-gamma-oxobenzenebutanoic acid.

5. A method as claimed in Claim 1 or 2, as applied to the preparation of (beta S*, gamma R*) 4-(3-(4-acetyl-3-hydroxy-2-propylphenoxy)propylthio)-gamma-hydroxy-beta-methylbenzenebutanoic acid sodium salt.

6. A method as claimed in Claim 1 or 2, as applied to the preparation of (beta S*, gamma R*) 4-(3-(4-acetyl-3-hydroxy-2-propylphenoxy)propyloxy)-gamma-hydroxy-beta-methylbenzenebutanoic acid sodium salt.

7. A method as claimed in Claim 1 or 2, as applied to the preparation of 4-(3-(4-Acetyl-3-hydroxy-2-propylphenoxy))propylthio)-epsilon-oxobenzenehexanoic acid.

8. A method as claimed in Claim 1 or 2, as applied to the preparation of a compound in which Y is oxygen and Y' is oxygen, sulfur, sulfoxide, sulfone, amino or cyanamido.

9. A method as claimed in Claim 1, 2 or 7, as applied to the preparation of a compound in which each m is 1.

**Patentansprüche für die Vertragsstaaten: BE CH DE FR GB IT LI LU NL SE**

1. Eine Verbindung mit einer der folgenden Formeln

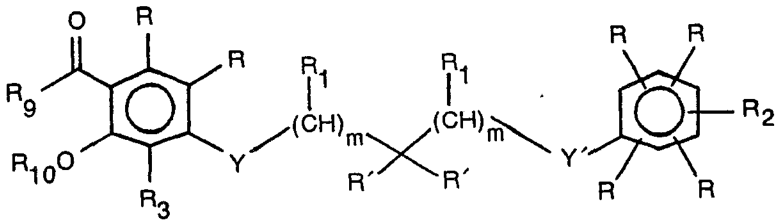

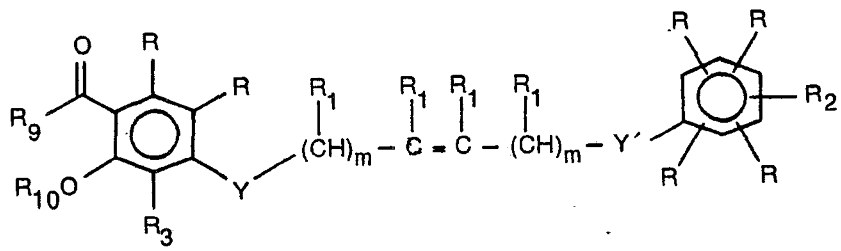

worin

jedes R unabhängig H, OH, Alkyl mit 1 bis 6 Kohlenstoffatomen, das geradkettig oder verzweigt sein kann; Alkenyl mit 2 bis 6 Kohlenstoffatomen, das geradkettig oder verzweigt sein kann; Trifluormethyl; Alkoxy mit 1 bis 6 Kohlenstoffatomen, das geradkettig oder verzweigt sein kann; SH; Thioalkyl mit 1 bis 6 Kohlenstoffatomen, das geradkettig oder verzweigt sein kann; Phenyl; Phenyl, substituiert durch Alkyl mit 1 bis 3 Kohlenstoffatomen oder durch Halogen; Benzyl; Phenylalkyl mit 2 bis 4 Alkylkohlenstoffatomen; Halogen, Amino; $N(R_4)_2$, worin $R_4$ H oder Alkyl mit 1 bis 6 Kohlenstoffatomen, das geradkettig oder verzweigt sein kann, ist; $COOR_4$; $CH_2OR_4$; Formyl; CH; Trifluormethylthio; oder Nitro bedeutet;

jedes R′ unabhängig $R_4$; $OR_4$; $COOR_4$; $N(R_4)_2$; $SR_4$; $CH_2OR_4$; CHO; oder zusammen R′ und R′ O; $CH_2$ oder

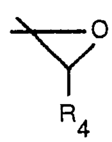 ;

Y Sauerstoff, Schwefel, Sufoxid, Sulfon;

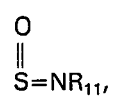

worin $R^{11}$ Alkyl mit 1 bis 4 Kohlenstoffatomen ist, das geradkettig oder verzweigt sein kann; $NR_{12}$, worin $R_{12}$ H, Alkyl mit 1 bis 4 Kohlenstoffatomen, das geradkettig oder verzweigt sein kann; oder

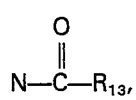

worin $R_{13}$ Alkyl mit 1 bis 4 Kohlenstoffatomen, das geradkettig oder verzweigt sein kann, Alkoxy mit 1 bis 4 Kohlenstoffatomen, das geradkettig oder verzweigt sein kann, bedeutet; oder N—CN darstellt;

Y′ Y, $—CH_2—$ oder

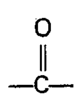 ist;

jedes $R_1$ unabhängig Wasserstoff oder Alkyl mit 1 bis 3 Kohlenstoffatomen ist;

jedes m unabhängig eine ganze Zahl von 0 bis 6 ist; und

$R_2$ 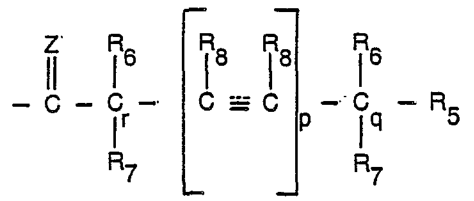 ist,

worin

Z O, S, $CH_2$, H und OH, Alkenyl mit 1 bis 4 Kohlenstoffatomen; oder N—$R_{14}$, worin $R_{14}$ OH, Alkyl oder Alkoxy mit 1 bis 6 Kohlenstoffatomen, Perhalogenalkyl mit 1 bis 6 Kohlenstoffatomen, Phenyl oder Phenyl, substituiert durch Alkyl- oder Alkoxygruppen mit 1 bis 3 Kohlenstoffatomen, Halogen, Hydroxy, Halogenalkyl, COOH, CN, Formyl oder Acyl mit 1 bis 6 Kohlenstoffatomen, ist; bedeutet;

jedes $R_6$ unabhängig H oder Alkyl mit 1 bis 4 Kohlenstoffatomen ist;

jedes $R_7$ unabhängig H, OH oder Alkyl mit 1 bis 4 Kohlenstoffatomen ist;

jedes $R_8$ unabhängig H oder Alkyl mit 1 bis 4 Kohlenstoffatomen ist und nicht vorhanden ist, wenn eine Dreifachbindung vorhanden ist;

$R_5$ $COOR_4$; $CH_2OH$; CHO; Tetrazol; $NHSO_2R_{14}$; $CONHSO_2R_{14}$ Hydroxymethylketon; CH; $CON(R_7)_2$; einen monocyclischen oder bicyclischen heterocyclischen Ring, der eine saure Hydroxylgruppe enthält; oder $COOR_{15}$, worin $R_{15}$ die Bedeutung hat von:

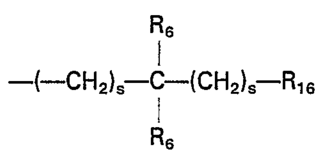

worin jedes s unabhängig 0—3 ist; bedeutet;

$R_{16}$ die Bedeutung hat von:

A) einem monocyclischen oder bicyclischen heterocyclischen Rest, der 3 bis 12 Kernkohlenstoffatome und 1 oder 2 Kernheteroatome, ausgewählt aus N und S, wobei mindestens eines N ist, enthält und wobei jeder Ring in dem heterocyclischen Rest aus 5 oder 5 Atomen gebildet wird; oder

B) dem Rest W—$R_{17}$, worin W O, S oder NH ist, und $R_{17}$ bis zu 21 Kohlenstoffatome enthält und (1) ein Kohlenwasserstoffrest oder (2) ein Acylrest einer organischen acyclischen oder monocyclischen Carbonsäure, die nicht mehr als ein Heteroatom im Ring enthält, ist;

r und q jeweils unabhängig 0 bis 20 sind, vorausgesetzt, daß die Gesamtheit von r und q 20 nicht überschreitet; und

p 0 oder 1 ist;

$R_3$ Alkyl mit 1 bis 6 Kohlenstoffatomen, das geradkettig oder verzweigt sein kann; oder Alkenyl mit 3 bis 6 Kohlenstoffatomen, das geradkettig oder verzweigt sein kann; ist;

$R_9$ Alkyl mit 1 bis 6 Kohlenstoffatomen, das geradkettig oder verzweigt sein kann; Alkoxy mit 1 bis 6 Kohlenstoffatomen, das geradkettig oder verzweigt sein kann; oder $(CH_2)_rR_5$; ist;

$R_{10}$ H; Alkyl mit 1 bis 6 Kohlenstoffatomen, das geradkettig oder verzweigt sein kann;

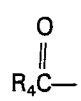

oder $R_4OCH_2$—; ist;

oder eine Verbindung, die ein Lacton davon ist, wenn in $R_2$, Z H und OH ist oder $R_7$ OH ist und $R_5$ Carboxy ist, oder eine Verbindung, die ein pharmazeutisch brauchbares Salz oder Säureadditionssalze davon ist.

2. Eine Verbindung, wie in Anspruch 1 beansprucht, worin Y Sauerstoff ist und Y' Sauerstoff, Schwefel, Sufoxid, Sulfon, Amino oder Cyanamido ist.

3. Eine Verbindung, wie in Anspruch 1 oder 2 beansprucht, worin jedes m 1 ist.

4. 4-((3-(4-Acetyl-3-hydroxy-2-propylphenoxy)-propyl)-thio)-gamma-oxobenzolbuttersäure.

5. 4-[3-(4-Acetyl-3-hydroxy-2-propylphenoxy)-propylsulfonyl]-gamma-oxobenzolbuttersäure.

6. (Beta S*, gamma R*)-4-(3-(4-acetyl-3-hydroxy-2-propylphenoxy)-propylthio)-gamma-hydroxy-beta-methylbenzolbuttersäure, Natriumsalz.

7. (Beta S*, gamma R*)-4-(3-(4-acetyl-3-hydroxy-2-propylphenoxy)-propyloxy)-gamma-hydroxy-beta-methyl-benzolbuttersäure, Natriumsalz.

8. Eine Verbindung, wie in Anspruch 1 beansprucht, mit folgender Bezeichnung:
4-(3-(4-Acetyl-3-hydroxy-2-propylphenoxy)-propoxy)-3-fluor-gamma-oxobenzolbuttersäure,
4-(3-(4-Acetyl-3-hydroxy-2-propylphenoxy)-propoxy)-3-fluor-gamma-oxobenzolbuttersäure,
4-((3-(4-Acetyl-3-hydroxy-2-propylphenoxy)-2-hydroxypropyl)-thio)-2-fluor-gamma-oxobenzolbuttersäure,
4-((3-(4-Acetyl-3-hydroxy-2-propylphenoxy)-2-hydroxypropyl)thio)-2-hydroxy-gamma-oxobenzolbuttersäure,
4-((3-(4-Acetyl-3-hydroxy-2-propylphenoxy)propyl)thio)-2-hydroxy-gamma-oxobenzolbuttersäure,
4-((3-(4-Acetyl-3-hydroxy-2-propylphenoxy)-2-hydroxypropyl)thio)-2-methoxy-gamma-oxobenzolbuttersäure,
4-((3-(4-Acetyl-3-hydroxy-2-propylphenoxy)propyl)thio)-3-fluor-gamma-oxobenzolbuttersäure,
4-((3-(4-Acetyl-3-hydroxy-2-propylphenoxy)propyl)thio)-2-fluor-gamma-oxobenzolbuttersäure,
4-(3-(4-Acetyl-3-hydroxy-2-propylphenoxy)propoxy)-3-fluor-gamma-oxobenzolhexansäure,
4-((3-(4-Acetyl-3-hydroxy-2-propylphenoxy)propoxy)-2-fluor-gamma-oxobenzolbuttersäure,
4-(3-(4-Acetyl-3-hydroxy-2-propylphenoxy)propoxy)-gamma-oxobenzolbuttersäure-methylester,
4-(3-(4-Acetyl-3-hydroxy-2-propylphenoxy)propoxy)-3-methyl-gamma-oxobenzolbuttersäure,
4-(3-(4-Acetyl-3-hydroxy-2-propylphenoxy)propoxy)-2-chlor-gamma-oxobenzolbuttersäure,
4-(4-(4-Acetyl-3-hydroxy-2-propylphenoxy)-2-butenoxy)-3-fluor-gamma-oxobenzolbuttersäure,
4-(3-(4-Acetyl-3-hydroxy-2-propylphenoxy)-2-hydroxypropylthio)-3-fluor-gamma-oxobenzolbuttersäure S-oxid,
4-(3-(4-Acetyl-3-hydroxy-2-propylphenoxy)propylthio)-3-fluor-gamma-oxobenzolbuttersäure, S-oxid,
4-(3-(4-Acetyl-3-hydroxy-2-propylphenoxy)propylthio)-2-fluor-gamma-oxobenzolbuttersäure S-oxid,
4-(3-(4-Acetyl-3-hydroxy-2-propylphenoxy)-2-hydroxypropylthio)-2-fluor-gamma-oxobenzolbuttersäure S-oxid,
4-(3-(4-Acetyl-3-hydroxy-2-propylphenoxy)propylsulfonyl)-2-fluor-gamma-oxobenzolbuttersäure,
4-(3-(4-Acetyl-3-hydroxy-2-propylphenoxy)-2-hydroxypropylsulfonyl)-2-fluoro-gamma-oxobenzolbuttersäure,
4-(3-(4-Acetyl-3-hydroxy-2-propylphenoxy)propylsulfonyl)-3-fluor-gamma-oxobenzolbuttersäure,
4-(3-(4-Acetyl-3-hydroxy-2-propylphenoxy)-2-hydroxypropylsulfonyl)-3-fluor-gamma-oxobenzolbuttersäure,
4-(3-(4-Acetyl-3-hydroxy-2-propylphenoxy)-2-hydroxypropylsulfonyl)-gamma-oxobenzolbuttersäure,
4-(3-(4-Acetyl-3-hydroxy-2-propylphenoxy)propoxy)-3-chlor-gamma-oxobenzolbuttersäure,
4-(3-(4-Acetyl-3-hydroxy-2-propylphenoxy)-2-hydroxypropoxy)-3-fluor-gamma-oxobenzolbuttersäure, Natriumsalz, Monohydrat
4-(3-(4-Acetyl-3-hydroxy-2-propylphenoxy)propoxy)-alpha, alpha-dimethyl-gamma-oxobenzolbuttersäure,
4-(3-(4-Acetyl-3-hydroxy-2-propylphenoxy)propoxy)beta-methyl-gamma-oxobenzolbuttersäure,
4-(3-(4-Acetyl-3-hydroxy-2-propylphenoxy)-2-methylidenylpropoxy)-gamma-oxobenzolbuttersäure,
4-(3-(4-Acetyl-6-chloro-3-hydroxy-2-propylphenoxy)-2-methylidenepropoxy)-gamma-oxobenzolbuttersäure,
4-(3-(4-Acetyl-3-hydroxy-2-propylphenoxy)-2-methylidenepropylthio)-gamma-oxobenzolbuttersäure,
4-(3-(4-Acetyl-3-hydroxy-2-propylphenoxy)-2-methylidenepropoxy)-3-fluoro-gamma-oxobenzolbuttersäure,
4-(3-(4-Acetyl-3-hydroxy-2-propyl-phenoxy)propylthio)-gamma-oxobenzolbutyronitril,
5-(3(4-(3-(4-Acetyl-3-hydroxy-2-propylphenoxy)propylthio)phenyl)-3-oxopropyl)-1H-tetrazol,
4(3(4-Acetyl-3-hydroxy-2-propylphenoxy)propylsulfonyl)-gamma-oxobenzolbutyronitril,
5-(3-(4-(3-(4-Acetyl-3-hydroxy-2-propylphenoxy)propylsulfonylphenyl)-3-oxopropyl)-1H-tetrazol
4-(3-(4-Acetyl-3-hydroxy-2-propylphenoxy)propylthio)-epsilon-oxobenzolhexansäure,
4(3-(4-Acetyl-3-hydroxy-2-propylphenoxy)propylsulfonyl)-epsilon-oxobenzolhexansäure,
4-(3-(4-Acetyl-3-hydroxy-2-propylphenoxy)-propylthio)-delta-oxobenzolbutanol,
4(3-(4-Acetyl-3-hydroxy-2-propylphenoxy)propylsulfonyl)-delta-oxobenzolbutanol,
4-(3-(4-Acetyl-3-hydroxy-2-propylphenoxy)propylthio-beta epsilon-dioxo-benzolpentanol,
4-(3-(4-Acetyl-3-acetoxy-2-propylphenoxy)propylthio)-gamma-oxobenzolbuttersäure,
4-(3-(4-Acetyl-3-acetoxy-2-propylphenoxy)-propylsulfonyl)-gamma-oxobenzolbuttersäure,
4-(3-(4-Acetyl-3-hydroxy-2-propylphenoxy)-propylsulfonyl-gamma-oxobenzolbutyramid,
N-Methylsulfonyl 4-(3-(4-acetyl-3-hydroxy-2-propylphenoxy)propylthio-gamma-oxo-benzolbutyramid,
5(4-(3-(4-Acetyl-3-hydroxy-2-propylphenoxy)propylsulfonyl)phenyl)-2(3H)-furanon,
4-(3-(4-Acetyl-3-hydroxy-2-propylphenoxy)propyloxy)-gamma-oxo-beta, beta-dimethylbenzolbuttersäure,
4-(3-(4-Acetyl-3-hydroxy-2-propylphenoxy)propyloxy)-gamma-hydroxy-beta, beta-dimethylbenzolbuttersäure, Natriumsalz,

4-(3-(4-Acetyl-3-hydroxy-2-propylphenoxy)-propylthio)-gamma-oxo-beta, beta-dimethylbenzolbuttersäure,

4-(3-(4-Acetyl-3-hydroxy-2-propylphenoxy)propylthio)-gamma-hydroxy-beta, beta-dimethylbenzolbuttersäure, Natriumsalz,

(+) Isomer von (beta S*, gamma R*)-4-(3-(4-Acetyl-3-hydroxy-2-propylphenoxy)propylthio)-gamma-hydroxy-beta-methylbenzolbuttersäure, Natriumsalz,

(–) Isomer von (beta S*, gamma R*)-4-(3-(4-Acetyl-3-hydroxy-2-propylphenoxy)propylthio)-gamma-hydroxy-beta-methylbenzolbuttersäure, Natriumsalz,

(Beta S*, gamma R*) 4-(3-(4-Acetyl-3-hydroxy-2-propylphenoxy)propylthio)gamma-hydroxy-beta-methylbenzolbuttersäure, gamma Lacton,

(Beta S*, gamma R*) 4-(3-(4-Acetyl-3-hydroxy-2-propylphenoxy)propylsulfonyl)-gamma-hydroxy-beta-methylbenzolbuttersäure, gamma Lacton,

(Beta S*, gamma R*) 4-(3-(4-cetyl-3-hydroxy-2-propylphenoxy)propylsulfonyl)-gamma-hydroxy-beta-methylbenzolbuttersäure, Natriumsalz,

(Beta R*, gamma R*) 4-(3-(4-Acetyl-3-hydroxy-2-propylphenoxy)propylsulfonyl)-gamma-hydroxy-beta-methylbenzolbuttersäure, Natriumsalz,

(Beta R*, gamma R*) 4-(3-(4-Acetyl-3-hydroxy-2-propylphenoxy)propylthio)-gamma-hydroxy-beta-methylbenzolbuttersäure, Natriumsalz,

(Beta R*, gamma R*) 4-(3-(4-Acetyl-3-hydroxy-2-propylphenoxy)propylsulfonyl)-gamma-hydroxy-beta-methylbenzolbuttersäure, gamma Lacton,

(Beta R*, gamma R*)-(4-(3-(4-Acetyl-3-hydroxy-2-propylphenoxy)propylthio)-gamma-hydroxy-beta-methyl-benzolbuttersäure, gamma Lacton,

(Beta S*, gamma R*)-4-(3-(4-Acetyl-3-hydroxy-2-propylphenoxy)propylthio)-gamma-hydroxy-beta-methylbenzolbuttersäure-S-oxid, Natriumsalz,

(Alpha S*, beta R*, gamma R*)-4-(3-(4-Acetyl-3-hydroxy-2-propylphenoxy)propylthio)-gamma-hydroxy-alpha, beta-dimethylbenzolbuttersäure, Natriumsalz,

4-(3-(4-Acetyl-3-hydroxy-2-propylphenoxy)propylthio)-gamma-hydroxybenzolbuttersäure, gamma Lacton,

4-(3-(4-Acetyl-3-hydroxy-2-propylphenoxy)propylsulfonyl)-gamma-hydroxybenzolbuttersäure, gamma Lacton,

4-((3-(4-Acetyl-6-fluor-3-hydroxy-2-propylphenoxy)propyl)thio)-gamma-oxobenzolbuttersäure,

4-((3-(4-Acetyl-6-chloro-3-hydroxy-2-propylphenoxy)propyl)thio)-gamma-oxobenzolbuttersäure,

4-(3-(4-Acetyl-6-chloro-3-hydroxy-2-propylphenoxy)propylsulfonyl)-gamma-oxobenzolbuttersäure,

4-((3-(4-Acetyl-6-bromo-3-hydroxy-2-propylphenoxy)propyl)thio)-gamma-oxobenzolbuttersäure,

4-(3-(4-Acetyl-6-bromo-3-hydroxy-2-propylphenoxy)propylsulfonyl)-gamma-oxobenzolbuttersäure,

4-((3-(4-Acetyl-3-hydroxy-6-methyl-2-propylphenoxy)propyl)thio)-gamma-oxobenzolbuttersäure,

(Beta S*, gamma R*) 4-(3-(4-acetyl-6-fluoro-3-hydroxy-2-propylphenoxy)propylthio)-gamma-hydroxy-beta-methylbenzolbuttersäure,

(Beta S*, gamma R*) 4-(3-(4-acetyl-5-chloro-3-hydroxy-2-propylphenoxy)propylthio)-gamma-hydroxy-beta-methylbenzolbuttersäure,

(Beta S*, gamma R*) 4-(3-(4-acetyl-3-hydroxy-6-methyl-2-propylphenoxy)propylthio)-gamma-hydroxy-beta-methylbenzolbuttersäure,

(Alpha S*, beta R*, gamma R*) 4-(3-(4-acetyl-3-hydroxy-2-propylphenoxy)propylthio)-gamma-hydroxy-alpha, beta-dimethylbenzolbuttersäure, gamma Lacton,

(Beta S*, gamma R*) 4-(3-(4-acetyl-6-ethyl-3-hydroxy-2-propylphenoxy)propylthio)-gamma-hydroxy-beta-methylbenzolbuttersäure,

4-(3-(4-Acetyl-6-fluoro-3-hydroxy-2-propylphenoxy)propyloxy)-gamma-hydroxy-beta, beta-dimethylbenzolbuttersäure,

4-(3-(4-Acetyl-6-chloro-3-hydroxy-2-propylphenoxy)propyloxy)-gamma-hydroxy-beta, beta-dimethylbenzolbuttersäure,

4-(3-(4-Acetyl-3-hydroxy-6-methyl-2-propylphenoxy)propyloxy)-gamma-hydroxy-beta, beta-dimethylbenzolbuttersäure,

4-(3-(4-Acetyl-6-fluoro-3-hydroxy-2-propylphenoxy)propylthio)-gamma-hydroxy-beta, beta-dimethylbenzolbuttersäure,

4-(3-(4-Acetyl-5-chloro-3-hydroxy-2-propylphenoxy)propylthio)-gamma-hydroxy-beta, beta-dimethylbenzolbuttersäure,

4-(3-(4-Acetyl-3-hydroxy-6-methyl-2-propylphenoxy)propylthio)-gamma-hydroxy-beta, beta-dimethylbenzolbuttersäure,

4-(3-(4-Acetyl-3-hydroxy-2-propylphenoxy)propylthio)-3-propyl-gamma-oxo-benzolbuttersäure,

4-(3-(4-Acetyl-3-hydroxy-2-propylphenoxy)propylsulfonyl)-3-propyl-gamma-oxobenzolbuttersäure,

(Beta R*, gamma S*)-4-(3-(4-acetyl-3-hydroxy-2-propylphenoxy)propylthio)-3-fluoro-gamma-hydroxy-beta-methylbenzolbuttersäure, Natriumsalz,

(Beta R*, gamma S*) 4-(3-(4-acetyl-3-hydroxy-2-propylphenoxy)propylthio)-3-bromo-gamma-hydroxy-beta-methylbenzolbuttersäure, Natriumsalz,

(Beta R*, gamma S*) 4-(3-(4-acetyl-3-hydroxy-2-propylphenoxy)propylthio)-3-chloro-gamma-hydroxy-beta-methylbenzolbuttersäure, Natriumsalz,

4-(3-(4-Acetyl-3-hydroxy-2-propylphenoxy)-propylsulfonyl)-gamma-oxo-beta, beta-dimethyl-benzolbuttersäure,

4-(3-(4-Acetyl-3-hydroxy-2-propylphenoxy)propylsulfonyl)-gamma-hydroxy-beta, beta-dimethyl-benzolbuttersäure, Natriumsalz,

4-(3-(4-Acetyl-3-hydroxy-2-propylphenoxy-propylthio)-3-propyl-gamma-oxo-beta, beta-dimethyl-benzolbuttersäure,

4-(3-(4-Acetyl-3-hydroxy-2-propylphenoxy)propylthio)-3-propyl-gamma-hydroxy-beta, beta-dimethyl-benzolbuttersäure, Natriumsalz,

4-(3-(4-Acetyl-3-hydroxy-2-propylphenoxy)propylthio)-3-fluoro-gamma-oxo-beta, beta-dimethylbenzol-buttersäure, Natriumsalz,

4(3-(4-Acetyl-3-hydroxy-2-propylphenoxy)propylthio)-3-fluoro-gamma-hydroxy-beta, beta-dimethyl-benzolbuttersäure, Natriumsalz,

4-(3-(4-Acetyl-3-hydroxy-2-propylphenoxy)-propylthio)-3-chloro-gamma-oxo-beta, beta-dimethyl-benzolbuttersäure,

4-(3-(4-Acetyl-3-hydroxy-2-propylphenoxy)propylthio)-3-chloro-gamma-hydroxy-beta, beta-dimethyl-benzolbuttersäure, Natriumsalz,

4-(3-(4-Acetyl-3-hydroxy-2-propylphenoxy)propylthio)-3-bromo-gamma-hydroxy-beta, beta-dimethylbenzolbuttersäure, Natriumsalz,

4-(3-(4-Acetyl-3-hydroxy-2-propylphenoxy)propyloxy)-3-fluoro-gamma-hydroxy-beta, beta-dimethylbenzolbuttersäure, Natriumsalz,

4-(3-(4-Acetyl-3-hydroxy-2-propylphenoxy)propyloxy)-3-bromo-gamma-hydroxy-beta, betadimethylbenzolsäure, Natriumsalz,

4-(3-(4-Acetyl-3-hydroxy-2-propylphenoxy)propyloxy)-3-chloro-gamma-hydroxy-beta, beta-dimethylbenzolbuttersäure, Natriumsalz,

4-(3-(4-Acetyl-3-hydroxy-2-propylphenoxy)propyloxy-3-fluoro-gamma-oxo-beta, beta-dimethyl-benzolbuttersäure,

4-(3-(4-Acetyl-3-hydroxy-2-propylphenoxy)propyloxy)-3-chloro-gamma-oxo-beta, beta-dimethylbenzolbuttersäure,

4-(3-(4-Acetyl-3-hydroxy-2-propylphenoxy)propyloxy)-3-bromo-gamma-oxo-beta, beta-dimethyl-benzolbuttersäure,

4-(3-(4-Acetyl-3-hydroxy-2-propylphenoxy)propyloxy)-3-propyl-gamma-oxo-beta, beta-dimethyl-benzolbuttersäure,

4-(3-(4-Acetyl-3-hydroxy-2-propylphenoxy)propylthio)-gamma-hydroxy-beta-methylbenzol-buttersäure,

3-(3-(4-Acetyl-3-hydroxy-2-propylphenoxy)propylthio)-gamma-hydroxy-beta, beta-dimethylbenzol-buttersäure, Natriumsalz,

3-(3-(4-Acetyl-3-hydroxy-2-propylphenoxy)propyloxy)-gamma-hydroxy-beta, beta-dimethylbenzol-buttersäure, Natriumsalz,

3-(3-(4-Acetyl-3-hydroxy-2-propylphenoxy)propylthio)-gamma-oxobenzolbuttersäure,

3-(3-(4-Acetyl-3-hydroxy-2-propylphenoxy)propylthio)-gamma-oxo-beta, beta, dimethylbenzol-buttersäure,

3-(3-(4-Acetyl-3-hydroxy-2-propylphenoxy)propyloxy)-gamma-oxo-beta, beta-dimethylbenzol-buttersäure,

(Beta R*, gamma S*) 4-(3-(4-acetyl-3-hydroxy-2-propylphenoxy)propylthio)-gamma-hydroxy-beta-methylbenzolbuttersäure, Natriumsalz,

4-(3-(4-Acetyl-3-hydroxy-2-propylphenoxy)propylsulfonyl)-3-propyl-gamma-oxobenzolbuttersäure-methylester,

4-(3-(4-Acetyl-3-hydroxy-2-propylphenoxy)propylthio)-3-propyl-gamma-oxo-benzolbuttersäure-methylester,

(Beta R*, gamma S*) 4-(3-4-acetyl-3-hydroxy-2-propylphenoxy)propylthio)-3-propyl-gamma-hydroxy-beta-methylbenzolbuttersäure,

4-(3-(4-Acetyl-3-hydroxy-2-propylphenoxy)propylsulfonyl)-gamma-oxo-beta, beta-dimethylbenzol-buttersäure,

4-(3-(4-Acetyl-3-hydroxy-2-propylphenoxy)propylsulfonyl)-gamma-hydroxy-beta, beta-dimethyl-benzolbuttersäure,

4-(3-(4-Acetyl-3-hydroxy-2-propylphenoxy)propylthio)-3-propyl-gamma-oxo-beta, beta-dimethyl-benzolbuttersäure-methylester,

4-(3-(4-Acetyl-3-hydroxy-2-propylphenoxy)propylthio)-3-fluoro-gamma-oxo-beta, beta-dimethyl-benzolbuttersäure,

4-(3-(4-Acetyl-3-hydroxy-2-propylphenoxy)propylthio)-3-fluoro-gamma-hydroxy-beta, beta-dimethylbenzolbuttersäure,

(Beta R*, gamma S*) 4-(3-(4-acetyl-3-hydroxy-2-propylphenoxy)propylthio)-3-fluoro-gamma-hydroxy-beta-methylbenzolbuttersäure, gamma Lacton,

(Beta S*, gamma R*) 4-(3-(4-acetyl-3-hydroxy-2-propylphenoxy)-propylthio)-3-chlor-gamma-hydroxy-beta-methylbenzolbuttersäure, gamma Lacton,

(Beta S*, gamma R*) 4-(3-(4-acetyl-3-hydroxy-2-propylphenoxy)propylthio-3-brom-gamma-hydroxy-beta-methylbenzolbuttersäure, gamma Lacton,

(Beta S*, gamma R*) 4-(3-(4-Acetyl-3-hydroxy-2-propylphenoxy)propylsulfonyl-3-fluor-gamma-hydroxy-beta-methylbenzolbuttersäure, gamma Lacton,

(Beta S*, gamma R*) 4-(3-(4-Acetyl-3-hydroxy-2-propylphenoxypropoxy)-3-bromo-gamma-hydroxy-beta-methylbenzolbuttersäure, gamma Lacton,

(Beta R*, gamma S*) 4-(3-(4-Acetyl-3-hydroxy-2-propylphenoxy)propylsulfonyl)-3-chlor-gamma-hydroxy-beta-methylbenzolbuttersäure, gamma Lacton,

(Beta S*, gamma R*) 4-(3-(4-Acetyl-3-hydroxy-2-propylphenoxy)propyloxy]-gamma-hydroxy-beta-methyl-benzolbuttersäure, gamma Lacton,

(Beta S*, gamma R*) 4-(3-(4-Acetyl-3-hydroxy-2-propylphenoxy)propyloxy)-gamma-hydroxy-beta-methyl-benzolbuttersäure,

4-(3-(4-acetyl-3-hydroxy-2-propylphenylthio)propyloxy)-3-fluoro-gamma-oxo-benzolbuttersäure,

4-(3-(4-acetyl-3-hydroxy-2-propylphenylthio)propyloxy)-3-fluoro-gamma-oxo-benzolbuttersäure-S-oxid,

4-(3-(4-acetyl-3-hydroxy-2-propylphenylsulfonyl)propyloxy)-3-fluoro-gamma-oxo-benzolbuttersäure,

4-(3-(4-Acetyl-3-hydroxy-2-propylphenylthio)-propylthio)-gamma-oxo-benzolbuttersäure,

4-(3-(4-Acetyl-3-hydroxy-2-propylphenylthio)-propylsulfonyl)-gamma-oxobenzolbuttersäure,

2-(3-(4-Acetyl-3-hydroxy-2-propylphenoxy)-propoxy)-3-fluor-gamma-oxobenzolbuttersäure,

2-(3-(4-Acetyl-3-hydroxy-2-propylphenoxy)-propoxy)-3-fluor-epsilon-oxobenzolhexansäure,

4-(3-(4-Acetyl-3-hydroxy-2-propylphenoxy)-propoxy-2,3-dichlor-gamma-oxobenzolbuttersäure.

9. Arzneimittelvorproduktderivate von Verbindungen, wie in Anspruch 1 beansprucht, mit der Formel

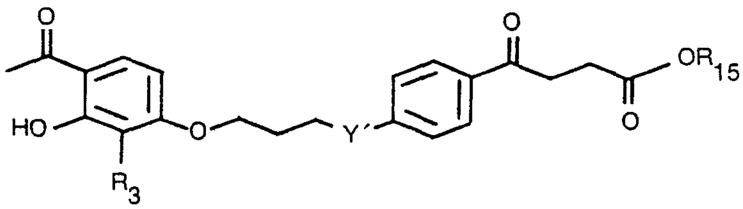

XV

worin Y' und $OR_{15}$ wie folgt sind:

| Y′ | $OR^{15}$ |
|---|---|
| S | 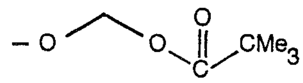 |
| $SO_2$ | 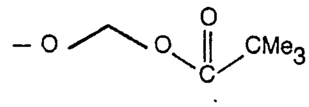 |
| S | 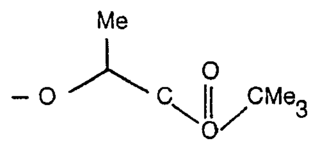 |
| $SO_2$ | 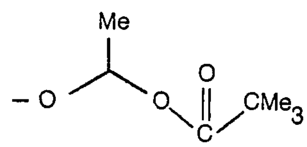 |
| S | 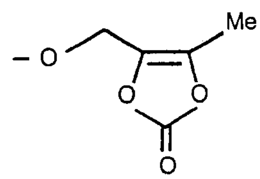 |
| $SO_2$ | 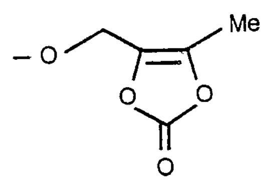 |
| S | 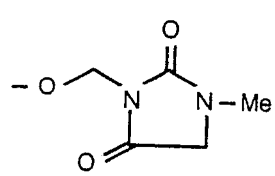 |
| $SO_2$ | 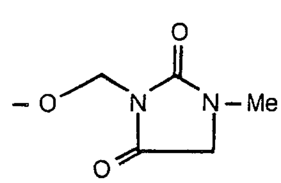 |

| Y´ | $OR^{15}$ |
|---|---|
| S | 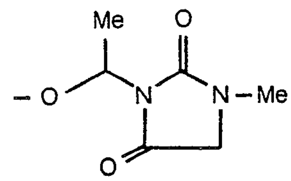 |
| $SO_2$ | 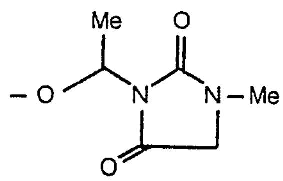 |
| S | 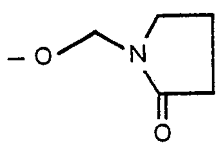 |
| $SO_2$ | 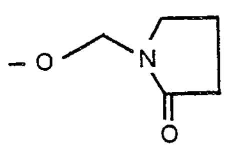 |
| S | 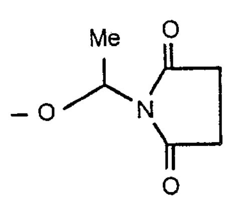 |
| $SO_2$ | 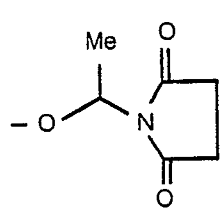 |

| Y′ | $OR^{15}$ |
|---|---|
| S | 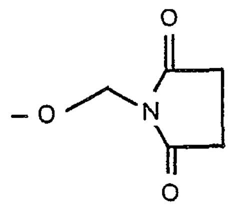 |
| $SO_2$ | 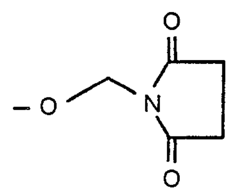 |

10. Eine pharmazeutische Zusammensetzung zur Antagonisierung der Leukotriene bei Säugern, insbesondere Menschen, enthaltend eine wirksame Menge einer Verbindung, wie in Anspruch 1 beansprucht, und einen pharmazeutisch brauchbaren Träger.

11. Eine Verbindung, wie in Anspruch 1 beansprucht, zur verwendung bei der Antagonisierung der Leukotrienwirkung bei einem Säuger.

12. Ein Verfahren zur Herstellung einer Verbindung, wie in Anspruch 1 beansprucht, das umfaßt, die Kondensation einer Verbindung der Formel

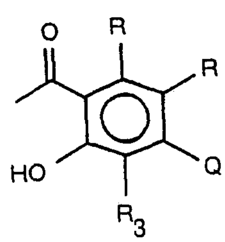

VII

worin Q 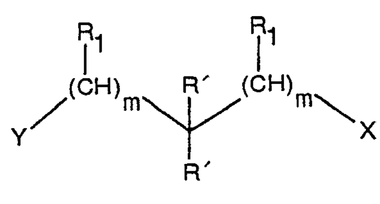 oder

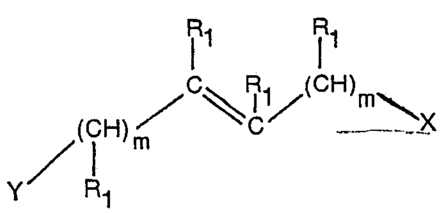 oder 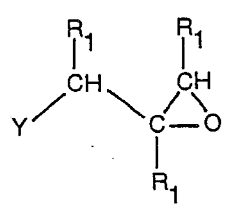 ist,

mit einer Verbindung der Formel

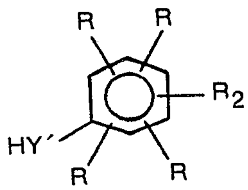

worin R, $R_1$, $R_2$, $R_3$, R', Y, Y' und m die in Anspruch 1 angegebene Bedeutung haben und X Halogen ist.

13. Ein Verfahren zur Herstellung einer Verbindung, wie in Anspruch 1 beansprucht, durch Kondensation einer Verbindung mit einer der folgenden Formeln

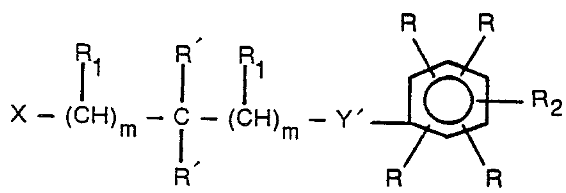

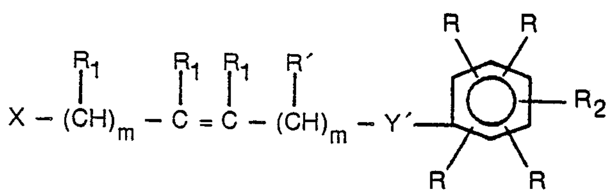

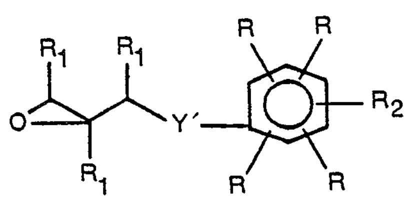

mit einer Verbindung der Formel

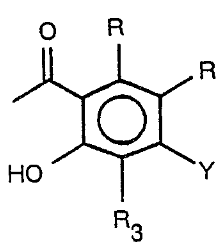

worin R, $R_1$, $R_2$, $R_3$, $R^1$, Y, Y' und m die in Anspruch 1 angegebene Bedeutung haben und X Halogen ist.

14. Eine Verbindung mit einer der folgenden Formeln

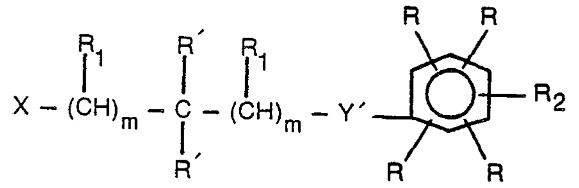

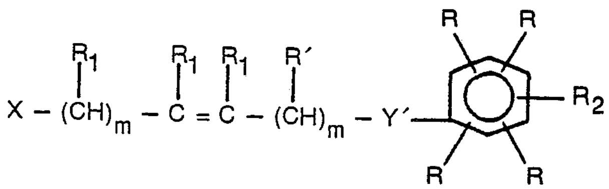

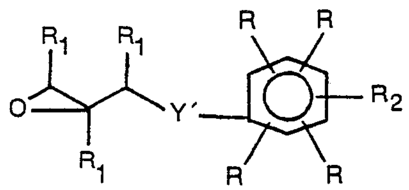

worin Y', R, $R_1$, $R_2$, R' und m die in Anspruch 1 angegebene Bedeutung haben und X Halogen ist.

**Patentansprüche für den Vertragsstaat: AT**

1. Ein Verfahren zur Herstellung einer Verbindung, die eine der folgenden Formeln aufweist:

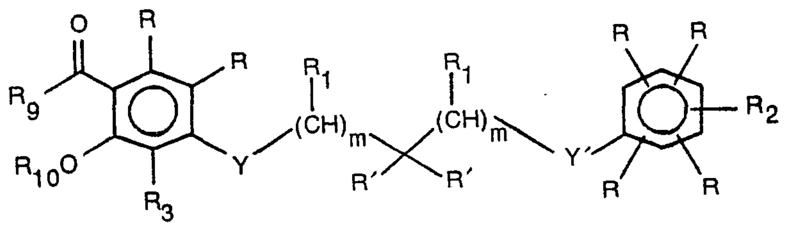
XI

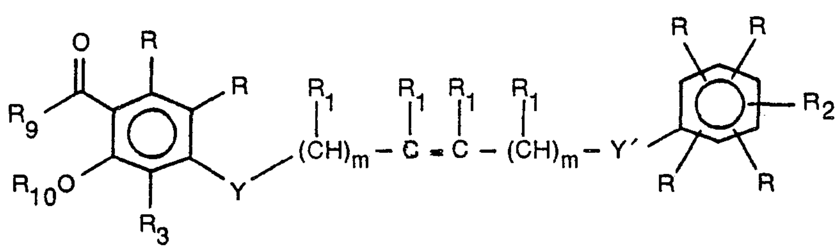
XII

worin

jeder Rest R unabhängig von anderen H, OH, Alkyl mit 1 bis 6 Kohlenstoffatomen, welche geradkettig oder verzweigt sein können; Alkenyl mit 2 bis 6 Kohlenstoffatomen, welche geradkettig oder verzweigt sein können; Trifluormethyl; Alkoxy mit 1 bis 6 Kohlenstoffatomen, welche geradkettig oder verzweigt sein können; SH; Thioalkyl mit 1 bis 6 Kohlenstoffatomen, welche geradkettig oder verzweigt sein können; Phenyl; Phenyl, das durch Alkyl mit 1 bis 3 Kohlenstoffatomen oder durch Halogen substituiert ist; Benzyl, Phenalkyl mit 2 bis 4 Alkylkohlenstoffatomen; Halogen, Amino; $N(R_4)_2$, worin $R_4$ H oder Alkyl mit 1 bis 6

Kohlenstoffatomen ist, welche geradkettig oder verzweigt sein können; $COOR_4$; $CH_2OR_4$; Formyl; CN; Trifluormethylthio; der Nitro ist;

jeder Rest R′ unabhängig vom anderen $R_4$; $OR_4$; $COOR_4$; $N(R_4)_2$; $SR_4$; $CH_2OR_4$; CHO ist; oder R′ und R′ gemeinsam O; $CH_2$ oder

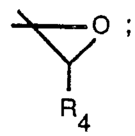

sind;

Y Sauerstoff, Schwefel, Sulfoxid, Sulfon;

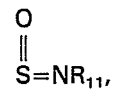

worin $R_{11}$ Alkyl mit 1 bis 4 Kohlenstoffatomen, die geradkettig oder verzweigt sein können, ist; $NR_{12}$, worin $R_{12}$ H, Alkyl mit 1 bis 4 Kohlenstoffatomen, die geradkettig oder verzweigt sein können, ist;

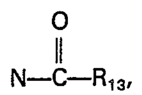

worin $R_{13}$ Alkyl mit 1 bis 4 Kohlenstoffatomen, die geradkettig oder verzweigt sein können, Alkoxy mit 1 bis 4 Kohlenstoffatomen, die geradkettig oder verzweigt sein können, ist; oder N—CN ist;

Y′ für Y, $—CH_2—$ oder

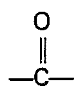

steht;

jeder Rest $R_1$ unabhängig von anderen Wasserstoff oder Alkyl mit 1 bis 3 Kohlenstoffatomen ist;

jedes Symbol m unabhängig vom anderen eine ganze Zahl von 0 bis 6 ist;

$R_2$

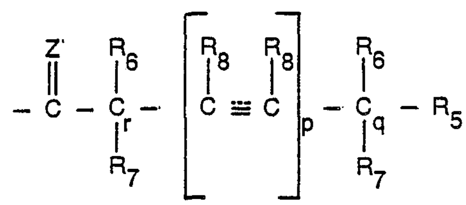

ist, worin

Z O, S, $CH_2$, H und OH, Alkenyl mit 1 bis 4 Kohlenstoffatomen; oder $N—R_{14}$ ist, wobei

$R_{14}$ OH, Alkyl oder Alkoxy mit 1 bis 6 Kohlenstoffatomen, Perhalogenalkyl mit 1 bis 6 Kohlenstoffatomen, Phenyl oder Phenyl, das durch Alkyl- oder Alkoxygruppen mit 1 bis 3 Kohlenstoffatomen, Halogen, Hydroxy, Halogenalkyl, COOH, CN, Formyl oder Acyl mit 1 bis 6 Kohlenstoffatomen substituiert ist, bedeutet;

jeder Rest $R_6$ unabhängig vom anderen H oder Alkyl mit 1 bis 4 Kohlenstoffatomen ist;

jeder Rest $R_7$ unabhängig vom anderen H, OH oder Alkyl mit 1 bis 4 Kohlenstoffatomen ist;

jeder Rest $R_8$ unabhängig vom anderen H oder Alkyl mit 1 bis 4 Kohlenstoffatomen ist, und für den Fall fehlt, daß eine Dreifachbindung vorliegt;

$R_5$ $COOR_4$; $CH_2OH$; CHO; Tetrazol; $NHSO_2R_{14}$; $CONHSO_2R_{14}$; Hydroxymethylketon; CN; $CON(R_7)_2$; ein monocyclischer oder bicyclischer, heterocyclischer Ring, der eine saure Hydroxylgruppe enthält; oder $COOR_{15}$ ist, wobei

$R_{15}$:

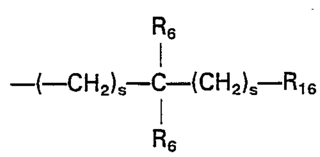

ist,

wobei jedes Symbol s unabhängig vom anderen 0 bis 3 ist;

$R_{16}$

(A) ein monocyclischer oder bicyclischer, heterocyclischer Rest mit 3 bis 12 Kernkohlenstoffatomen und 1 oder 2 Kernheteroatomen, ausgewählt aus N und S, wobei wenigstens eines davon N ist, und wobei jeder Ring in dem heterocyclischen Rest aus 5 oder 6 Atomen gebildet ist, oder

(B) der Rest W—$R_{17}$ ist, wobei W für O, S oder NH steht, und $R_{17}$ bis zu 21 Kohlenstoffatome enthält, und (1) ein Kohlenwasserstoffrest oder (2) ein Acylrest einer organischen, acyclischen oder monocyclischen Carbonsäure, die nicht mehr als 1 Heteroatom in dem Ring enthält, ist;

r und q jeweils unabhängig voneinander 0 bis 20 mit der Maßgabe sind, daß die Summe von r und q 20 nicht überschreitet; und

p 0 oder 1 ist;

$R_3$ Alkyl mit 1 bis 6 Kohlenstoffatomen, welche geradkettig oder verzweigt sein können; oder Alkenyl mit 3 bis 6 Kohlenstoffatomen, welche geradkettig oder verzweigt sein können, ist;

$R_9$ Alkyl mit 1 bis 6 Kohlenstoffatomen, welche geradkettig oder verzweigt sein können; Alkoxy mit 1 bis 6 Kohlenstoffatomen, welche geradkettig oder verzweigt sein können; oder $(CH_2)_rR_5$ ist; und

$R_{10}$ H; Alkyl mit 1 bis 6 Kohlenstoffatomen, welche geradkettig oder verzweigt sein können;

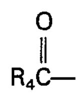

oder $R_4OCH_2$— ist;

oder einer Verbindung, die ein Lacton davon ist, für den Fall, daß in $R_2$ Z H und OH darstellt, oder $R_7$ OH ist und $R_5$ für Carboxy steht, oder einer Verbindung, die ein pharmazeutisch annehmbares Salz oder Säureadditionssalz davon ist, welches Verfahren das Kondensieren einer Verbindung der Formel:

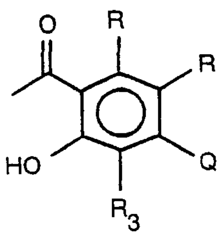

VII

worin Q 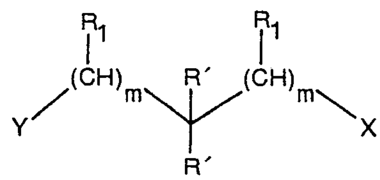 oder

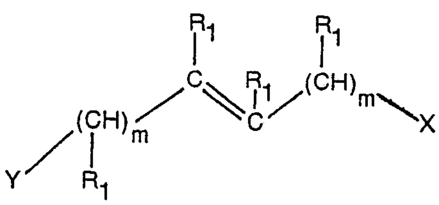 oder 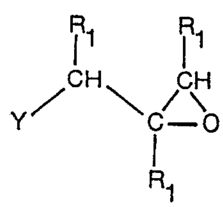 ist,

mit einer Verbindung der Formel

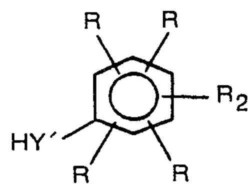

worin R, $R_1$, $R_2$, $R_3$, R′, Y, Y′ und m wie oben definiert sind und X ein Halogen ist, umfaßt.

2. Ein Verfahren zur Herstellung einer Verbindung, die eine der Formeln XI oder XII in Anspruch 1 hat, welches das Kondensieren einer Verbindung, die eine der folgenden Formeln aufweist

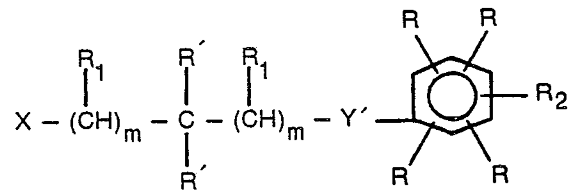

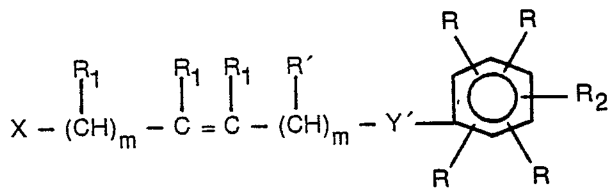

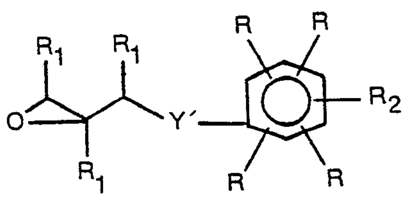

mit einer Verbindung der Formel

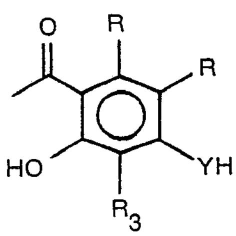

worin R, $R_1$, $R_2$, $R_3$, R', Y, Y', m und X wie in Anspruch 1 definiert sind, umfaßt.

3. Ein Verfahren wie in Anspruch 1 oder 2 beansprucht, angewendet bei der Herstellung von 4-((3-(4-Acetyl-3-hydroxy-2-propylphenoxy)propyl)thio)-gamma-oxobenzolbutansäure.

4. Ein Verfahren wie in Anspruch 1 oder 2 beansprucht, angewendet bei der Herstellung von 4-[3-(4-Acetyl-3-hydroxy-2-propylphenoxy)propylsulfonyl]-gamma-oxobenzolbutansäure.

5. Ein Verfahren wie in Anspruch 1 oder 2 beansprucht, angewendet bei der Herstellung von (beta S*, gamma R*)-4-(3-(4-Acetyl-3-hydroxy-2-propylphenoxy)propylthio)-gamma-hydroxy-beta-methylbenzolbutansäure-Natriumsalz.

6. Ein Verfahren wie in Anspruch 1 oder 2 beansprucht, angewendet bei der Herstellung von (beta S*, gamma R*)-4-(3-(4-Acetyl-3-hydroxy-2-propylphenoxy)propyloxy)-gamma-hydroxy-beta-methylbenzolbutansäure-Natriumsalz.

7. Ein Verfahren wie in Anspruch 1 oder 2 beansprucht, angewendet bei der Herstellung von 4-(3-(4-Acetyl-3-hydroxy-2-propylphenoxy)propylthio)-epsilon-oxobenzolhexansäure.

8. Ein Verfahren wie in Anspruch 1 oder 2 beansprucht, angewendet bei der Herstellung einer Verbindung, in welcher Y Sauerstoff ist und Y' Sauerstoff, Schwefel, Sulfoxid, Sulfon, Amino oder Cyanamido ist.

9. Ein Verfahren wie in Anspruch 1, 2 oder 7 beansprucht, angewendet bei der Herstellung einer Verbindung, in welcher jedes Symbol m 1 ist.

**Revendications pour les Etats contractants: BE CH DE FR GB IT LI LU NL SE**

1. Composé répondant à l'une quelconque des formules

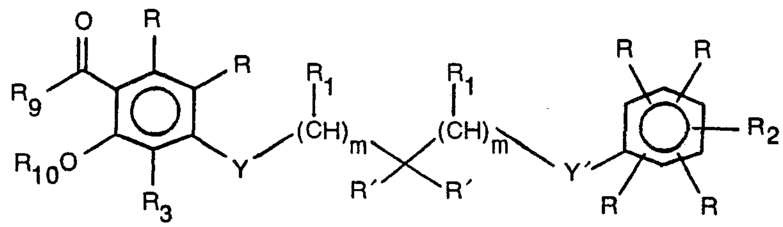

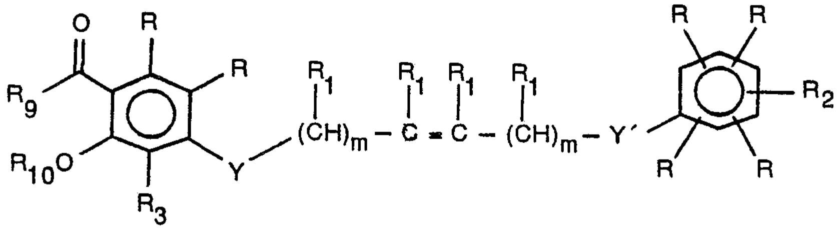

dans lesquelles

chaque R est indépendamment H, OH, un groupe alkyle de 1 à 6 atomes de carbone qui peut être à chaîne droite ou ramifiée, alcényle de 2 à 6 atomes de carbone qui peut être à chaîne droite ou ramifiée, trifluorométhyle, alcoxy de 1 à 6 atomes de carbone qui peut être à chaîne droite ou ramifiée, SH, thioalkyle de 1 à 6 atomes de carbone qui peut être à chaîne droite ou ramifiée, phényle, phényle substitué par un groupe alkyle de 1 à 3 atomes de carbone ou par un halogène, benzyle, phénylalkyle ayant de 2 à 4 atomes de carbone dans le reste alkyle, halogène, amino, $N(R_4)_2$ dans lequel $R_4$ est H ou un groupe alkyle de 1 à 6 atomes de carbone qui peut être à chaîne droite ou ramifiée, $COOR_4$, $CH_2OR_4$, formyle, CN, trifluorométhylthio ou nitro;

chaque R' est indépendamment $R_4$, $OR_4$, $COOR_4$, $N(R_4)_2$, $SR_4$, $CH_2OR_4$, CHO ou R' et R' sont ensemble O, $CH_2$ ou

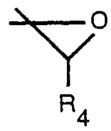 ;

Y est l'oxygène, le soufre, un groupe sulfoxyde, sulfone,

$$\overset{\displaystyle O}{\overset{\|}{S}}{=}NR_{11}$$

dans lequel $R_{11}$ est un groupe alkyle de 1—4 atomes de carbone, qui peut être à chaîne droite ou ramifiée, $NR_{12}$ dans lequel $R_{12}$ est H, un groupe alkyle de 1—4 atomes de carbone, qui peut être à chaîne droite ou ramifiée, ou

$$N{-}\overset{\displaystyle O}{\overset{\|}{C}}{-}R_{13}$$

dans lequel $R_{13}$ est un groupe alkyle de 1—4 atomes de carbone, qui peut être à chaîne droite ou ramifiée, alcoxy de 1—4 atomes de carbone, qui peut être à chaîne droite ou ramifiée, ou N—CN;

Y' est Y, $—CH_2—$ ou

$$—\overset{\displaystyle O}{\overset{\|}{C}}—;$$

chaque $R_1$ est indépendamment l'hydrogène ou un groupe alkyle de 1—3 atomes de carbone;
chaque m est indépendamment un nombre entier de 0—6; et
$R_2$ est

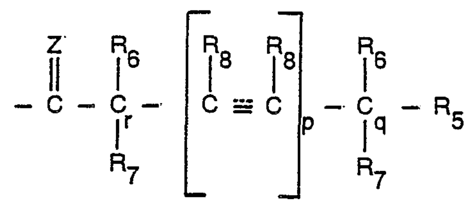

dans lequel Z est O, S, $CH_2$, H et OH, un groupe alcényle de 1—4 atomes de carbone; ou N—$R_{14}$ dans lequel $R_{14}$ est OH, un groupe alkyle ou alcoxy de 1 à 6 atomes de carbone, perhalogénoalkyle de 1 à 6 atomes de carbone, phényle ou phényle substitué par des groupes alkyle ou alcoxy de 1 à 3 atomes de carbone, halogène, hydroxy, halogénoalkyle, COOH, CN, formyle ou acyle de 1 à 6 atomes de carbone;

chaque $R_6$ est indépendamment H ou un groupe alkyle de 1—4 atomes de carbone;

chaque $R_7$ est indépendamment H, OH, ou un groupe alkyle de 1—4 atomes de carbone;

chaque $R_8$ est indépendamment H ou un groupe alkyle de 1—4 atomes de carbone, et est absent lorsqu'une triple liaison est présente;

$R_5$ est $COOR_4$; $CH_2OH$; CHO; tétrazole; $NHSO_2R_{14}$; $CONHSO_2R_{14}$; hydroxyméthylcétone; CN; $CON(R_7)_2$; un noyau hétérocyclique monocyclique ou bicyclique contenant un groupe hydroxyle acide; ou $COOR_{15}$ dans lequel $R_{15}$ est

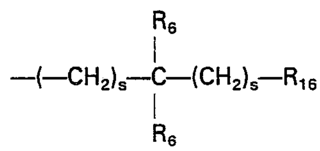

dans lequel chaque s est indépendamment 0—3; $R_{16}$ est

A) un radical hétérocyclique monocyclique ou bicyclique contenant de 3 à 12 atomes de carbone dans le noyau et 1 ou 2 hétéroatomes dans le noyau choisis parmi N et S, l'un au moins étant N, et chaque cycle du radical hétérocyclique étant formée de 5 ou 6 atomes, ou

B) le radical W—$R_{17}$ dans equel W est O, S ou NH et $R_{17}$ contient jusqu'à 21 atomes de carbone et est (1) un radical hydrocarbure ou (2) un radical acyle d'un acide carboxylique organique acyclique ou monocyclique ne contenant pas plus de 1 hétéroatome dans le cycle;

r et q sont chacun indépendamment 0—20 à condition que le total de r et q ne dépasse pas 20; et

p est 0 ou 1;

$R_3$ est un groupe alkyle de 1 à 6 atomes de carbone qui peut être à chaîne droite ou ramifiée; ou alcényle de 3 à 6 atomes de carbone qui peut être à chaîne droite ou ramifiée;

$R_9$ est un groupe alkyle de 1 à 6 atomes de carbone qui peut être à chaîne droite ou ramifiée; alcoxy de 1 à 6 atomes de carbone qui peut être à chaîne droite ou ramifiée; ou $(CH_2)_rR_5$;

$R_{10}$ est H; un groupe alkyle de 1 à 6 atomes de carbone qui peut être à chaîne droite ou ramifiée;

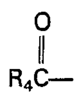

ou $R_4OCH_2$—;

ou composé qui est une lactone de ce composé lorsque, dans $R_2$, Z est H et OH ou $R_7$ est OH et $R_5$ est un groupe carboxy, ou composé qui est un sel pharmaceutiquement acceptable ou un sel d'addition avec un acide de ce composé.

2. Composé selon la revendication 1, dans lequel Y est l'oxygène et Y' est l'oxygène, le soufre, un groupe sulfoxyde, sulfone, amino ou cyanamido.

3. Composé selon la revendication 1 ou 2, dans lequel chaque m est 1.

4. Acide 4-((3-(4-acétyl-3-hydroxy-2-propylphénoxy)propyl)thio)-gamma-oxobenzène-butanoïque.

5. Acide 4-[3-(4-acétyl-3-hydroxy-2-propylphénoxy)propylsulfonyl]-gamma-oxobenzène-butanoïque.

6. Sel de sodium de l'acide (bêta S*, gamma R*)-4-(3-(4-acétyl-3-hydroxy-2-propyl-phénoxy)propylthio)-gamma-hydroxy-bêta-méthyl-benzène-butanoïque.

7. Sel de sodium de l'acide (bêta S*, gamma R*)-4-(3-(4-acétyl-3-hydroxy-2-propyl-phénoxy)propyloxy)-gamma-hydroxy-bêta-méthyl-benzène-butanoïque.

8. Composé selon la revendication 1, ayant pour nom:

Acide 4-(3-(4-acétyl-3-hydroxy-2-propylephénoxy)propoxy)-3-fluoro-gamma-oxobenzène-butanoïque,

Acide 4-(3-(4-acétyl-3-hydroxy-2-propylphénoxy)propoxy)-gamma-oxobenzène-butanoïque,

Acide 4-((3-(4-acétyl-3-hydroxy-2-propylphénoxy)-2-hydroxypropyl)thio)-2-fluoro-gamma-oxo-benzène-butanoïque,

Acide 4-((3-(4-acétyl-3-hydroxy-2-propylphénoxy)-2-hydroxypropyl)thio)-2-hydroxy-gamma-oxo-benzène-butanoïque,

Acide 4-((3-(4-acétyl-3-hydroxy-2-propylphénoxy)propyl)thio)-2-hydroxy-gamma-oxobenzène-butanoïque,

Acide 4-((3-(4-acétyl-3-hydroxy-2-propylphénoxy)-2-hydroxypropyl)thio)-2-méthoxy-gamma-oxobenzène-butanoïque,

Acide 4-((3-(4-acétyl-3-hydroxy-2-propylphénoxy)propyl)thio)-3-fluoro-gamma-oxobenzène-butanoïque,

Acide 4-((3-(4-acétyl-3-hydroxy-2-propylphénoxy)propyl)thio)-2-fluoro-gamma-oxobenzène-butanoïque,

Acide 4-((3-(4-acétyl-3-hydroxy-2-propylphénoxy)propoxy)-3-fluoro-gamma-oxobenzène-butanoïque,

Acide 4-((3-(4-acétyl-3-hydroxy-2-propylphénoxy)propoxy)-2-fluoro-gamma-oxobenzène-butanoïque,

Ester méthylique de l'acide 4-(3-(4-acétyl-3-hydroxy-2-propylphénoxy)propoxy)-gamma-oxobenzène-butanoïque,

Acide 4-(3-(4-acétyl-3-hydroxy-2-propylphénoxy)propoxy)-3-méthyl-gamma-oxobenzène-butanoïque,

Acide 4-((3-(4-acétyl-3-hydroxy-2-propylphénoxy)propoxy)-2-chloro-gamma-oxobenzène-butanoïque,

Acide 4-(4-(4-acétyl-3-hydroxy-2-propylphénoxy)-2-buténoxy)-2-fluoro-gamma-oxobenzène-butanoïque,

S-oxyde de l'acide 4-(3-(4-acétyl-3-hydroxy-2-propylphénoxy)-2-hydroxypropylthio)-3-fluoro-gamma-oxobenzène-butanoïque,

S-oxyde de l'acide 4-(3-(4-acétyl-3-hydroxy-2-propylphénoxy)-propylthio)-3-fluoro-gamma-oxobenzène-butanoïque,

S-oxyde de l'acide 4-(3-(4-acétyl-3-hydroxy-2-propylphénoxy)-propylthio)-2-fluoro-gamma-oxobenzène-butanoïque,

S-oxyde de l'acide 4-(3-(4-acétyl-3-hydroxy-2-propylphénoxy)-2-hydroxypropylthio)-2-fluoro-gamma-oxobenzène-butanoïque.

Acide 4-(3-(4-acétyl-3-hydroxy-2-propylphénoxy)propylsulfonyl)-2-fluoro-gamma-oxobenzène-butanoïque,

Acide 4-(3-(4-acétyl-3-hydroxy-2-propylphénoxy)-2-hydroxypropylsulfonyl)-2-fluoro--gamma-oxobenzène-butanoïque,

Acide 4-(3-(4-acétyl-3-hydroxy-2-propylphénoxy)propylsulfonyl)-3-fluoro-gamma-oxobenzène-butanoïque,

Acide 4-(3-(4-acétyl-3-hydroxy-2-propylphénoxy)-2-hydroxypropylsulfonyl)-3-fluoro-gamma-oxobenzène-butanoïque,

Acide 4-(3-(4-acétyl-3-hydroxy-2-propylphénoxy)-2-hydroxypropylsulfonyl)-gamma-oxobenzène-butanoïque,

Acide 4-(3-(4-acétyl-3-hydroxy-2-propylphénoxy)propoxy)-3-chloro-gamma-oxobenzène-butanoïque,

Sel de sodium monohydraté de l'acide 4-(3-(4-acétyl-3-hydroxy-2-propylphénoxy)-2-hydroxypropoxy-3-fluoro-gamma-oxobenzène-butanoïque,

Acide 4-(3-(4-acétyl-3-hydroxy-2-propylphénoxy)propoxy)-alpha,alpha-diméthyl-gamma-oxobenzène-butanoïque,

Acide 4-(3-(4-acétyl-3-hydroxy-2-propylphénoxy)propoxy)-bêta-méthyl-gamma-oxobenzène-butanoïque,

Acide 4-(3-(4-acétyl-3-hydroxy-2-propylphénoxy)-2-méthylidénylpropoxy)-gamma-oxobenzène-butanoïque,

Acide 4-(3-(4-acétyl-6-chloro-3-hydroxy-2-propylphénoxy)-2-méthylidénepropoxy)-gamma-oxobenzène-butanoïque,

Acide 4-(3-(4-acétyl-3-hydroxy-2-propylphénoxy)-2-méthylidènepropylthio)-gamma-oxobenzène-butanoïque,

Acide 4-(3-(4-acétyl-3-hydroxy-2-propylphénoxy)-2-méthylidènepropoxy)-3-fluoro-gamma-oxobenzène-butanoïque,

4-(3-(4-acétyl-3-hydroxy-2-propylphénoxy)propyl)thio)-gamma-oxobenzène-butyronitrile,

5-(3-(4-(3-(4-acétyl-3-hydroxy-2-propylphénoxy)propyl)thio)phényl)-3-oxopropyl)-1H-tétrazole,

4-(3-(4-acétyl-3-hydroxy-2-propylphénoxy)propylsulfonyl)-gamma-oxobenzène-butyronitrile,

5-(3-(4-(3-(4-acétyl-3-hydroxy-2-propylphénoxy)propylsulfonyl)phényl)-3-oxopropyl)-1H-tétrazole,

Acide 4-(3-(4-acétyl-3-hydroxy-2-propylphénoxy)propylthio)-epsilon-oxobenzène-hexanoïque,

Acide 4-(3-(4-acétyl-3-hydroxy-2-propylphénoxy)propylsulfonyl)-epsilon-oxobenzène-hexanoïque,

4-(3-(4-acétyl-3-hydroxy-2-propylphénoxy)propylthio)-delta-oxobenzène-butanol,
4-(3-(4-acétyl-3-hydroxy-2-propylphénoxy)propylsulfonyl)-delta-oxobenzène-butanol,
4-(3-(4-acétyl-3-hydroxy-2-propylphénoxy)propylthio)-bêta,epsilon-dioxo-benzène-pentanol,
Acide 4-(3-(4-acétyl-3-hydroxy-2-propylphénoxy)propylthio)-gamma-oxobenzène-butanoïque,
Acide 4-(3-(4-acétyl-3-hydroxy-2-propylphénoxy)propylsulfonyl)-gamma-oxobenzène-butanoïque,
4-(3-(4-acétyl-3-hydroxy-2-propylphénoxy)propylsulfonyl)-gamma-oxobenzène-butyramide,
N-méthylsulfonyl-4-(3-(4-acétyl-3-hydroxy-2-propylphénoxy)propylthio)-gamma-oxo-benzène-butyramide,
5-(4-(3-(4-acétyl-3-hydroxy-2-propylphénoxy)propylsulfonyl)phényl)-2(3H)-furannone,
Acide 4-(3-(4-acétyl-3-hydroxy-2-propylphénoxy)propyloxy)-gamma-oxo-bêta,bêta-diméthylbenzène-butanoïque,
Sel de sodium de l'acide 4-(3-(4-acétyl-3-hydroxy-2-propylphénoxy)propyloxy)-gamma-hydroxy-bêta,bêta-diméthylbenzène-butanoïque,
Acide 4-(3-(4-acétyl-3-hydroxy-2-propylphénoxy)propylthio)-gamma-oxo-bêta,bêta-diméthylbenzène-butanoïque,
Sel de sodium de l'acide 4-(3-(4-acétyl-3-hydroxy-2-propylphénoxy)propylthio)-gamma-hydroxy-bêta,bêta-diméthylbenzène-butanoïque,
Isomère (+) du sel de sodium de l'acide (bêta S*, gamma R*)-4-(3-(4-acétyl-3-hydroxy-2-propylphénoxy)propylthio)-gamma-hydroxy-bêta-méthylbenzène-butanoïque,
Isomère (−) du sel de sodium de l'acide (bêta S*, gamma R*)-4-(3-(4-acétyl-3-hydroxy-2-propylphénoxy)propylthio)-gamma-hydroxy-bêta-méthylbenzène-butanoïque,
Gamma-lactone de l'acide (bêta S*, gamma R*)-4-(3-(4-acétyl-3-hydroxy-2-propylphénoxy)propylthio)-gamma-hydroxy-bêta-méthylbenzène-butanoïque,
Gamma-lactone de l'acide (bêta S*, gamma R*)-4-(3-(4--acétyl-3-hydroxy-2-propylphénoxy)propylsulfonyl)-gamma-hydroxy-bêta-méthylbenzène-butanoïque,
Sel de sodium de l'acide (bêta S*, gamma R*)-4-(3-(4-acétyl-3-hydroxy-2-propylphénoxy)propylsulfonyl)-gamma-hyroxy-bêta-méthylbenzène-butanoïque,
Sel de sodium de l'acide (bêta R*, gamma R*)-4-(3-(4-acétyl-3-hydroxy-2-propylphénoxy)propylsulfonyl)-gamma-hydroxy-bêta-méthylbenzène-butanoïque,
Sel de sodium de l'acide (bêta R*, gamma R*)-4-(3-(4-acétyl-3-hydroxy-2-propylphénoxy)propylthio)-gamma-hydroxy-bêta-méthylbenzène-butanoïque,
Sel de sodium de l'acide (bêta R*, gamma R*)-4-(3-(4-acétyl-3-hydroxy-2-propylphénoxy)propylsulfonyl)-gamma-hydroxy-bêta-méthylbenzène-butanoïque,
Sel de sodium de l'acide (bêta R*, gamma R*)-4-(3-(4-acétyl-3-hydroxy-2-propylphénoxy)propylthio)-gamma-hydroxy-bêta-méthylbenzène-butanoïque,
Gamma-lactone de l'acide (bêta R*, gamma R*)-4-(3-(4-acétyl-3-hydroxy-2-propylphénoxy)propylsulfonyl)-gamma-hydroxy-bêta-méthylbenzène-butanoïque,
Gamma-lactone de l'acide (bêta R*, gamma R*)-4-(3-(4-acétyl-3-hydroxy-2-propylphénoxy)propylthio)-gamma-hydroxy-bêta-méthylbenzène-butanoïque,
Sel de sodium de l'acide S-oxyde-(bêta S*, gamma R*)-4-(3-(4-acétyl-3-hydroxy-2-propylphénoxy)propylthio)-gamma-hydroxy-bêta-méthylbenzène-butanoïque,
Sel de sodium de l'acide (alpha S*, bêta R*, gamma R*)-4-(3-(4-acétyl-3-hydroxy-2-propylphénoxy)propylthio)-gamma-hydroxy-alpha, bêta-diméthylbenzène-butanoïque,
Gamma-lactone de l'acide 4-(3-(4-acétyl-3-hydroxy-2-propylphénoxy)propylthio)-gamma-hydroxybenzène-butanoïque,
Gamma-lactone de l'acide 4-(3-(4-acétyl-3-hydroxy-2-propylphénoxy)propylsulfonyl)-gamma-hydroxybenzène-butanoïque,
Acide 4-((3-(4-acétyl-6-fluoro-3-hydroxy-2-propylphénoxy)propyl)thio)-gamma-oxobenzène-butanoïque,
Acide 4-((3-(4-acétyl-6-chloro-3-hydroxy-2-propylphénoxy)propyl)thio)-gamma-oxobenzène-butanoïque,
Acide 4-(3-(4-acétyl-6-chloro-3-hydroxy-2-propylphénoxy)propylsulfonyl)-gamma-oxobenzène-butanoïque,
Acide 4-((3-(4-acétyl-6-bromo-3-hydroxy-2-propylphénoxy)propyl)thio)-gamma-oxobenzène-butanoïque,
Acide 4-(3-(4-acétyl-6-bromo-3-hydroxy-2-propylphénoxy)propylsulfonyl)-gamma-oxobenzène-butanoïque,
Acide 4-((3-(4-acétyl-3-hydroxy-6-méthyl-2-propylphénoxy)propyl)thio)-gamma-oxobenzène-butanoïque,
Acide (bêta S*, gamma R*)-4-(3-(4-acétyl-6-fluoro-3-hydroxy-2-propylphénoxy)propylthio)-gamma-hydroxy-bêta-méthlbenzène-butanoïque,
Acide (bêta S*, gamma R*)-4-(3-(4-acétyl-5-chloro-3-hydroxy-2-propylphénoxy)propylthio)-gamma-hydroxy-bêta-méthylbenzène-butanoïque,
Acide (bêta S*, gamma R*)4-(3-(4-acétyl-3-hydroxy-6-méthyl-2-propylphénoxy)propylthio)-gamma-hydroxy-bêta-méthylbenzène-butanoïque,

Gamma-lactone de l'acide (alpha S*, bêta R*, gamma R*)4-(3-(4-acétyl-3-hydroxy-2-propylphénoxy)propylthio)-gamma-hydroxy-alpha, bêta-diméthylbenzène-butanoïque,

Acide (bêta S*, gamma R*)4-(3-(4-acétyl-6-éthyl-3-hydroxy-2-propylphénoxy)propylthio)-gamma-hydroxy-bêta-méthylbenzène-butanoïque,

Acide 4-(3-(4-acétyl-6-fluoro-3-hydroxy-2-propylphénoxy)propyloxy)-gamma-hydroxy-bêta,bêta-diméthylbenzène-butanoïque,

Acide 4-(3-(4-acétyl-6-chloro-3-hydroxy-2-propylphénoxy)propyloxy)-gamma-hydroxy-bêta,bêta-diméthylbenzène-butanoïque,

Acide 4-(3-(4-acétyl-3-hydroxy-6-méthyl-2-propylphénoxy)propyloxy)-gamma-hydroxy-bêta,bêta-diméthylbenzène-butanoïque,

Acide 4-(3-(4-acétyl-6-fluoro-3-hydroxy-2-propylphénoxy)propylthio)-gamma-hydroxy-bêta,bêta-diméthylbenzène-butanoïque,

Acide 4-(3-(4-acétyl-5-chloro-3-hydroxy-2-propylphénoxy)propylthio)-gamma-hydroxy-bêta,bêta-diméthylbenzène-butanoïque,

Acide 4-(3-(4-acétyl-3-hydroxy-6-méthyl-2-propylphénoxy)propylthio)-gamma-hydroxy-bêta,bêta-diméthylbenzène-butanoïque,

Acide 4-(3-(4-acétyl-3-hydroxy-2-propylphénoxy)propylthio)-gamma-oxobenzène-butanoïque,

Acide 4-(3-(4-acétyl-3-hydroxy-2-propylphénoxy)propylsulfonyl)-3-propyl-gamma-oxo-benzène-butanoïque,

Sel de sodium de l'acide (bêta R*, gamma S*)-4-(3-(4-acétyl-3-hydroxy-2-propylphénoxy)propylthio)-3-fluoro-gamma-hydroxy-bêta-méthylbenzène-butanoïque,

Sel de sodium de l'acide (bêta R*, gamma S*)-4-(3-(4-acétyl-3-hydroxy-2-propylphénoxy)propylthio)-3-bromo-gamma-hydroxy-bêta-méthylbenzène-butanoïque,

Sel de sodium de l'acide (bêta R*, gamma S*)-4-(3-(4-acétyl-3-hydroxy-2-propylphénoxy)propylthio)-3-chloro-gamma-hydroxy-bêta-méthylbenzène-butanoïque,

Acide 4-(3-(4-acétyl-3-hydroxy-2-propylphénoxy)propylsulfonyl)-gamma-oxo-bêta,bêta-diméthylbenzène-butanoïque,

Sel de sodium de l'acide 4-(3-(4-acétyl-3-hydroxy-2-propylphénoxy)propylsulfonyl)-gamma-hydroxy-bêta,bêta-diméthyl benzènebutanoïque,

Acide 4-(3-(4-acétyl-3-hydroxy-2-propylphénoxy)propylthio)-3-propyl-gamma-oxo-bêta,bêta-diméthylbenzènebutanoïque,

Sel de sodium de l'acide 4-(3-(4-acétyl-3-hydroxy-2-propylphénoxy)propylthio)-3-propyl-gamma-hydroxy-bêta,bêta-diméthylbenzènebutanoïque,

Sel de sodium de l'acide 4-(3-(4-acétyl-3-hydroxy-2-propylphénoxy)propylthio)-3-fluoro-gamma-oxo-bêta,bêta-diméthylbenzènebutanoïque,

Sel de sodium de l'acide 4-(3-(4-acétyl-3-hydroxy-2-propylphénoxy)propylthio)-3-fluoro-gamma-hydroxy-bêta,bêta-diméthylbenzènebutanoïque,

Acide 4-(3-(4-acétyl-3-hydroxy-2-propylphénoxy)propylthio)-3-chloro-gamma-oxo-bêta,bêta-diméthylbenzènebutanoïque,

Sel de sodium de l'acide 4-(3-(4-acétyl-3-hydroxy-2-propylphénoxy)propylthio)-3-chloro-gamma-hydroxy-bêta,bêta-diméthylbenzènebutanoïque,

Sel de sodium de l'acide 4-(3-(4-acétyl-3-hydroxy-2-propylphénoxy)propylthio)-3-bromo-gamma-hydroxy-bêta,bêta-diméthylbenzènebutanoïque,

Sel de sodium de l'acide 4-(3-(4-acétyl-3-hydroxy-2-propylphénoxy)propylthio)-3-fluoro-gamma-hydroxy-bêta,bêta-diméthylbenzènebutanoïque,

Sel de sodium de l'acide 4-(3-(4-acétyl-3-hydroxy-2-propylphénoxy)propyloxy)-3-bromo-gamma-hydroxy-bêta,bêta-diméthylbenzènebutanoïque,

Sel de sodium de l'acide 4-(3-(4-acétyl-3-hydroxy-2-propylphénoxy)propyloxy)-3-chloro-gamma-hydroxy-bêt,bêta-diméthylbenzènebutanoïque,

Acide 4-(3-(4-acétyl-3-hydroxy-2-propylphénoxy)propyloxy)-3-fluoro-gamma-oxo-bêta,bêta-diméthylbenzène-butanoïque,

Acide 4-(3-(4-acétyl-3-hydroxy-2-propylphénoxy)propyloxy)-3-chloro-gamma-oxo-bêta,bêta-diméthylbenzène-butanoïque,

Acide 4-(3-(4-acétyl-3-hydroxy-2-propylphénoxy)propyloxy)-3-bromo-gamma-oxo-bêta,bêta-diméthylbenzène-butanoïque,

Acide 4-(3-(4-acétyl-3-hydroxy-2-propylphénoxy)propyloxy)-3-propyl-gamma-oxo-bêta,bêta-diméthylbenzène-butanoïque,

Acide 4-(3-(4-acétyl-3-hydroxy-2-propylphénoxy)propylthio)-gamma-hydroxy-bêta-méthylbenzène-butanoïque,

Sel de sodium de l'acide 4-(3-(4-acétyl-3-hydroxy-2-propylphénoxy)propylthio)-gamma-hyroxy-bêta,bêta-diméthylbenzène-butanoïque,

Sel de sodium de l'acide 4-(3-(4-acétyl-3-hydroxy-2-propylphénoxy)propyloxy)-gamma-hydroxy-bêta,bêta-diméthylbenzène-butanoïque,

Acide 3-(3-(4-acétyl-3-hydroxy-2-propylphénoxy)propylthio)-gamma-oxobenzène-butanoïque,

Acide 3-(3-(4-acétyl-3-hydroxy-2-propylphénoxy)propylthio)-gamma-oxo-bêta,bêta-diméthylbenzène-butanoïque,

Acide 3-(3-(4-acétyl-3-hydroxy-2-propylphénoxy)propyloxy)-gamma-oxo-bêta,bêta-diméthylbenzène-butanoïque,

Sel de sodium de l'acide (bêta R*, gamma S*)-4-(3-(4-acétyl-3-hydroxy-2-propylphénoxy)propylthio)-gamma-hydroxy-bêta-méthylbenzène-butanoïque,

Ester méthylique de l'acide 4-(3-(4-acétyl-3-hydroxy-2-propylphénoxy)propylsulfonyl)-3-propyl-gamma-oxobenzène-butanoïque,

Ester méthylique de l'acide 4-(3-(4-acétyl-3-hydroxy-2-propylphénoxy)propylthio)-3-propyl-gamma-oxobenzènebutanoïque,

Acide (bêta R*, gamma S*)-4-(3-(4-acétyl-3-hydroxy-2-propylphénoxy)propylthio)-3-propyl-gamma-hydroxy-bêta-méthylbenzène-butanoïque,

Acide 4-(3-(4-acétyl-3-hydroxy-2-propylphénoxy)propylsulfonyl)-gamma-oxo-bêta,bêta-diméthylbenzène-butanoïque,

Acide 4-(3-(4-acétyl-3-hydroxy-2-propylphénoxy)propylsulfonyl)-gamma-hydroxy-bêta,bêta-diméthylbenzène-butanoïque,

Ester méthylique de l'acide 4-(3-(4-acétyl-3-hydroxy-2-propylphénoxy)propylthio)-3-propyl-gamma-oxo-bêta,bêta-diméthylbenzène-butanoïque,

Acide 4-(3-(4-acétyl-3-hydroxy-2-propylphénoxy)propylthio)-3-fluoro-gamma-oxo-bêta,bêta-diméthylbenzène-butanoïque,

Acide 4-(3-(4-acétyl-3-hydroxy-2-propylphénoxy)propylthio)-3-fluoro-gamma-hydroxy-bêta,bêta-diméthylbenzène-butanoïque,

Gamma-lactone de l'acide (bêta R*, gamma S*)-4-(3-(4-acétyl-3-hydroxy-2-propylphénoxy)propylthio)-3-fluoro-gamma-hydroxy-bêta-méthylbenzène-butanoïque,

Gamma-lactone de l'acide (bêta S*, gamma R*)-4-(3-(4-acétyl-3-hydroxy-2-propylphénoxy)propylthio)-3-chloro-gamma-hydroxy-bêta-méthylbenzène-butanoïque,

Gamma-lactone de l'acide (bêta S*, gamma R*)-4-(3-(4-acétyl-3-hydroxy-2-propylphénoxy)propylthio)-3-bromo-gamma-hydroxy-bêta-méthylbenzène-butanoïque,

Gamma-lactone de l'acide (bêta S*, gamma R*)-4-(3-(4-acétyl-3-hydroxy-2-propylphénoxy)propylsulfonyl)-3-fluoro-gamma-hydroxy-bêta-méthylbenzène-butanoïque,

Gamma-lactone de l'acide (bêta S*, gamma R*)-4-(3-(4-acétyl-3-hydroxy-2-propylphénoxy)propoxy)-3-bromo-gamma-hydroxy-bèta-méthylbenzène-butanoïque,

Gamma-lactone de l'acide (bêta R*, gamma S*)-4-(3-(4-acétyl-3-hydroxy-2-propylphénoxy)propylsulfonyl)-3-chloro-gamma-hydroxy-bêta-méthylbenzène-butanoïque,

Gamma-lactone de l'acide (bêta S*, gamma R*)-4-(3-(4-acétyl-3-hydroxy-2-propylphénoxy)propyloxy)-gamma-hydroxy-bêta-méthylbenzène-butanoïque,

Acide (bêta S*, gamma R*)-4-(3-(4-acétyl-3-hydroxy-2-propylphénoxy)propyloxy)-gamma-hydroxy-bêta-méthylbenzène-butanoïque,

Acide 4-(3-(4-acétyl-3-hydroxy-2-propylphénylthio)propyloxy)-3-fluoro-gamma-oxo-benzène-butanoïque,

S-oxyde de l'acide 4-(3-(4-acétyl-3-hydroxy-2-propylphénylthio)propyloxy)-3-fluoro-gamma-oxo-benzène-butanoïque,

Acide 4-(3-(4-acétyl-3-hydroxy-2-propylphénylsulfonyl)propyloxy)-3-fluoro-gamma-oxo-benzène-butanoïque,

Acide 4-(3-(4-acétyl-3-hydroxy-2-propylphénylthio)propylthio)-gamma-oxo-benzène-butanoïque,

Acide 4-(3-(4-acétyl-3-hydroxy-2-propylphénylthio)propylsulfonyl)-gamma-oxo-benzène-butanoïque,

Acide 2-(3-(4-acétyl-3-hydroxy-2-propylphénoxy)propoxy)-3-fluoro-gamma-oxo-benzène-butanoïque,

Acide 2-(3-(4-acétyl-3-hydroxy-2-propylphénoxy)propoxy)-3-fluoro-epsilon-oxo-benzène-hexanoïque,

Acide 4-(3-(4-acétyl-3-hydroxy-2-propylphénoxy)propoxy)-2,3-dichloro-gamma-oxo-benzène-butanoïque,

9. Dérivés utilisables comme médicaments des composés selon la revendication 1, répondant à la formule:

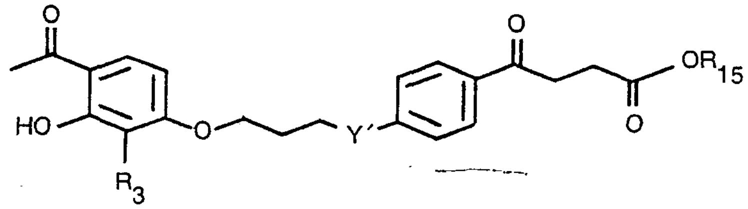

XV

dans laquelle Y' et $OR_{15}$ sont comme suite:

| Y′ | $OR^{15}$ |
|---|---|
| S | 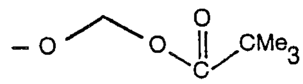 |
| $SO_2$ | 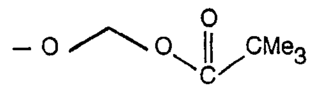 |
| S | 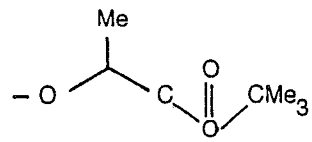 |
| $SO_2$ | 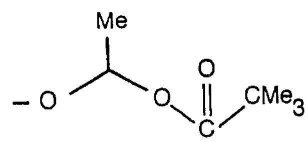 |
| S | 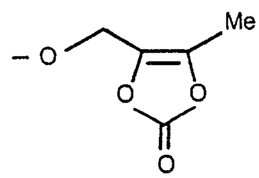 |
| $SO_2$ | 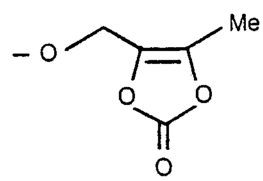 |
| S | 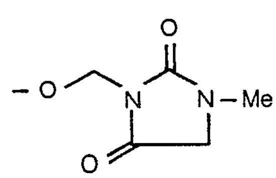 |
| $SO_2$ | 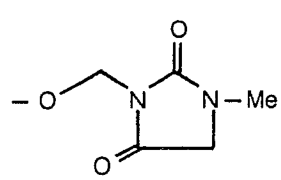 |

| Y′ | $OR^{15}$ |
|---|---|
| S | 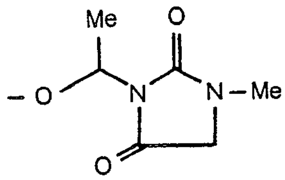 |
| $SO_2$ | 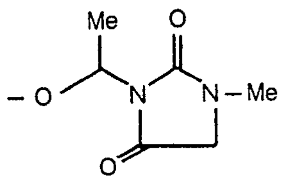 |
| S | 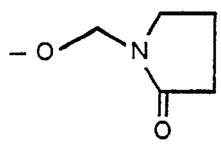 |
| $SO_2$ | 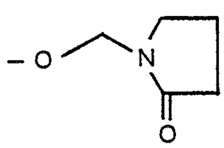 |
| S | 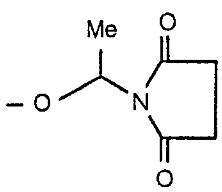 |
| $SO_2$ | 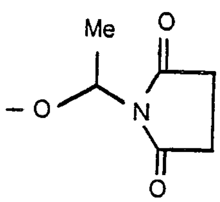 |

| Y′ | $OR^{15}$ |
|---|---|
| S | 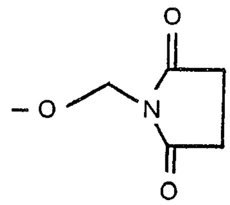 |
| $SO_2$ | 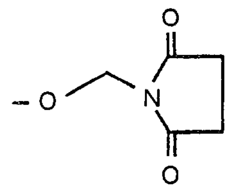 |

10. Composition pharmaceutique pour antagoniser les leucotriènes chez le mammifère, en particulier chez l'homme, contenant une quantité efficace d'un composé selon la revendication 1 et un véhicule pharmaceutiquement acceptable.

11. Composé selon la revendication 1 utilisable pour antagoniser l'action des leucotriènes chez le mammifère.

12. Procédé de préparation d'un composé selon la revendication 1 qui comprend la condensation d'un composé de formule:

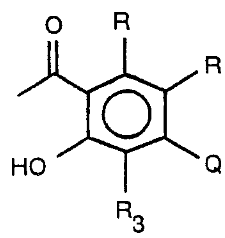

VII

dans laquelle Q est

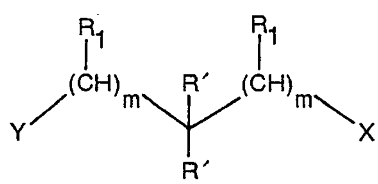

ou

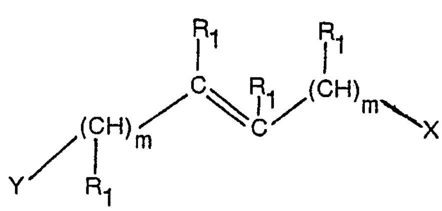

ou

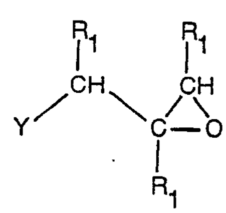

avec un composé de formule

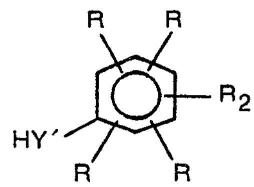

dans laquelle R, $R_1$, $R_2$, $R_3$, R', Y, Y' et m ont la signification donnée dans la revendication 1 et X est un halogène.

13. Procédé de préparation d'un composé selon la revendication 1, qui comprend la condensation d'un composé répondant à l'une des formules suivantes

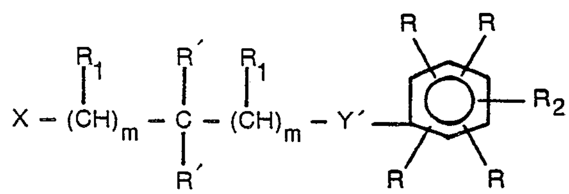

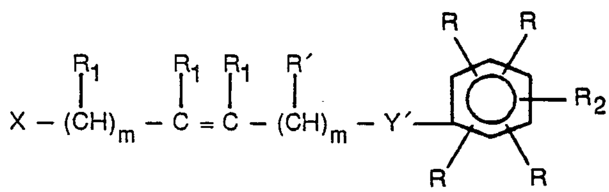

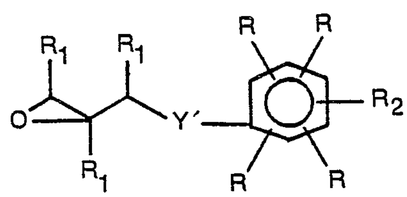

avec un composé de formule

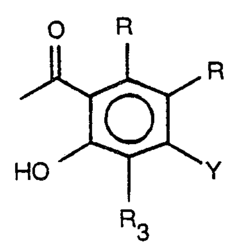

dans laquelle R, $R_1$, $R_2$, $R_2$, $R^1$, Y, Y' et m ont la signification donnée dans la revendication 1 et X est un halogène.

14. Composé répondant à l'une des formules suivantes

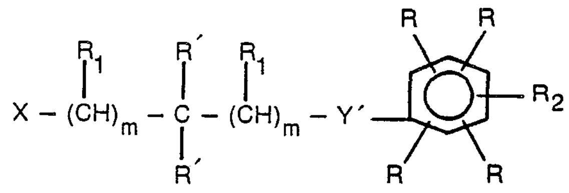

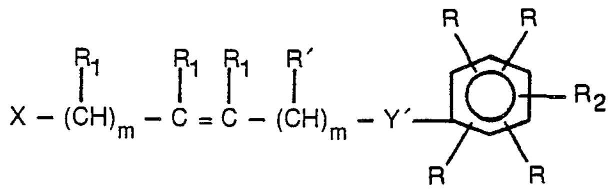

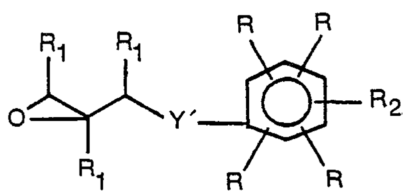

dans lesquelles Y', R, $R_1$, $R_2$, R' et m ont la signification donnée dans la revendication 1 et X est un halogène.

**Revendications pour l'Etat contractant: AT**

1. Procédé de préparation d'un composé répondant à l'une quelconque des formules suivantes:

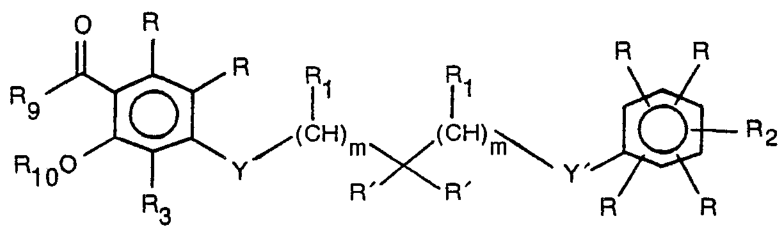

XI

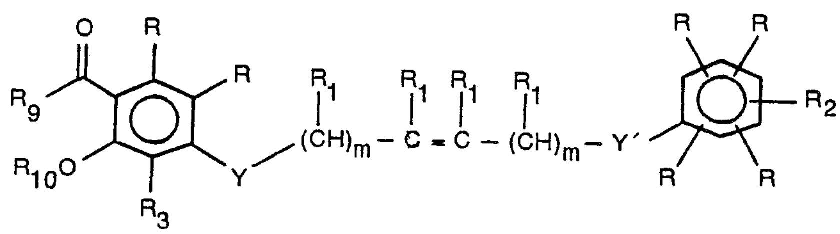

XII

dans lesquelles

chaque R est indépendamment H, OH, un groupe alkyle de 1 à 6 atomes de carbone qui peut être à chaîne droite ou ramifiée, alcényle de 2 à 6 atomes de carbone qui peut être à chaîne droite ou ramifiée, trifluorométhyle, alcoxy de 1 à 6 atomes de carbone qui peut être à chaîne droite ou ramifiée, SH, thioalkyle de 1 à 6 atomes de carbone qui peut être à chaîne droite ou ramifiée, phényle, phényle substitué par un groupe alkyle de 1 à 3 atomes de carbone ou par un halogène, benzyle, phénylalkyle ayant de 2 à 4 atomes de carbone dans le reste alkyle, halogène, amino, $N(R_4)_2$ dans lequel $R_4$ est H ou un groupe alkyle de 1 à 6 atomes de carbone qui peut être à chaîne droite ou ramifiée, $COOR_4$, $CH_2OR_4$, formyle, CN, trifluorométhylthio ou nitro;

chaque R′ est indépendamment $R_4$, $OR_4$, $COOR_4$, $N(R_4)_2$, $SR_4$, $CH_2OR_4$, CHO ou R′ et R′ sont ensemble O, $CH_2$ ou

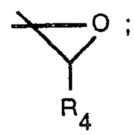

Y est l'oxygène, le soufre, un groupe sulfoxyde, sulfone,

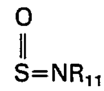

dans lequel $R_{11}$ est un groupe alkyle de 1—4 atomes de carbone, qui peut être à chaîne droite ou ramifiée, $NR_{12}$ dans lequel $R_{12}$ est H, un groupe alkyle de 1—4 atomes de carbone, qui peut être à chaîne droite ou ramifiée, ou

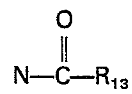

dans lequel $R_{13}$ est un groupe alkyle de 1—4 atomes de carbone, qui peut être à chaîne droite ou ramifiée, alcoxy de 1—4 atomes de carbone, qui peut être à chaîne droite ou ramifiée, ou N—CN;

Y′ est Y, $—CH_2—$ ou

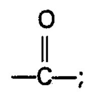

chaque $R_1$ est indépendamment l'hydrogène ou un groupe alkyle de 1—3 atomes de carbone;

chaque m est indépendamment un nombre entier de 0—6; et

$R_2$ est

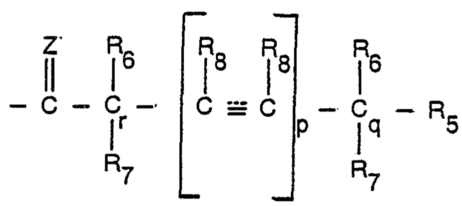

dans lequel Z est O, S, $CH_2$, H et OH, un groupe alcényle de 1—4 atomes de carbone; ou $N—R_{14}$ dans lequel $R_{14}$ est OH, un groupe alkyle ou alcoxy de 1 à 6 atomes de carbone, perhalogénoalkyle de 1 à 6 atomes de carbone, phényle ou phényle substitué par des groupes alkyle ou alcoxy de 1 à 3 atomes de carbone, halogène, hydroxy, halogénoalkyle, COOH, CN, formyle ou acyle de 1 à 6 atomes de carbone;

chaque $R_6$ est indépendamment H ou un groupe alkyle de 1—4 atomes de carbone;

chaque $R_7$ est indépendamment H, OH, ou un groupe alkyle de 1—4 atomes de carbone;

chaque $R_8$ est indépendamment H ou un groupe alkyle de 1—4 atomes de carbone, et est absent lorsqu'une triple liaison est présente;

$R_5$ est $COOR_4$; $CH_2OH$; CHO; tétrazole; $NHSO_2R_{14}$; $CONHSO_2R_{14}$; hydroxyméthylcétone; CN; $CON(R_7)_2$; un noyau hétérocyclique monocyclique ou bicyclique contenant un groupe hydroxyle acide; ou $COOR_{15}$ dans lequel $R_{15}$ est

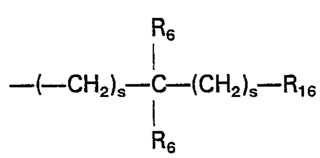

dans lequel chaque s est indépendamment 0—3; $R_{16}$ est

A) un radical hétérocyclique monocyclique ou bicyclique contenant de 3 à 12 atomes de carbone dans le noyau et 1 ou 2 hétéroatomes dans le noyau choisis parmi N et S, l'un au moins étant N, et chaque cycle du radical hétérocyclique étant formée de 5 ou 6 atomes, ou

B) le radical W—$R_{17}$ dans lequel W est O, S ou NH et $R_{17}$ contient jusqu'à 21 atomes de carbone et est (1) un radical hydrocarbure ou (2) un radical acyle d'un acide carboxylique organique acyclique ou monocyclique ne contenant pas plus de 1 hétéroatome dans le cycle;

r et q sont chacun indépendamment 0—20 à condition que le total de r et q ne dépasse pas 20; et p est 0 ou 1;

$R_3$ est un groupe alkyle de 1 à 6 atomes de carbone qui peut être à chaîne droite ou ramifiée; ou alcényle de 3 à 6 atomes de carbone qui peut être à chaîne droite ou ramifiée;

$R_9$ est un groupe alkyle de 1 à 6 atomes de carbone qui peut être à chaîne droite ou ramifiée; alcoxy de 1 à 6 atomes de carbone qui peut être à chaîne droite ou ramifiée; ou $(CH_2)_rR_5$;

$R_{10}$ est H; un groupe alkyle de 1 à 6 atomes de carbone qui peut être à chaîne droite ou ramifiée;

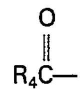

ou $R_4OCH_2$—;

ou d'un composé qui est une lactone de ce composé lorsque, dans $R_2$, Z est H et OH ou $R_7$ est OH et $R_5$ est un groupe carboxy, ou d'un composé qui est un sel pharmaceutiquement acceptable ou un sel d'addition avec un acide de ce composé, qui comprend la condensation d'un composé de formule:

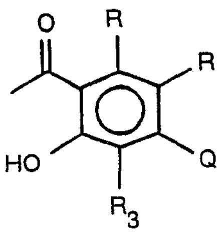

VII

dans laquelle Q est

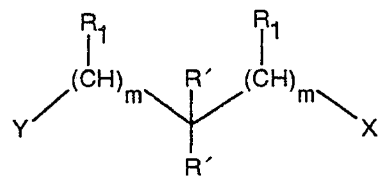

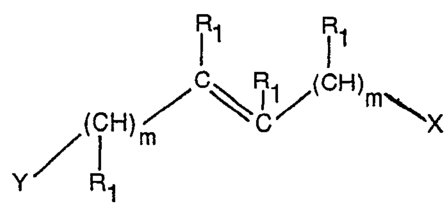

ou

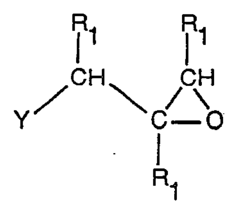

acvec un composé de formule

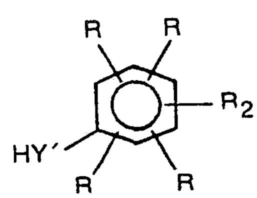

dans laquelle R, $R_1$, $R_2$, $R_3$, R', Y, Y' et m sont tels que définis et X est un halogène.

2. Procédé de préparation d'un composé répondant à l'une quelconque des formules XI ou XII de la revendication 1, qui comprend la condensation d'un composé répondant à l'une des formules suivantes:

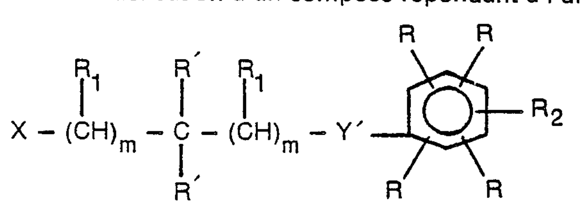

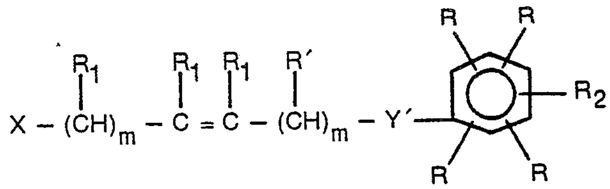

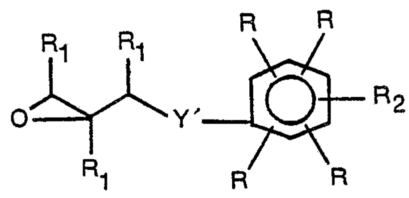

avec un composé de formule

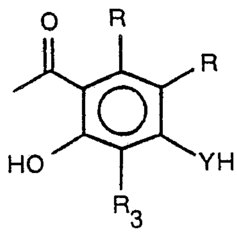

dans laquelle R, $R_1$, $R_2$, $R_3$, $R^1$, Y, Y', m et X sont tels que définis dans la revendication 1.

3. Procéde selon la revendication 1 ou 2, s'appliquant à la préparation de l'acide 4-((3-(4-acétyl-3-hydroxy-2-propylphénoxy)propyl)thio)-gamma-oxobenzène-butanoïque.

4. Procéde selon la revendication 1 ou 2, s'appliquant à la préparation de l'acide 4-[3-(4-acétyl-3-hydroxy-2-propylphénoxy)propylsulfonyl]-gamma-oxobenzène-butanoïque.

5. Procéde selon la revendication 1 ou 2, s'appliquant à la préparation de sel de sodium de l'acide (bêta S*, gamma R*)-4-(3-(4-acétyl-3-hydroxy-2-propylphénoxy)propylthio)-gamma-hydroxy-bêta-méthyl-benzène-butanoïque.

6. Procéde selon la revendication 1 ou 2, s'appliquant à la préparation du sel de sodium de l'acide (bêta S*, gamma R*)-4-(3-(4-acétyl-3-hydroxy-2-propylphénoxy)propyloxy)-gamma-hydroxy-bêta-méthyl-benzène-butanoïque.

7. Procéde selon la revendication 1 ou 2, s'appliquant à la préparation de l'acide 4-(3-(4-acétyl-3-hydroxy-2-propylphénoxy)propylthio)-epilson-oxobenzène-hexanoïque.

8. Procéde selon la revendication 1 ou 2, s'appliquant à la préparation d'un composé dans lequel Y est l'oxygène et Y' est l'oxygène, le soufre, un groupe sulfoxyde, sulfone, amino ou cyanamido.

9. Procédé selon la revendication 1, 2 ou 7, s'appliquant à la préparation d'un composé dans lequel chaque m est 1.